# EUROPEAN PATENT APPLICATION

(11) **EP 4 570 308 A1**
(43) Date of publication of application: **18.06.2025**
(21) Application number: 23852652.9
(22) Date of filing: 14.08.2023
(51) Int. Cl.: A61N 1/40

(54) **LIQUID CONTROL DEVICE**

(30) Priority: 11.08.2022 JP 2022128566; 11.08.2023 TW 112130356
(71) Applicant: Evertron Holdings Pte. Ltd., Tokyo 107-0061 (JP)
(72) Inventor: TANAKA Hisao, Tokyo 106-0032 (JP)
(74) Representative: dompatent von Kreisler Selting Werner - Partnerschaft von Patent- und Rechtsanwälten mbB
(86) International application number: PCT/JP2023/029386
(87) International publication number: WO 2024/034690

(57) **Abstract**

Provided is a liquid control apparatus that can adjust an electric field, a magnetic field, an electromagnetic field, or electromagnetic waves generated from an electrode and perform effective therapies or treatments with a compact device configuration. A liquid control apparatus in an embodiment of the present invention includes at least one electrode, and a controller configured to control at least one of a voltage value, a current value, a frequency, or a phase of voltage or current applied to the electrode. While the electrode is disposed to face a target object, the controller adjusts a voltage value, a current value, a frequency, or a phase of voltage or current applied to the electrode, controls an electric field, a magnetic field, an electromagnetic field, or electromagnetic waves generated from the electrode, and performs control so that at least one of a frequency or a voltage applied to the electrode, or an emission direction of an electric field, a magnetic field, an electromagnetic field, or electromagnetic waves generated from the electrode changes over time, or controls a duration of voltage applied to the electrode, according to the target object facing the electrode.

## Description

### Field

The present invention relates to a liquid control apparatus.

### Background

Research has been conducted on the effects of electromagnetic field exposure on the human body in therapies for headache, stiff shoulder, and the like by applying an electromagnetic field to the human body.

For example, in Patent Literature 1, electrodes are installed on the head side and the sole side of a chair on which a person to be treated is seated, and AC high voltage of different frequencies is applied to these two electrodes, so that the difference in frequency of the AC high voltage applied between the two electrodes is approximately the same as the low frequency (1 to 1.8 Hz) equal to or lower than the normal heartbeat cycle of the human body (60 to 90 beats per minute). This is expected to have a parasympathetic nervous system-dominant relaxing effect. As an example of such a potential therapy device, a Hakuju AC high-voltage electric field health care device Healthtron HES-A30 has been approved by the Japanese Ministry of Health, Labour and Welfare (approval number 21100BZZ00265000) and is commercially available.

### Citation List

### Patent Literature

Patent Literature 1: Japanese Patent No. 5235040

### Summary

### Technical Problem

However, the potential therapy device described in Patent Literature 1 applies a high voltage of about 9000 V to the electrodes and requires a transformer to boost 100 V (50/60 Hz) of commercial power to a high voltage, which makes the device larger and more complicated to ensure safety. In addition, Patent Literature 1 is expected to have a parasympathetic nervous system-dominant relaxing effect, and is limited to the psychological effect on the subject and does not exert a more direct therapeutic effect.

An object of the present invention is to provide a liquid control apparatus that can adjust an electric field, a magnetic field, an electromagnetic field, or electromagnetic waves generated from an electrode and perform effective therapies or treatments with a compact device configuration.

### Solution to Problem

The object of each embodiment of the present invention can be achieved by the following configuration. Specifically, a liquid control apparatus according to one aspect of the present invention includes at least one electrode, and a controller configured to control at least one of a voltage value, a current value, a frequency, or a phase of voltage or current applied to the electrode. While the electrode is disposed to face a target object, the controller adjusts at least one of a voltage value, a current value, a frequency, or a phase of voltage or current having a DC component and/or an AC component applied to the electrode, controls at least one of an electric field, a magnetic field, an electromagnetic field, or electromagnetic waves generated from the electrode, and controls a state of liquid in the target object facing the electrode to perform control of improving or preventing edema, cellular edema, or neuronal edema, to control hormonal, endocrine, lymphatic, or pathway balance, or to control mitochondrial activity, autophagy activity, egg activity, or sperm activity.

A liquid control apparatus according to a second aspect of the present invention includes at least one electrode, and a controller configured to control at least one of a voltage value, a current value, a frequency, or a phase of voltage or current applied to the electrode. While the electrode is disposed to face a target object, the controller adjusts at least one of a voltage value, a current value, a frequency, or a phase of voltage or current having a DC component and/or an AC component applied to the electrode, controls at least one of an electric field, a magnetic field, an electromagnetic field, or electromagnetic waves generated from the electrode, and controls a state of liquid in the target object facing the electrode, to perform control of improving or preventing clogging of body fluid, drug solution, cosmetics, or liquid, to perform control of improving or enhancing fluidity of body fluid, drug solution, cosmetics, or liquid, to perform control of improving or preventing induration or hardening, to perform control of improving or preventing water release from cells or tissues, to perform control of removing active oxygen or lactic acid, to perform control of improving or preventing muscle imbalance, to perform control of improving or enhancing body balance, myofascial balance, occlusion, osteopathy, or manual therapy, to perform control of improving or preventing bone fracture or joint disease, to perform control of improving or enhancing heart beat or pulse, to perform control of improving or normalizing blood pressure, to perform control of improving or enhancing respiratory function or lung function, to perform control of improving or enhancing dialysis function, to perform control of improving or enhancing visual acuity or dynamic vision, to perform control of improving or preventing ophthalmologic disease, to perform control of improving or preventing internal medicine disease, to perform control of improving or enhancing immune function, to perform control of improving a drug delivery system or permeability of cosmetics, to perform control of improving or enhancing efficacy or quality of medicine, cosmetics, or liquid, to perform control of improving or enhancing extraction of body fluid, medicine, cosmetics, or liquid, or to control wave motion of liquid or ectoplasm.

A liquid control apparatus according to a third aspect of the present invention includes at least one electrode, and a controller configured to control at least one of a voltage value, a current value, a frequency, or a phase of voltage or current applied to the electrode. While the electrode is disposed to face a target object, the controller adjusts at least one of a voltage value, a current value, a frequency, or a phase of voltage or current having a DC component and/or an AC component applied to the electrode, controls at least one of an electric field, a magnetic field, an electromagnetic field, or electromagnetic waves generated from the electrode, and controls a state of liquid in the target object facing the electrode, to perform control of preventing growth of microorganisms, bacteria, fungi, or viruses, to perform control of improving or preventing burns, necrosis, or decubitus ulcer, to perform control of preventing metastasis, to perform control of improving or preventing dementia, Alzheimer's disease, or Parkinson's disease, to perform control of sleep improvement, cosmetic improvement, PMS, menstrual pain, pain, itching, health promotion, motor function improvement, or anti-aging, to perform control of improving or enhancing formation of teeth, bones, joints, blood vessels, lymph vessels, nerves, cells, skin, hair, or organs, to perform control of improving storage, freezing, thawing, culturing, or logistics of organs, body fluids, blood, cells, tissues, skin, hair, dead bodies, DNA, stem cells, sperms, eggs, medicines, cosmetics, or liquids, or to improve an electrode or a container for use in storage, freezing, thawing, culturing, or logistics of organs, body fluids, blood, cells, tissues, skin, hair, dead bodies, DNA, stem cells, sperms, eggs, medicines, cosmetics, or liquids.

According to a fourth aspect of the present invention, in the liquid control apparatus according to any one of the first to third aspects, the voltage value of voltage applied to the electrode is controlled in a range of 0 V to 7000 V or the frequency of the AC component of the voltage is controlled in a range of 0 Hz to 1 MHz.

According to a fifth aspect of the present invention, in the liquid control apparatus according to any one of the first to third aspects, the voltage value and/or the frequency of the voltage varies over time smoothly or stepwise within a predetermined range.

According to a sixth aspect of the present invention, in the liquid control apparatus according to any one of the first to third aspects, the liquid control apparatus performs controlling a pearl-chain structure, arrangement direction, moisture bonding, or moisture activity of moisture in the target object, controlling interfacial polarization, interfacial tension or emulsion state between an aqueous phase and another phase in the target object, or controlling a state of active oxygen in the target object.

According to a seventh aspect of the present invention, in the liquid control apparatus according to any one of the first to third aspects, a control effect is sustained for a predetermined period of time even after an electric field, a magnetic field, an electromagnetic field, or electromagnetic waves are generated from the electrode for a predetermined period of time, and then the electric field, the magnetic field, the electromagnetic field, or the electromagnetic waves are removed.

According to an eighth aspect of the present invention, in the liquid control apparatus according to any one of the first to third aspects, the voltage applied to the electrode includes the AC component in addition to the DC component.

According to a ninth aspect of the present invention, in the liquid control apparatus according to any one of the first to third aspects, the electrode has a shape of plate, rod, sheet, needle, or comb, or a shape combining two or more electrodes with each other.

According to a tenth aspect of the present invention, in the liquid control apparatus according to any one of the first to third aspects, the controller is managed via a cloud, a server, or a network.

According to an eleventh aspect of the present invention, in the liquid control apparatus according to any one of the first to third aspects, the controller sets a control parameter by machine learning.

According to a twelfth aspect of the present invention, in the liquid control apparatus according to any one of the first to third aspects, control is performed to achieve efficacy for at least one of infarction, necrosis, decubitus ulcer, burns, removal of active oxygen, removal of lactic acid, improvement of blood flow, improvement of lymph flow, cerebral infarction, myocardial infarction, thrombosis, embolism, arteriosclerosis, improvement or prevention of clogging of body fluid, drug solution, cosmetics, or liquid, improvement of fluidity of body fluid, drug solution, cosmetics, or liquid, improvement or prevention of induration or hardening, improvement or prevention of water release from cells or tissues, cellular edema, neuronal edema, hydropsy, pulmonary edema, joint edema, ascites edema, insulin secretion disorder, defecation disorder, defecation difficulty, constipation, urinary disorder, congestion, blisters, enhancement of drug delivery, reduction of viscosity of drug solution or body fluid, cell culture, regenerative medicine, reduction of culture time, enhancement of culture quality, or enhancement of culture efficiency in regenerative medicine, cell tissue regeneration, reduction of lactic acid, enlargement, swelling, edema, body fluid retention, dehydration of extracellular or intracellular fluid, skin diseases, pigmentation, chloasma, freckle, epidermal wrinkles, dermal wrinkles, expression wrinkles, xeroderma, improvement of physical condition, induration, muscular induration, myofascial release, myofascial potential improvement, nerve deformation disease, myofascial electromagnetic therapy, muscle imbalance, myofascial potential balance improvement, body balance improvement, myofascial balance improvement, occlusion improvement, osteopathic improvement, manual therapeutic improvement, improvement or prevention of bone fracture or joint disease, endocrine, lymphatic, or pathway balance improvement, bone fracture, joint disease, skin care, moisture retention, fertility improvement, infertility, improvement of sperm motility, sperm activity, egg activity, mitochondrial activity, autophagy activity, PMS, pain, itching, abnormal sensation, cold sensitivity, insensitivity, cramp, anti-aging, hair follicle care, improvement of menopausal disorder, enhancement of detergency, alopecia, AGA, ED, lipolysis promotion, improvement of contact lens fit, dry eye, visual acuity improvement, dynamic vision improvement, sleep disorder, sleep improvement, sleep apnea syndrome, preventive medicine improvement, nerve cell improvement, cellular edema improvement, control of viruses, bacteria, or molds, cancer, glaucoma, cataract, age-related macular degeneration, eye disease, hearing loss, hearing impairment, visual impairment, dementia, Alzheimer's disease, Parkinson's disease, water balance, gastrointestinal diseases, respiratory diseases, cardiovascular diseases, neurological diseases, hematologic diseases, kidney diseases, endocrinological diseases, internal medicine diseases, improvement or normalization of blood pressure, improvement or enhancement of pulse or heartbeat, improvement or enhancement of respiratory function or pulmonary function, improvement or enhancement of dialysis function, osteopathic therapy, rigor mortis improvement, or therapy for rehabilitation medicine improvement.

According to a thirteenth aspect of the present invention, in the liquid control apparatus according to any one of the first to third aspects, control is performed to achieve efficacy for at least one of improvement of storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement of an electrode or a container for use in storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement or enhancement of efficacy or quality of medicines, cosmetics, or liquids, improvement or enhancement of extraction of body fluids, medicines, cosmetics, or liquids, improvement or enhancement of formation of teeth, bones, joints, blood vessels, lymph vessels, nerves, cells, skin, hair, or organs, improvement or enhancement of emulsion properties, improvement or enhancement of drug delivery properties, improvement or enhancement of efficacy or performance of medicines, prevention or control of growth of microorganisms, bacteria, fungi, or viruses, prevention of metastasis, reduction of viscosity of drug solution, or control of wave motion of liquids, or control of ectoplasm.

According to a fourteenth aspect of the present invention, in the liquid control apparatus according to any one of the first to third aspects, control is performed to enhance performance of at least one of following devices: an electromagnetic therapy device; a potential therapy device; a low frequency therapy device, an EMS; a massage device; a facial device; a vibration device; a cavitation device; a micro-bubble device; a micro-nano bubble device; a nano bubble device; a fine bubble device; a terahertz device; a high frequency device; a quantum therapy device; a hair growth device; a cellulite device; a muscle relaxation device; an ultrasonic therapy device; an ozone generating device; a hydrogen generating device; an LED device; a beauty device; and a slimming device.

A liquid control apparatus according to a fifteenth aspect of the present invention includes at least one electrode, and a controller configured to control at least one of a voltage value, a current value, a frequency, or a phase of voltage or current applied to the electrode. While the electrode is disposed to face a target part, the controller adjusts at least one of a voltage value, a current value, a frequency, or a phase of voltage or current having a DC component and/or an AC component applied to the electrode, and controls at least one of an electric field, a magnetic field, an electromagnetic field, or electromagnetic waves generated from the electrode, and the controller performs control so that at least one of a frequency or a voltage applied to the electrode, or an emission direction of an electric field, a magnetic field, an electromagnetic field, or electromagnetic waves generated from the electrode changes over time, or controls a duration of voltage applied to the electrode, according to the target part facing the electrode.

### Advantageous Effects of Invention

The liquid control apparatus according to the present invention can adjust an electric field, a magnetic field, an electromagnetic field, or electromagnetic waves generated from an electrode and perform effective therapies or treatments with a compact device configuration. Specifically, the liquid control apparatus according to each aspect has the following effects.

The liquid control apparatus according to the first aspect controls the state of liquid in the target object facing the electrode to increase the interfacial polarization of the moisture in the target object and decrease the interfacial tension, whereby the particles of the liquid are atomized into fine particles. In the liquid control apparatus according to the present aspect, the size of water droplet particles obtained by the application of an electric field is 2.5 to 8 µm. Since the spacing between cells in a living body is 12 µm or less, the water droplets with a particle diameter of 12 µm or less obtained in the present embodiment have the property of easily penetrating between cells in a living body. In the present embodiment, the moisture particles atomized into fine particles by the application of an electric field penetrate between the cells of a living body such as an animal and a plant, resulting in a variety of efficacy. The improvement of liquid metabolism enables control of improving or preventing edema, cellular edema, or neuronal edema. In addition, the improvement of liquid metabolism enables control of hormonal, endocrine, lymphatic, or pathway balance. In addition, the improvement of liquid metabolism enables control of mitochondrial activity, autophagy activity, egg activity, or sperm activity.

The liquid control apparatus according to the second aspect of the present invention controls the state of liquid in the target object facing the electrode to increase the interfacial polarization of the moisture in the target object and decrease the interfacial tension, whereby the particles of the liquid are atomized into fine particles. In the liquid control apparatus according to the present aspect, the size of water droplet particles obtained by the application of an electric field is 2.5 to 8 µm. Since the spacing between cells in a living body is 12 µm or less, the water droplets with a particle diameter of 12 µm or less obtained in the present embodiment have the property of easily penetrating between cells in a living body. In the present embodiment, the moisture particles atomized into fine particles by the application of an electric field penetrate between the cells of a living body such as animal and plant, resulting in a variety of efficacy. The atomization of liquid into fine particles enables control of improving or preventing clogging of body fluid, drug solution, cosmetics, or liquid, or control of improving or enhancing the fluidity of body fluid, drug solution, cosmetics, or liquid. Maintaining an appropriate amount of water in cells and tissues enables control of improving or preventing induration or hardening. In addition, the seepage of moisture through cells and tissues enables control of improving or preventing water release from cells or tissues. The improvement of liquid flow results in improvement of metabolism, which enables control of removing active oxygen or lactic acid. For example, muscle hardens due to water release from the muscle. The liquid control apparatus according to the present aspect applies an electric field from the electrode to perform control to decrease the interfacial tension of liquid and atomize the moisture into fine particles. This results in improvement of metabolism of cells and tissues to enable control of improving or preventing muscle imbalance, or control of improving or enhancing body balance, myofascial balance, occlusion, osteopathy, or manual therapy. For bone fracture and vascular disease, sufficient supply of blood and lymphatic fluid to an affected area is essential to enhancing natural healing power. The improvement of fluidity of liquid enables control of improving or preventing bone fracture or joint disease. With blood flow improvement, it is possible to perform control of improving or enhancing heartbeat or pulse, and perform control of improving or normalizing blood pressure. The blood flow improvement and the liquid fluidity improvement can contribute to control of improving or enhancing respiratory function or pulmonary function, and control of improving or enhancing dialysis function. The improvement of liquid metabolism enables control of improving or enhancing visual acuity and dynamic vision, and control of improving or preventing ophthalmologic diseases. The blood flow improvement or the water balance improvement enables control of improving or preventing internal medicine diseases. In addition, the improvement of liquid metabolism enables control of improving or enhancing immune function. The improvement of liquid fluidity enables control of improving a drug delivery system or permeability of cosmetics. By applying an electric field from the electrode, the liquid control apparatus of the present aspect can improve the emulsion state of liquids, that is, micronize two liquids and enhance the degree of mixing of two liquids, resulting in, for example, a state in which oil is well dispersed in water or water is well dispersed in oil. Thus, it is possible to perform control of improving or enhancing the efficacy or quality of medicine, cosmetics, or liquid. In addition, it is possible to perform control of improving or enhancing extraction of body fluid, medicine, cosmetics, or liquid. The conventional extraction time is greatly reduced, the extract is transformed into a constitution that does not deteriorate for a long time, and a large amount of extract can be obtained from the same raw material. Thus, the extraction efficiency can be enhanced. By applying an electric field from the electrode, the liquid control apparatus according to the present aspect can reduce the interfacial tension of liquid, arrange the particles of liquid into a pearl-chain structure, and enhance the emulsion properties. In addition, the enhancement of quality of moisture, for example, the improvement of wave motion of liquid can be achieved. The improvement of moisture quality enables control on all natural phenomena involving moisture, such as control of ectoplasm.

The liquid control apparatus according to the third aspect of the prevent invention controls the state of liquid in the target object facing the electrode, and performs control to reduce the interfacial tension of liquid to atomize the liquid particles into fine particles, and performs control so that the particles of the liquid atomized into fine particles form a pearl-chain structure. In the body, tissues, and cells, microorganisms, bacteria, fungi, and viruses bond to free water in the body and grow using the moisture of the free water. The growth of microorganisms, bacteria, fungi, and viruses can be prevented or suppressed by performing control so that the free water in the body, tissues, and cells achieves a pearl-chain structure. Thus, it is possible to perform control of preventing the growth of microorganisms, bacteria, fungi, or viruses. In addition, the liquid control apparatus according to the present aspect can reduce the interfacial tension of liquids, atomize the liquid particles into fine particles, and arrange the fine liquid particles into a pearl-chain structure. The enhancement of liquid metabolism and the prevention of the growth of microorganisms, bacteria, fungi, or viruses enable control of improving or preventing burns, necrosis, or decubitus ulcer. Metastasis is the growth of lesions migrated to other organs through the blood and lymphatic fluid flow. The enhancement of liquid metabolism contributes to enhancement of immune function, which enables control of preventing metastasis. The enhancement of liquid metabolism is also necessary for brain activation. The liquid control apparatus according to the present aspect can perform control of improving or preventing dementia, Alzheimer's disease, or Parkinson's disease by improving the water balance and water metabolism in the brain. By enhancing the water balance and water metabolism, the liquid control apparatus according to the present aspect can control sleep improvement, beauty improvement, PMS, menstrual pain, pain, itching, health promotion, motor function improvement, or anti-aging. By applying an electric field from the electrode, the liquid control apparatus according to the present aspect can decrease the interfacial tension of liquid, atomize the liquid particles into fine particles, and improve the emulsion state of liquids, that is, micronize two liquids, and enhance the degree of mixing of two liquids, resulting in, for example, a state in which oil is well dispersed in water or water is well dispersed in oil. For the formation of teeth, bones, joints, blood vessels, lymph vessels, nerves, cells, skin, hair, or organs, the liquid control apparatus according to the present aspect functions to improve moisture permeability, improve moisture retention characteristics, and improve emulsion properties by micronizing water particles by decreasing the interfacial tension of moisture. Thus, even when tissues are artificially generated, it is possible to perform control of improvement or enhancement such as quality enhancement, reduction in formation time, simplification of quality procedures, and cost reduction. The liquid control apparatus according to the present aspect controls the state of liquid in the target object facing the electrode, and performs control to decrease the interfacial tension of liquid to atomize the liquid particles into fine particles, and performs control to arrange the particles of the liquid atomized into fine particles into a pearl-chain structure. In the body, tissues, and cells, microorganisms, bacteria, fungi, and viruses bond to free water in the body and grow using the moisture of the free water. The growth of microorganisms, bacteria, fungi, and viruses can be prevented or suppressed by performing control so that the free water in the body, tissues, and cells achieves a pearl-chain structure. In addition, a pearl-chain structure of moisture in tissues and cells enables prevention of moisture evaporation or water release, thereby maintaining the freshness of tissues and cells. As a result, it is possible to perform control of improving the storage, freezing, thawing, culturing, or logistics of organs, body fluids, blood, cells, tissues, skin, hair, dead bodies, DNA, stem cells, sperms, eggs, medicines, cosmetics, or liquids, and to improve an electrode or a container for use in the storage, freezing, thawing, culturing, or logistics of organs, body fluids, blood, cells, tissues, skin, hair, dead bodies, DNA, stem cells, sperms, eggs, medicines, cosmetics, or liquids.

The liquid control apparatus according to the fourth aspect of the present invention is controlled such that the voltage value and the frequency set in advance according to the site and the state of the target object by the controller are selected from a range of 0 V to 7000 V as the voltage value of voltage applied to the electrode and a range of 0 Hz to 1 MHz as the frequency of the AC component of the voltage.

The liquid control apparatus according to the fifth aspect of the present invention is controlled such that the voltage value and/or the frequency of the voltage varies over time smoothly or stepwise within a predetermined range. As a result, even when the optimal voltage and frequency are not known in advance, the optimal voltage value and frequency are always supplied to the electrode.

The liquid control apparatus according to the sixth aspect of the present invention performs controlling a pearl-chain structure, arrangement direction, moisture bonding, or moisture activity of moisture in the object, controlling interfacial polarization, interfacial tension, or emulsion state between an aqueous phase and another phase in the object, or controlling a state of active oxygen in the object.

In the liquid control apparatus according to the seventh aspect of the present invention, a control effect is sustained for a predetermined period of time even after an electric field, a magnetic field, an electromagnetic field, or electromagnetic waves are generated from the electrode for a predetermined period of time, and then the electric field, the magnetic field, the electromagnetic field, or the electromagnetic waves are removed.

In the liquid control apparatus according to the eighth aspect of the present invention, the voltage applied to the electrode includes the AC component in addition to the DC component.

In the liquid control apparatus according to the ninth aspect of the present invention, the electrode has a shape of plate, rod, sheet, needle, or comb, or a shape combining two or more electrodes with each other.

In the liquid control apparatus according to the tenth aspect of the present invention, the controller is managed via a cloud, a server, or a network.

In the liquid control apparatus according to the eleventh aspect of the present invention, the controller sets a control parameter by machine learning.

The liquid control apparatus according to the twelfth aspect of the present invention performs control to achieve efficacy for at least one of infarction, necrosis, decubitus ulcer, burns, removal of active oxygen, removal of lactic acid, improvement of blood flow, improvement of lymph flow, cerebral infarction, myocardial infarction, thrombosis, embolism, arteriosclerosis, improvement or prevention of clogging of body fluid, drug solution, cosmetics, or liquid, improvement of fluidity of body fluid, drug solution, cosmetics, or liquid, improvement or prevention of induration or hardening, improvement or prevention of water release from cells or tissues, cellular edema, neuronal edema, hydropsy, pulmonary edema, joint edema, ascites edema, insulin secretion disorder, defecation disorder, defecation difficulty, constipation, urinary disorder, congestion, blisters, enhancement of drug delivery, reduction of viscosity of drug solution or body fluid, cell culture, regenerative medicine, reduction of culture time, enhancement of culture quality, or enhancement of culture efficiency in regenerative medicine, cell tissue regeneration, reduction of lactic acid, enlargement, swelling, edema, body fluid retention, dehydration of extracellular or intracellular fluid, skin diseases, pigmentation, chloasma, freckle, epidermal wrinkles, dermal wrinkles, expression wrinkles, xeroderma, improvement of physical condition, induration, muscular induration, myofascial release, myofascial potential improvement, nerve deformation disease, myofascial electromagnetic therapy, muscle imbalance, myofascial potential balance improvement, body balance improvement, myofascial balance improvement, occlusion improvement, osteopathic improvement, manual therapeutic improvement, improvement or prevention of bone fracture or joint disease, endocrine, lymphatic, or pathway balance improvement, bone fracture, joint disease, skin care, moisture retention, fertility improvement, infertility, improvement of sperm motility, sperm activity, egg activity, mitochondrial activity, autophagy activity, PMS, pain, itching, abnormal sensation, cold sensitivity, insensitivity, cramp, anti-aging, hair follicle care, improvement of menopausal disorder, enhancement of detergency, alopecia, AGA, ED, lipolysis promotion, improvement of contact lens fit, dry eye, visual acuity improvement, dynamic vision improvement, sleep disorder, sleep improvement, sleep apnea syndrome, preventive medicine improvement, nerve cell improvement, cellular edema improvement, control of viruses, bacteria, or molds, cancer, glaucoma, cataract, age-related macular degeneration, eye disease, hearing loss, hearing impairment, visual impairment, dementia, Alzheimer's disease, Parkinson's disease, water balance, gastrointestinal diseases, respiratory diseases, cardiovascular diseases, neurological diseases, hematologic diseases, kidney diseases, endocrinological diseases, internal medicine diseases, improvement or normalization of blood pressure, improvement or enhancement of pulse or heartbeat, improvement or enhancement of respiratory function or pulmonary function, improvement or enhancement of dialysis function, osteopathic therapy, rigor mortis improvement, or therapy for rehabilitation medicine improvement.

The liquid control apparatus according to the thirteenth aspect of the present invention performs control to achieve efficacy for at least one of improvement of storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement of an electrode or a container for use in storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement or enhancement of efficacy or quality of medicines, cosmetics, or liquids, improvement or enhancement of extraction of body fluids, medicines, cosmetics, or liquids, improvement or enhancement of formation of teeth, bones, joints, blood vessels, lymph vessels, nerves, cells, skin, hair, or organs, improvement or enhancement of emulsion properties, improvement or enhancement of drug delivery properties, improvement or enhancement of efficacy or performance of medicines, prevention or control of growth of microorganisms, bacteria, fungi, or viruses, prevention of metastasis, reduction of viscosity of drug solution, or control of wave motion of liquids, or control of ectoplasm.

The liquid control apparatus according to the fourteenth aspect of the present invention performs control to enhance performance of at least one of following devices: an electromagnetic therapy device; a potential therapy device; a low frequency therapy device; an EMS; a massage device; a facial device; a vibration device; a cavitation device; a micro-bubble device; a micro-nano bubble device; a nano bubble device; a fine valve device; a terahertz device; a high frequency device; a quantum therapy device; a hair growth device; a cellulite device; a muscle relaxation device; an ultrasonic therapy device; an ozone generating device; a hydrogen generating device; an LED device; a beauty device; and a slimming device.

The liquid control apparatus according to the fifteenth aspect of the present invention includes at least one electrode, and a controller configured to control at least one of a voltage value, a current value, a frequency, or a phase of voltage or current applied to the electrode. While the electrode is disposed to face a target object, the controller adjusts at least one of a voltage value, a current value, a frequency, or a phase of voltage or current having a DC component and/or an AC component applied to the electrode, and controls at least one of an electric field, a magnetic field, an electromagnetic field, or electromagnetic waves generated from the electrode, and the controller performs control so that at least one of a frequency or a voltage applied to the electrode, or an emission direction of an electric field, a magnetic field, an electromagnetic field, or electromagnetic waves generated from the electrode changes over time, or controls a duration of voltage applied to the electrode, according to the target object facing the electrode. By applying an electric field from the electrode, the liquid control apparatus according to the present aspect performs control to increase the interfacial polarization of liquid, decrease the interfacial tension to atomize the particles of the liquid into fine particles, and arrange the fine liquid particles into a pearl-chain structure. In addition, it is possible to enhance the emulsion properties of the liquid atomized into fine particles, and to enhance the quality of liquid, for example, improve the wave energy of liquid. With these functions, the liquid control apparatus according to the present aspect can adjust an electric field, a magnetic field, an electromagnetic field, or electromagnetic waves generated from the electrode and perform effective therapies or treatments with a compact device configuration.

### Brief Description of Drawings

FIG. 1 is a conceptual view of an electrode according to a first embodiment.
FIG. 2 is a schematic view of water molecules, FIG. 2A illustrating water molecules in a freely moving state, and FIG. 2B illustrating water molecules in a pearl-chain structure.
FIG. 3 is a photomicrograph of free water, FIG. 3A showing a state of the free water before application of an electric field and FIG. 3B showing a state of the free water after the application of the electric field.
FIG. 4 illustrates potential simulation results of water particles, FIG. 4A being a diagram illustrating a simulation model and FIG. 4B illustrating a potential simulation result.
FIG. 5 is a graph of interfacial tension between cooking oil and water as a result of changing the frequency and voltage value (0 to 75 V) of the voltage applied.
FIG. 6 is a graph of interfacial tension between cooking oil and water as a result of changing the frequency and voltage value (0 to 150 V) of the voltage applied to the electrode.
FIG. 7 includes photographs showing dropping of droplets in oil.
FIG. 8 includes photographs showing fine particles around droplets in oil.
FIG. 9 is a conceptual view of electrodes according to a first modification of the first embodiment.
FIG. 10 is a conceptual view of a different electrode according to the first modification of the first embodiment, FIG. 10A illustrating an example where a single electrode is used, and FIG. 10B illustrating an example where a single electrode and two electrodes facing the electrode are used.
FIG. 11 is a diagram illustrating waveforms according to a second modification of the first embodiment in cases where voltages with different frequencies are used.
FIG. 12 is a diagram illustrating waveforms according to the second modification of the first embodiment in cases where voltages with different phases are used.
FIG. 13 is a block diagram illustrating a liquid control apparatus according to a third modification of the first embodiment.
FIG. 14 is a graph showing sweeping of a voltage value, a current value, and a frequency according to a fourth modification of the first embodiment.
FIG. 15 illustrates the results of blood flow improvement after 30 minutes.
FIG. 16 illustrates the results of blood flow improvement over a four-week period.
FIG. 17 illustrates the result of ghost vessels being restored.
FIG. 18 illustrates the result of blood flow improvement in a mouse lower limb ischemia model.
FIG. 19 illustrates the result of decrease in viscosity of a contrast agent.
FIG. 20 illustrates the result of antibacterial activity on anthracnose causal fungi.
FIG. 21 illustrates the measurement result of particle diameters of water droplets.
FIG. 22 illustrates the improvement result in hydroponic cultivation of green leaf lettuce.
FIG. 23 illustrates the improvement result in perilla preservation.
FIG. 24 illustrates the improvement result of the edema rate of rheumatism.
FIG. 25 illustrates the effect of improving blood flow in a mouse model of lower limb ischemia.
FIG. 26 illustrates the effect of reducing the viscosity of a highly viscous agent.
FIG. 27 illustrates the effect of improving blood CPK in a heart transplanted mouse.
FIG. 28 illustrates the verification result of adverse reactions caused by electrical stimulation by radio frequency vibration.
FIG. 29 illustrates experimental conditions in FIG. 28.
FIG. 30 illustrates the improvement result of rigor mortis and blood coagulation of fish.
FIG. 31 illustrates a body decomposition preventing device.
FIG. 32 illustrates a blood coagulation suppressing device.
FIG. 33 illustrates the effect of suppressing mold of strawberries.
FIG. 34 illustrates the effect of suppressing Bacillus anthracis growth.
FIG. 35 illustrates the effect of improving masticatory balance by relaxing masseter tension.
FIG. 36 illustrates the measurement result of sacrum 1 (efficacy on bladder, ovary, and prostate).
FIG. 37 illustrates the measurement result of thoracic vertebra 12 (efficacy on kidney).
FIG. 38 illustrates the measurement result of thoracic vertebra 8 (efficacy on liver).
FIG. 39 illustrates the measurement result of thoracic vertebra 1 (efficacy on heart and lungs).
FIG. 40 illustrates the measurement result of cervical vertebra 3 (efficacy on throat and paranasal sinuses).
FIG. 41 illustrates the effect of enhancing the viability of human cultured cells.
FIG. 42 illustrates the observation result of peripheral blood mononuclear cells.
FIG. 43 illustrates a sleep improvement example 1.
FIG. 44 illustrates a sleep improvement example 2.
FIG. 45 illustrates an experiment on menstrual pain improvement.
FIG. 46 illustrates the effect of improving menstrual pain.
FIG. 47 illustrates the effect of improving a contrast agent.
FIG. 48 illustrates the effect of preventing mold and rotting.
FIG. 49 illustrates the effect of preventing fish rotting.
FIG. 50 illustrates the effect of maintaining freshness.
FIG. 51 illustrates the effect of preventing bread mold.
FIG. 52 illustrates a freckles/wrinkles improving device.
FIG. 53 illustrates a freckles/wrinkles improving effect example 1.
FIG. 54 illustrates a freckles/wrinkles improving effect example 2.
FIG. 55 illustrates a freckles/wrinkles improving effect example 3.
FIG. 56 illustrates an electrode structure example 1.
FIG. 57 illustrates an electrode structure example 2.
FIG. 58 illustrates an electrode structure example 3.
FIG. 59 illustrates an electrode structure example 4.
FIG. 60 illustrates an electrode structure example 5.
FIG. 61 illustrates an electrode structure example 6.
FIG. 62 illustrates an electrode structure example 7.
FIG. 63 illustrates an electrode structure example 8.
FIG. 64 illustrates an electrode structure example 9.
FIG. 65 illustrates extraction of green tea.
FIG. 66 illustrates extraction of kelp.
FIG. 67 illustrates extraction of broth.
FIG. 68 illustrates noodle aging.
FIG. 69 illustrates a water wave motion enhancement result 1.
FIG. 70 illustrates a water wave motion enhancement result 2.
FIG. 71 illustrates a water wave motion enhancement result 3.
FIG. 72 illustrates a water wave motion enhancement result 4.

### Description of Embodiments

A liquid control apparatus according to embodiments of the present invention will be described below with reference to the figures. The following embodiments are intended to illustrate a liquid control apparatus for embodying the technical concept of the present invention, and are not intended to limit the present invention to these embodiments. The present invention is equally applicable to any other embodiments within the scope of the appended claims. While the embodiments illustrate "moisture" such as free water and the like as liquid contained inside or on the surface of a target part, examples of the moisture contained inside or on the surface of an object according to the present invention are not limited to water, and can be applied to any liquids such as aqueous solution, blood, body fluid, chemicals, emulsion, oil, organic substance, ionic fluid, viscous fluid, non-viscous fluid, compressive fluid, noncompressive fluid, and any other liquids. The expression "moisture" may sometimes be used in the respective embodiments, but this is not intended to limit the liquid to water alone, and is widely applicable to any liquid.

### [First embodiment]

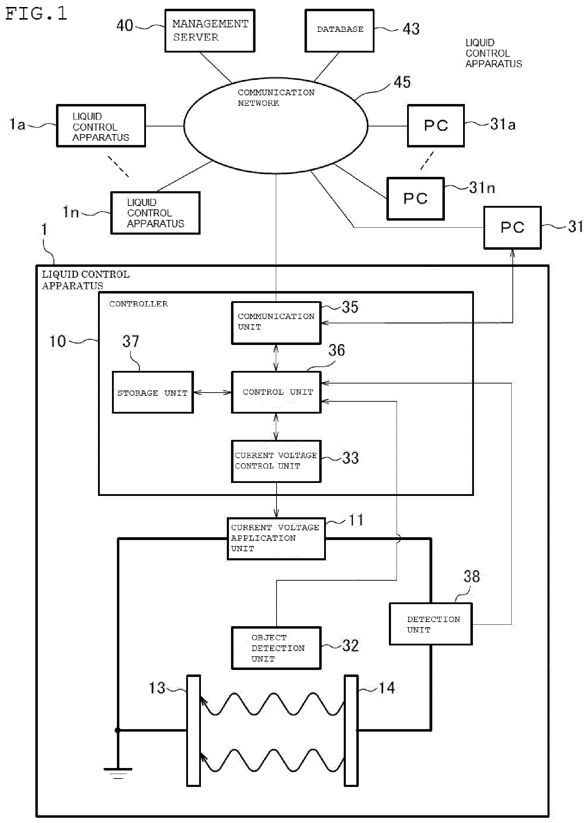

A liquid control apparatus according to a first embodiment will be described with reference to FIG. 1 to FIG. 19.

FIG. 1 is a conceptual diagram of a liquid control apparatus 1. The liquid control apparatus 1 includes a controller 10 and a pair of electrodes 13 and 14. The controller 10 includes a current voltage control unit 33, a control unit 36, a communication unit 35, and a storage unit 37. The electrodes 13 and 14 are supplied with at least one of DC voltage or AC voltage from a current voltage application unit 11 controlled by the current voltage control unit 33. The current and/or voltage applied to the electrodes 13 and 14 is detected by a detection unit 38 and fed back to the control unit 36. An object detection unit 32 (e.g., a camera) is also provided to detect the type and/or size of an object disposed in a space between the electrodes 13 and 14. In an actual circuit configuration of the controller 10, the current voltage application unit 11 and the detection unit 38 may be integrated with the controller 10. For example, the controller 10 and the current voltage application unit 11 may be integrated with each other and accommodated in a single housing.

The detection unit 38 may also be provided in the electrodes 13 and 14 so as to detect the state of generated electromagnetic waves. In this case, the detection unit 38 configured to detect the state of electromagnetic waves may be integrated with the electrodes 13 and 14. It is also possible to integrate a cable for the detection unit 38 with a cable for the electrodes 13 and 14, and in such a case, routing the cable would become easier. The electrodes 13 and 14 may also be used as a unit configured to detect the generation status of an electromagnetic field. In this case, although the detection unit 38 includes a current and/or voltage detection unit, the current voltage application unit 11 can also detect current and/or voltage. Therefore, the current and/or voltage detection unit of the detection unit 38 can be integrated with the current voltage application unit 11, and can further be integrally incorporated into the housing of a controller or the like. Moreover, when the current and/or voltage detection unit of the detection unit 38 detects data, values relating to generation of electromagnetic waves are superimposed on the data. Accordingly, the electromagnetic waves generated in the electrodes 13 and 14 can be detected from the detection data.

Although the object detection unit 32 can be integrated with the controller, the object detection unit 32 may alternatively be configured separately from the controller 10 in order to prevent enlargement due to addition of a camera or the like, and also for the convenience of the arrangement of the camera or the like. FIG. 1 illustrates an example where a pair of electrodes 13 and 14 are provided. However, the present embodiment is not limited to this, and the number of the electrodes may be any number as long as the number is one or more, and various shapes can be adopted as will be described later. For example, when the whole of electrodes 13 and 14 is a single electrode, an object may be disposed so as to face the electrode. When the object detection unit 32 detects the type, the state, the size or the like of an object disposed to face the electrodes 13 and 14, the control unit 36 calculates a control command value in response to the detection of the object. According to the control command value, the current voltage control unit 33 controls the current voltage application unit 11 to control current and voltage applied to the electrodes 13 and 14, so that at least one of an electric field, a magnetic field, an electromagnetic field, or electromagnetic waves is applied to the object disposed to face the electrodes. Hereafter, expressions such as "an electromagnetic field is applied", "irradiated with electromagnetic waves", or "an electric field is applied" are used in certain parts, but these expressions are not intended to limit the electromagnetic field, the electromagnetic waves, and the electric field. Rather, the expressions include the magnetic field, the electromagnetic field, or the electromagnetic waves. At this time, the electromagnetic field applied to an object is controlled, by the control unit 36, to be in a desired state, which depends on the control command that has been calculated by the feedback control based on the detection value from the detection unit 38. A voltage including at least an AC component is applied to the electrodes 13 and 14, and an electric field as well as a magnetic field is generated from the electrodes 13 and 14 in response to the applied voltage. As used herein, an electromagnetic field includes at least one of an electric field or a magnetic field. Since the electrodes 13 and 14 have the function of an antenna, electromagnetic waves are generated from the electrodes 13 and 14 in response to the applied voltage. The electric field, the magnetic field, the electromagnetic field, and the electromagnetic waves generated from the electrodes 13 and 14 correspond to the applied voltage and current and are associated with each other, and can be calculated by computation. Therefore, in the present embodiment, the strength of the electric field, the magnetic field, the electromagnetic field, and the electromagnetic waves generated from the electrodes 13 and 14 is quantitatively evaluated based on the electric field strength. Only a DC component can be applied to the electrodes 13 and 14. Also in this case, it is possible to reduce the interfacial tension of a liquid, to control a pearl-chain structure, and to control emulsion, for example. A DC component and an AC component may be applied to the electrodes 13 and 14. However, in the present embodiment, only an AC component is applied, for example, an AC voltage of 50 KHz and 100 V is applied, as an example, to facilitate evaluation between the examples. Since electric current is also supplied by applying a voltage to the electrodes 13 and 14, targets to be controlled by the controller 10 are voltage value, current value, frequency, and phase, and the controller 10 can set a target value of each physical quantity. In the present embodiment, the voltage value and the frequency will be described as the target values in order to provide consistent explanation in the examples. However, the target values for other physical quantities can also be set as a matter of course.

The communication unit 35 communicates with a management server 40, a database 43, other PCs 31a to 31n, and other liquid control apparatuses 1a to 1n to receive control parameters and/or control values from the management server 40. The storage unit 37 stores programs, and the control unit 36 including CPU and the like can operate using the programs stored in the storage unit 37. The control unit 36 controls the current voltage application unit 11, based on the control parameters and/or the control values received from the management server 40, via a current voltage control unit 33 built in the controller 10, to control current and/or voltage applied to the electrodes 13 and 14. The programs can be rewritten from the management server 40 via the communication unit 35. The programs may be stored in a removable memory such as a flash memory, so that the programs for the controller 10 can be rewritten by using the removable memory. Furthermore the programs can be set or rewritten by using a man-machine interface 31 connected to the communication unit 35.

The management server 40 includes the functions, such as updating or performing maintenance of the program of the controller 10, surveying or monitoring the use status of the controller 10, collecting or analyzing position information or environmental information on the controller 10, collecting or analyzing improvement request information from the controller 10, performing maintenance of the controller 10, collecting control information from the controller 10 and/or information from the database 43, generating and providing learning model information for the controller 10, providing control information based on the learning model information, or providing control parameters of the controller 10.

For surveying or monitoring the use status of the controller 10, the management server 40 can constantly collect the control information on the controller 10, and therefore the management server 40 has the functions of constantly discerning the use status of the controller 10 to perform surveillance or monitoring. Here, the monitoring function includes discerning the state of a user using the liquid control apparatus 1 corresponding to the controller 10 by analyzing how and how much the user uses the liquid control apparatus 1 in which time band. For example, when a certain restaurant uses the liquid control apparatus, the management server 40 can discern a business status, a visitor status, a cooking status, a preparation status, or other statuses, of the restaurant. In the case of an individual user, the management server 40 can discern a life status, a safety status, or the like of the individual as a user, based on the use status of the liquid control apparatus. Therefore, when the management server 40 determines that there is an abnormality in the liquid control apparatus 1, it is possible to notify the abnormality to the pertinent user and to also notify the abnormality to registered contacts and emergency contacts such as police and fire departments.

For collecting or analyzing position information or environmental information on the controller 10, the management server 40 can collect, from the controller 10, position information, climate information, regional information, or the like about the place where the liquid control apparatus 1 is disposed, and the management server 40 can discern the position information or the environmental status about the place where the liquid control apparatus 1 is used. For example, the management server 40 can thus transmit control information corresponding to the operating environment or the like to the controller 10.

For collecting or analyzing improvement request information from the controller 10, the management server 40 can collect, from the controller 10, the improvement request information that is input into the controller 10 from the PC 31, or can collect the improvement request information, which is input from the PC 31 that communicates with the controller 10, directly from the PC 31. The improvement request information includes information such as requests for improvement of the liquid control apparatus 1 from users, evaluations of the control results, and request items. These pieces of improvement request information are analyzed in the management server 40 and used to set control parameters of each liquid control apparatus 1.

For the maintenance of the controller 10, the management server 40 can collect and survey information such as an operating status of the controller 10, the status of programs, the status of apparatus control parameters, the information stored in the storage unit 37, the statue of devices, and the environmental status of the liquid control apparatus, to perform maintenance of the programs of the controller 10, the control parameters, detection information, control result information, various setting parameters, and the content of stored data in the storage unit. The content of maintenance includes, but is not particularly limited to, setting or update of programs, setting or update of control parameters and setting parameters, and setting or update of the learning model described later. Various operating status information, control information or the like, collected from the controller 10 and/or the information collected from the database 43 are used for deep learning in the management server 40 as described later. Learning model information trained by deep learning and/or control parameters or the like calculated by the learning model are provided to the controller 10 of each of the liquid control apparatuses 1.

The controller 10 is connected to the object detection unit 32 configured to detect the type and/or the state of an object disposed between the electrodes. Thus, the controller 10 recognizes the type and/or the state of the object and controls the current voltage application unit 11 incorporated therein, to achieve output voltage and/or output current suitable for the type, state, size and the like of the object. The current voltage application unit 11 has a function that is at least one of DC-DC conversion, DC-AC conversion, AC-DC conversion, and AC-AC conversion as described later. For example, the current voltage application unit 11 can be a variable voltage variable frequency (VVVF) inverter. The current voltage application unit 11 can apply, to the electrodes 13 and 14, voltage/current that is DC voltage/current superimposed on AC voltage/current.

The man-machine interface 31 communicates with the controller 10, and thus a user can set and operate the controller by inputs from the man-machine interface 31. Examples of the man-machine interface 31 include a display, a touch panel, a keyboard, and a mouse. When the controller 10 is operated by a smartphone, a mobile phone, a tablet terminal, a mobile terminal, or a personal computer such as a laptop computer (hereinafter, the man-machine interface 31 may be simply referred to as "PC"), the smartphone or the like can also have the functions of the man-machine interface 31, the communication unit 35, and the like.

By communicating with the controller 10, the PC 31 can perform setting of the control parameters, update of the control program or the like, and can also survey the status, such as an operational status and a control status of the controller 10. When the PC 31 and the controller 10 are connected via a communication network, the PC 31 can perform setting, operation, surveillance and the like of the controller 10 from a remote location.

The liquid control apparatus 1 is connected to an external power supply (not illustrated). The external power supply may be an AC power supply or a DC power supply. The DC power supply may be a battery including a primary cell, a secondary cell, and the like. If the liquid control apparatus 1 can be moved, conveyed, or carried around, it is convenient to use a battery as the external power supply 39 in terms of securing power supply.

The controller 10 performs feedback control on at least one of values of current and/or voltage applied to the electrode, their frequencies, and their phases on the basis of a detection signal from the detection unit 38, which will be described later.

A processing target object is disposed between the electrodes 13 and 14. The processing target object is not particularly limited as long as the object is at least one of solid, liquid, and gas. Various objects can be the processing target as will be described later.

The controller 10 is connected to a communication network 45 via the communication unit 35 or via the PC 31, and the communication network 45 is connected to the management server 40, the database 43, liquid control apparatuses 1a to 1n, and PCs 31a to 31n. The management server 40 can collect, from the controller 10, the control information including detection data from the object detection unit 32 and/or the detection unit 38 via the communication unit 35 or the PC 31. The management server 40 uses data from the database 43, information from each of the liquid control apparatuses 1 and 1a to 1n, and information from each of the PCs 31 and 31a to 31n, to calculate a learning model for obtaining control parameters of the controller by machine learning such as deep learning, for example. The management server 40 transmits the trained learning model or calculation parameters obtained by the trained model, to the controller 10 of each of the liquid control apparatuses 1 and 1a to 1n. The controller 10 uses the learning model in the control unit 36 to calculate parameters adaptive to an object disposed to face the electrodes 13 and 14, based on the detection data from the object detection unit 32 and/or the detection unit 38, or uses adaptive parameters transmitted from the management server 40 in the control unit 36 to perform arithmetic calculation for the current voltage control unit 33 to control the current and/or voltage to be applied from the current voltage application unit 11 to the electrodes 13 and 14. The storage unit 37 stores a control program. The controller 10 is controlled based on the control program. Since the control program is rewritable from the management server 40, it is possible to update and upgrade the program in a timely manner. It is also possible to set, change, and update the control program from the PC 31. In addition, various control parameters of the controller 10 can be set and changed by the PC 31.

### [Electrode]

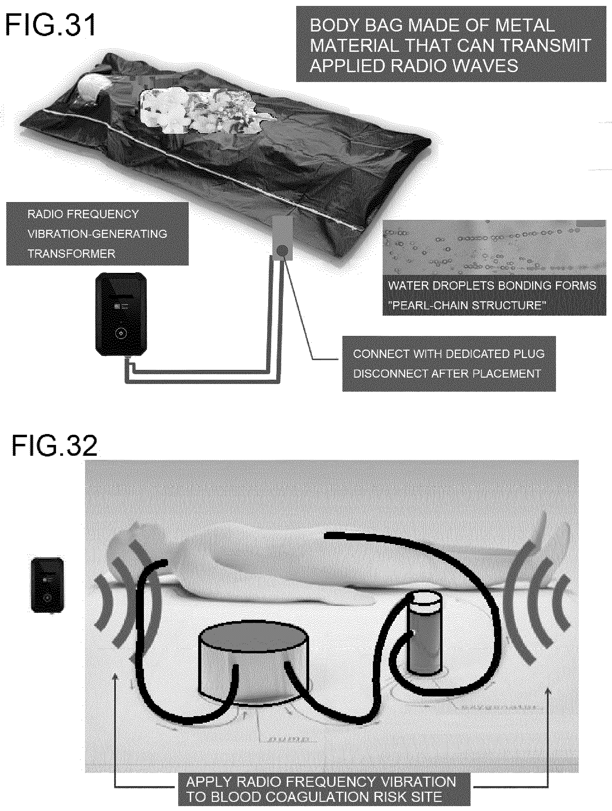

In FIG. 1, the pair of electrodes 13 and 14 are illustrated as electrodes in a form of a plate as an example. However, the electrodes 13 and 14 are not limited to the plate form, and may be in a form of a foil, a film, or a layer, and can also have various shapes such as a rod shape, a spherical shape, a semi-spherical shape, a cylindrical shape, a semi-cylindrical shape, a conical shape, a semi-conical shape, a substantially L shape, a substantially rectangular U shape, a polygonal shape, a polygonal columnar shape, a polygonal pyramid shape, a curved shape, or a bent shape (see FIG. 32 to FIG. 39 and the like referenced later). The electrodes 13 and 14 in a form of a foil and a film can have an extremely small thickness, and thus are space saving. Furthermore, such electrodes 13 and 14 can have a shape freely designed and are light weight. Thus, such electrodes 13 and 14 can be easily installed. The electrode of a layer form includes a thin-film electrode provided to be stacked on a predetermined substrate, for example.

The shape of the electrodes 13 and 14 is not limited to a flat plate shape, and may be any shape. When the electrodes 13 and 14 in a form of a foil are used, the electrodes can be shaped as desired to conform to the shape of their installed locations. For example, the electrodes can be in a form of a curved shape.

The electrodes 13 and 14 may be provided with a plurality of through holes. With the plurality of through holes provided, the electrodes can have improved characteristics for generating electromagnetic waves and air permeability, and can also ensure visibility through the electrodes. The holes can have various shapes such as a circular shape, an elliptical shape, a polygonal shape, a slit shape, a linear shape, or a combination of these. For example, hexagonal holes may be provided.

The material of the electrodes 13 and 14 is not particularly limited as long as the material has conductivity. For example, conductive metal such as copper, iron, stainless steel, aluminum, titanium, gold, silver, and platinum, an alloy of these metals, a conductive material such as a conductive oxide or a conductive glass, or the like is used. The electrodes 13 and 14 may have surfaces coated with an insulating material. For example, when the electrodes are provided to a fryer, an inner surface of the fryer and the electrodes are insulated from each other. For example, when the electrodes are provided on an inner surface of a container, the inner surface of the container and the electrodes are preferably insulated from each other. The pair of electrodes 13 and 14 may be made of different materials. For example, the material of the electrode 13 may be stainless steel and the material of the electrode 14 may be titanium. Furthermore, combinations between stainless steel and aluminum, between stainless steel and copper, and the like may be employed. By changing the materials of the electrodes 13 and 14, the characteristics of the electromagnetic waves generated from the electrodes can be adjusted. In such a case, the characteristics of the electromagnetic waves can also be adjusted by exchanging the materials of the electrode 13 and the electrode 14. As will be described later, the number of electrodes is not limited to one pair, and may be set as appropriate to be one, three or more, two pairs or more, or the like. Also in these cases, the characteristics of the electromagnetic waves generated from the electrodes can be adjusted by appropriately selecting the material of each electrode. For example, the characteristics of the electromagnetic waves generated from two pairs of electrodes can be adjusted with one pair of electrodes made of stainless steel and the other pair of electrodes made of copper. The electrodes 13 and 14 generate at least one of an electric field, a magnetic field, an electromagnetic field, electromagnetic waves, sound waves, and ultrasonic waves. When only the sound waves or the ultrasonic waves are generated, the material of the electrodes 13 and 14 is not limited to a conductive material. For example, nonconductive material such as resin may be used.

A dedicated housing may be provided for installation of the liquid control apparatus 1. However, this should not be construed in a limiting sense, and the liquid control apparatus 1 may be installed in an existing housing, for example. The existing housing in which the liquid control apparatus 1 can be installed can be selected from various housings including: a refrigerator; a freezer; a refrigerating warehouse; a freezer warehouse; a storage house; a warehouse; a refrigerator car; a freezing car; a cooler box; a container for transport; a container for storage; a showcase; a shelf; a drawer; a fryer; a cultivation container (for hydroponics, etc.); a fuel tank; a PC; a mobile phone; a chair bed; furniture; bedding; home appliances; various manufacturing equipment in a factory; processing equipment; medical equipment; health equipment; beauty equipment; cooking equipment; polishing equipment; vehicles; semiconductor cleaning equipment; and equipment for controlling vapor resulting from cooling during a refining step; a baking step; and a drying step.

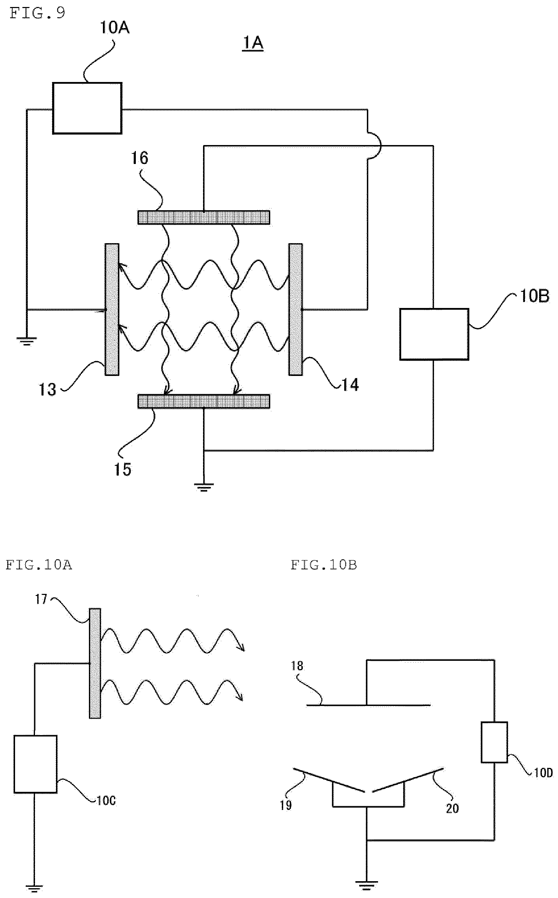

In a case of the refrigerator, for example, the pair of electrodes 13 and 14 can be arranged along a ceiling surface and a bottom surface in the refrigerator, along side wall surfaces facing each other, along the ceiling surface, a tray, and the bottom surface, along the ceiling surface, the bottom surface, and the side surface, or along an inner surface of the door and a back side surface. In a case of the fryer, for example, the electrodes are provided along both inner side surfaces of an oil container. Thus, the pair of electrodes 13 and 14 may be in any arrangement as long as they face each other. The pair of electrodes do not need to be arranged in parallel, and may be in an orthogonal positional relationship. Thus, the electrodes can be in any arrangement as long as the space to accommodate the processing target object can be provided between the electrodes. The number, arrangement, and shape of the electrodes are not particularly limited. The number of electrodes is not limited to one pair, and may be one, three or more, or two pairs or more, as can be seen in FIGS. 9, 10, and 23 to 30 described later, for example.

The liquid control apparatus 1 is not limited to the installation in a housing, and can be disposed at any location as long as the pair of electrodes 13 and 14 can be disposed. For example, any location such as a shelf or a wall can be used as long as the pair of electrodes 13 and 14 can be disposed to face each other. Furthermore, a screen shaped member can be used to fix the electrodes 13 and 14, for example. For example, a chopping board may be used. The number of electrodes is not limited to one pair, and may be one, three or more, or two pairs or more, as can be seen in FIGS. 9, 10, and 23 to 30 described later, for example.

### [Voltage applied to electrode]

The controller 10 can apply, to the pair of electrodes 13 and 14, at least any one of the DC component voltage and the AC component voltage. The DC component voltage is not particularly limited, and can be adjusted between 0 V and 7000 V, for example, can be adjusted between 0 V and 5000 V, for example, can be adjusted between 0 V and 2000 V, for example, can be adjusted between 0 V and 500 V, for example, can be adjusted between 0 V and 200 V, for example, can be adjusted between 0 V and 100 V, for example, can be adjusted between 5 V and 20 V, for example, and can be adjusted between 10 V and 15 V, for example. The polarity may be positive or negative. Thus, when both positive and negative polarities are taken into consideration in the example of the adjustment between 0 V and 200 V, the voltage can be adjusted between -200 V and +200 V. When both positive and negative polarities are taken into consideration, the voltage can be adjusted between -7000 V and +7000 V, for example, can be adjusted between -5000 V and +5000 V, for example, can be adjusted between -2000 V and +2000 V, for example, can be adjusted between -500 V and +500 V, for example, and can be adjusted between -200 V and +200 V, for example. A DC power supply or an AC power supply may be used for the power supply voltage. When the DC power supply is used, a battery featuring excellent portability can be used as the power supply, for example. On the other hand, when the AC power supply is used, a commercial power supply can be used, for example, meaning that the power supply can easily be ensured. The power supply voltage may be AC voltage of 100 V to 400 V, for example, DC voltage of 5 V to 20 V, for example, and DC voltage of 10 V to 15 V, for example. When expressed in terms of the spatial electric field, it can be adjusted between -7000 V/cm and +7000 V/cm, for example, can be adjusted between -5000 V/cm and +5000 V/cm, for example, can be adjusted between -2000 V/cm and +2000 V/cm, for example, or can be adjusted between -500 V/cm and +500 V/cm or can be adjusted between -200 V/cm and +200 V/cm, for example.

At least the DC component voltage is applied to the pair of electrodes 13 and 14. Thus, only the DC component voltage may be applied with the AC component voltage set to be 0 V, for example.

The orientation of the DC component voltage may be positive (+) or negative (-). In the present embodiment, the orientation of the DC component voltage is positive when the potential of the electrode 14 is higher than the potential of the electrode 13 (ground potential), and is negative when the potential of the electrode 14 is lower than the potential of the electrode 13. The effect of improving the property of the object is obtained with the positive DC component voltage and with the negative DC component voltage.

To the pair of electrodes 13 and 14, the AC component voltage can be applied in addition to the DC component voltage. Furthermore, only the AC component voltage may be applied with the DC component voltage set to be 0 V. The frequency of the AC component voltage is not particularly limited, and can be adjusted between 0 and 1 MHz, for example, can be adjusted between 0 Hz and 500 kHz, for example, can be adjusted between 0 Hz and 200 kHz, for example, and can be adjusted between 5 Hz and 100 kHz, for example. Depending on the target, the voltage may be used in a lower frequency band or in a higher frequency band. In the lower frequency band, the frequency band of about 5 Hz to 500 Hz may be used, for example.

The voltage of the AC component voltage is not particularly limited, and the spatial electric field/cm between peaks can be adjusted between 0 and 7000 Vpp/cm, for example, can be adjusted between 0 and 5000 Vpp/cm, for example, can be adjusted between 0 and 2000 Vpp/cm, for example, can be adjusted between 0 and 500 Vpp/cm, for example, and can be adjusted between 0 and 200 Vpp/cm, for example. For example, the voltage between 0 and 7000 V can be supplied to the electrodes. The voltage between 0 and 5000 V can be supplied, for example, the voltage between 0 and 2000 V can be supplied, for example, the voltage can be adjusted between 0 and 500 V, for example, and can be adjusted between 50 and 250 V, for example. For example, in the case of a pair of electrodes, the electrode-to-electrode voltage between 0 and 7000 V can be supplied, the voltage can be adjusted between 0 and 5000 V, for example, can be adjusted between 0 and 2000 V, for example, can be adjusted between 0 and 500 V, for example, and can be adjusted between 50 V and 250 V, for example.

Note that the application of the DC component voltage results in high effect of improving the property of the object. Still, such an effect can be obtained also with the application of the AC component voltage only with the DC component voltage being 0 V. Hereinafter, for the AC voltage component, in principle, [Vpp] is used as a unit to represent a peak-to-peak voltage value, and [V] is used to represent the effective value of the voltage.

As described above, the voltage of the external power supply may be DC voltage or AC voltage, and the external power supply may be an AC power supply or a DC power supply. For example, a commercial power supply can be used as the AC power supply. For example, the DC power supply may be a battery including a primary cell and a secondary cell. Furthermore, various batteries such as 12-V battery and a dry cell can be used.

For adjusting the voltage value of the DC component voltage in the controller 10, a method of performing voltage control on the DC power supply by a DC-DC converter, a method of performing the voltage control by the DC-DC converter when the AC power supply is rectified by an AC-DC converter or after the AC power supply has been rectified, and the like can be employed. The voltage value and the frequency of the AC component voltage can be controlled in the controller 10 with methods including: a method of controlling the DC power supply with a DC-AC converter (inverter); a method of rectifying the AC power supply with an AC-DC converter and then controlling the resultant power supply with a DC-AC converter (inverter); and a method of controlling the AC power supply with an AC-AC converter.

When the target voltage value of the DC component voltage is equal to the power supply voltage of the DC power supply, the power supply voltage of the DC power supply may be directly used as the DC component voltage. Similarly, when the target voltage and the target frequency of the AC component voltage are equal to the power supply voltage of the AC power supply, the power supply voltage of the AC power supply may be directly used as the AC component voltage.

The DC component voltage and the AC component voltage are added, that is, the DC component voltage is added as offset voltage to the AC component voltage, and the resultant voltage is applied between the pair of electrodes 13 and 14. For example, when the AC component voltage is controlled through power conversion using the DC-AC converter, the DC component voltage may also be controlled.

The AC component of voltage applied to an electrode includes sinusoidal voltage. However, the AC voltage component according to the present embodiment is not limited to sinusoidal voltage, and includes voltage of any waveform such as rectangular waveforms or PWM waveforms. The sinusoidal wave and the rectangular wave are not limited to the sinusoidal wave and the rectangular wave in a strict sense, and indicate waveforms taking noise, distortion, and the like into consideration. The DC component of the voltage applied to the electrode is not limited to constant voltage, and DC component voltage varying over time may also be used.

A voltage controlling unit in the controller 10 may be any of an analog circuit, a digital circuit, and a circuit obtained by combining analog and digital circuits. For example, sinusoidal voltage may be generated by the analog circuit, or equivalent sinusoidal waves can be generated with the PWM waveform. For example, as a circuit that generates voltage with a rectangular waveform, a digital circuit may be used, and an analog circuit can also be used.

The controller 10 controls voltage or current to be applied to the electrodes 13 and 14 to be at least one voltage or current selected from the group consisting of:
(1) voltage or current that reduces an interfacial tension of an object;
(2) voltage or current that prevents food and drink or a liquid from becoming rotten;
(3) voltage or current that contributes to at least one of fresh flower preservation, drinking water preservation, hydroponic cultivation promotion or environmental improvement, germination rate improvement, hatching rate improvement, aquarium antifouling or purification, water quality improvement, rock sugar growth promotion, fuel reforming, or fuel efficiency improvement;
(4) voltage or current that contributes to at least one of preservation of blood or blood components, improvement in symptoms of diabetes, improvement in symptoms of chronic kidney disease, improvement in artificial dialysis, improvement of blood flow, revascularization, improvement in symptoms of peripheral neuropathy, improvement in symptoms of arthropathy or rheumatism, organ preservation, antitumor effect, improvement in symptoms of ischemia, improvement in symptoms of lymphatic edema, improvement in symptoms of decubitus ulcer, necrosis prevention or improvement, improvement in symptoms of circulatory diseases, or infection control;
(5) voltage or current that improves efficiency of at least one of charging or discharging of a capacitor, a generator, or a power transmission facility;
(6) voltage that promotes emulsification or generation of an emulsion or voltage or current that achieves a longer emulsion state maintained period;
(7) voltage or current that increases the effect of an air purifier or an ionizer;
(8) voltage or current that separates atoms or molecules into types;
(9) voltage for controlling temperature or humidity in a space;
(10) voltage or current that separates moisture from at least one of bacteria, germs, viruses, or microorganisms; and
(11) voltage or current that facilitates chemical polishing, mechanical polishing, chemical-mechanical polishing, or magnetic polishing.

### [Control by controller]

The liquid control apparatus 1 is driven by the controller 10 and an electric field is generated between the pair of electrodes 13 and 14. In this case, the electrodes 13 and 14 function as an antenna, and an electromagnetic field is generated with electromagnetic waves radiated between the electrodes 13 and 14. The electrodes 13 and 14 may also be vibrated by an electric, magnetic, or mechanical unit, so that sound waves and/or ultrasonic waves can be generated between the electrodes. An example of the unit that can be used for generating the sound waves and/or ultrasonic waves between the electrodes includes a piezoelectric element. Thus, at least one of an electric field, a magnetic field, an electromagnetic field, electromagnetic waves, sound waves, and ultrasonic waves is generated between the electrodes 13 and 14. With the sound waves and/or ultrasonic waves used in addition to the electric field, magnetic field, electromagnetic field, or electromagnetic waves, a higher effect of improving the characteristics of an object can be achieved.

The controller 10 performs feedback control on at least one of the values of the current and/or the voltage applied to the electrode, the frequency of the current and/or the voltage, and the phase of the current and/or the voltage, based on a detection signal from the detection unit 38. The detection unit 38 includes at least one of a voltage sensor configured to detect the voltage applied to the electrode, a current sensor configured to detect the current applied to the electrode, a frequency sensor configured to detect the frequency of the voltage and/or current applied to the electrode, a phase sensor configured to detect the phase of the voltage and/or current applied to the electrode, a magnetic field sensor configured to detect a magnetic field between the electrodes 13 and 14, an electric field sensor configured to detect an electric field between the electrodes 13 and 14, a sound wave sensor configured to detect the magnitude and/or the frequency of the sound waves between the electrodes 13 and 14, and an ultrasonic wave sensor configured to detect the magnitude and/or the frequency of the ultrasonic waves between the electrodes 13 and 14.

The electrode may be provided with the sensor. The electrode itself can be used as the sensor. When the electrode is provided with a sensor, wires (for example, two wires) for the sensor are required in addition to the power supply line for supplying power to the electrode. The number of wires between the controller 10 and the electrode is smaller the better. In this context, the power supply line and the sensor lines may be combined into a single cord. The single cord used in such a case is at least covered with an insulating material. Furthermore, the cord preferably also has durability and heat resisting property. Furthermore, considering the application in a freezer chamber, the cord is preferably capable of withstanding cold temperatures. For example, considering the application in a fryer, the cord is required to have durability and heat resisting property in addition to insulating property, and thus may be coated using a material such as fluorine resin, for example. When a pair of electrodes are provided, only one of the electrodes may be provided with the sensor. Alternatively, both electrodes may be provided with sensors, so that the sensor provided to one of the electrodes can detect a physical quantity generated by the other electrode. When three or more electrodes are provided, the sensor may be provided to at least one of the electrodes. However, this should not be construed in a limited sense, and the sensor may be provided to a plurality of electrodes or to all of the electrodes.

At least one of the control target values in the controller 10, which are the current value, the voltage value, their frequencies, and their phases, is set in accordance with the type and/or the state of the target object. The control target value may be remotely set through an unillustrated communication device. The control parameters and/or the control amount of the controller 10 can also be remotely controlled. Thus, the controllers 10 of a plurality of the liquid control apparatuses 1 can be collectively managed by the server 40 at a remote location, whereby the controllers 10 can be appropriately controlled. However, the control mode for the controller 10 is not limited to the remote control from the server 40. The controller 10 of each liquid control apparatus 1 can be individually controlled with the control target value and/or the control parameter directly set to each controller 10, for example.

The controller 10 is provided with the storage unit 37 storing a control program. The controller 10 is controlled based on this control program. The control program is rewritable through communications or the storage medium, and thus a program version can be upgraded by updating the program as appropriate. The controller 10 and the server 40 can communicate with each other. Thus, the storage unit 37 stores the control parameter, the control amount, the control program, or various setting values transmitted from the server 40. The control program can be stored in any appropriate storage medium.

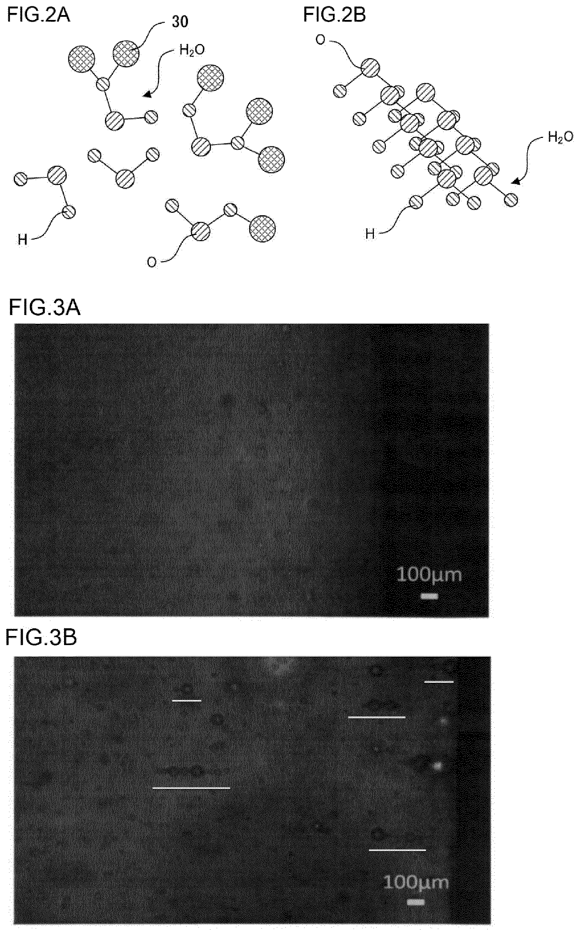

FIG. 2 is a schematic view of water molecules. FIG. 2A illustrates water molecules in a freely moving state, and FIG. 2B illustrates water molecules in a pearl-chain structure.

The target object (a food product such as meat, fish, and vegetable, beverage, animal/plant cells, oil, and the like, for example) contains water molecules as moisture such as free water.

Generally, water molecules (H2O) are randomly arranged as illustrated in FIG. 2A. Thus, hydrogen atoms H may take in active oxygen 30 or cause hydrogen bond. This results in larger and thus less active water molecules. Then, oxidation of the water molecules starts.

The electric field generated between the pair of electrodes 13 and 14 causes the water molecules to be arranged in a single orientation. This is because, in the water molecules, oxygen atoms O with strong attractive force for electrons become slightly negative and hydrogen atoms H, which are likely to emit electrons, become slightly positive, and thus the atoms are oriented in the respective directions toward the electric field between the pair of electrodes 13 and 14.

When the controller 10 causes the AC component voltage to be generated, the water molecules changes their orientation in an alternating manner. Specifically, the water molecules change their orientations at a frequency that is the same as that of the AC component voltage to be in a state as if they are vibrating. As the water molecules repeatedly vibrate, as illustrated in FIG. 2B, the hydrogen bonding with the active oxygen 30 or other components is released. Thus, the water molecules are gradually finely grained and arranged.

The same applies to water particles (fine water drops) as moisture such as free water in the object. Thus, the electric field between the pair of electrodes 13 and 14 causes the water particles to be attracted to each other, whereby the pearl-chain structure is achieved.

The DC component voltage applied between the pair of electrodes 13 and 14 involves a force component causing the water molecules to be arranged along the orientation of the electric field generated by the DC component voltage. Thus, the regular arrangement of the water molecules can also be achieved by applying only the DC component voltage between the pair of electrodes 13 and 14. Application of the AC component voltage to the DC component voltage results in the water molecules changing their orientation at a frequency that is the same as that of the AC component voltage, and involves the force component causing the water molecules to be arranged in a single direction. Thus, the movement of the water molecules to be regularly arranged is facilitated. The same applies to the state of water particles. Specifically, the electric field between the pair of electrodes 13 and 14 causes the water particles as the moisture such as free water to be attracted to each other, whereby the pearl-chain structure is achieved.

When the voltage applied between the pair of electrodes 13 and 14 includes no DC component voltage, the water molecules change their orientation at a frequency that is the same as that of the AC component voltage to be in the vibrating state. As the water molecules repeatedly vibrate, the hydrogen bonding with the active oxygen 30 or other components is released. Thus, the water molecules are gradually finely grained and arranged. This effect of the AC component voltage in the case where the voltage applied between the pair of electrodes 13 and 14 includes no DC component voltage similarly applies to the state of the water particles. Specifically, the electric field between the pair of electrodes 13 and 14 causes the water particles as the moisture such as free water to be attracted to each other, whereby the pearl-chain structure is achieved.

The sound waves or the ultrasonic waves have an effect of vibrating the water molecules. Thus, application of DC component voltage and/or AC component voltage between the pair of electrodes 13 and 14 with the sound waves and/or ultrasonic waves at a predetermined frequency and with a predetermined intensity generated between the electrodes increases the effect of facilitating the movement of the water molecules to be arranged. When the water molecules are vibrated by the predetermined sound waves and/or ultrasonic waves, the water molecules can be aligned even if no voltage is applied between the electrodes.

Since an arrangement direction of water molecules or water particles is along the direction of application of the electromagnetic field, it is possible to control the arrangement direction of water molecules or water particles by controlling the electromagnetic field to be applied. For example, the electromagnetic field applied by a pair of electrodes 13 and 14 is in a constant direction. However, an electromagnetic field is applied from each of two orthogonal pairs of electrodes (the electrodes 13 and 14, and electrodes 15 and 16) in FIG. 9 described later, so that controlling the current or voltage applied to each electrode can adjust the direction of the electromagnetic field generated between each electrodes, in addition to the strength of the electromagnetic field. This makes it possible to control the arrangement direction of water molecules or water particles.

Water can be classified into "bonded water" and "free water". The bonded water is in a stable state due to hydrogen bonding with other components. On the other hand, the free water is in a freely movable state corresponding to a fresh and moist state when the object is a food product. However, molecules of the free water are likely to bond with other components, meaning that the food containing free water is perishable. Specifically, the food product is perishable as a result of free water bonding with germs, viruses, microorganisms, or active enzyme. Also in the bonded water state, the food product could be perishable, because bonded water turns into free water due to elapse of time, temperature rise, and dry environment and cell components that has been hydrogen-bonded may partly be picked up by the food product. In view of this, a bonded state where free water is in the pearl-chain structure (this is distinguished from the "bonded water state" described above) or a state of bonding to other cell and the like is achieved to enable the freshness to be maintained.

The liquid control apparatus 1 according to the present embodiment is expected to enable the water molecules to be in the pearl-chain structure so that free water bonding is achieved to establish a structure as stable as that of the bonded water. Specifically, the water molecules regularly arranged by the liquid control apparatus 1 according to the present embodiment do not bond with other components while being held in the object, whereby a food product can be maintained to be in a fresh and moist state.

Thus, with the liquid control apparatus 1 according to the present embodiment installed in a container, the arrangement of free water in an object in the container can be controlled. Thus, when the object is a food product, a medicine, or a cell, the freshness of the food product, the medicine, or the cell can be maintained. For example, the liquid control apparatus 1 may be used as a transportation container, so that a food product can be transported for a longer distance with the freshness maintained compared with conventional cases. The container may be a styrofoam container or the like, and a transportation container can be formed by attaching the liquid control apparatus 1 according to the present embodiment to an existing styrofoam container.

Once the water molecules are regularly arranged by the liquid control apparatus 1 according to the present embodiment, the water molecules are maintained to be in the regularly arranged state for few days to several tens of days. Thus, when the object is a food product, a medicine, or a cell, the freshness of the food product, the medicine, or the cell can be maintained even when the object is relocated and stored in a different container after the pearl-chain structure state of free water has been achieved by the liquid control apparatus 1 according to the present embodiment.

When predetermined voltage is applied to the electrodes 13 and 14, the water molecules in moisture of an object are electrically aligned, to be oriented in substantially the same direction (orientation of the electric field). With the water molecules aligned, the conductivity of the object increases. The water molecules can be aligned also when the object is liquid. Thus, the conductivity can be increased even when the object is pure water, for example. The water molecules slightly vibrate at a predetermined frequency in an electric field, and thus do not crystallize at a temperature close to 0°C.

When predetermined voltage is applied to the electrodes 13 and 14, the hydrogen bonding between water molecules in the object is suppressed (reduced), and thus physiological water can be obtained, for example. Fine bubbles such as microbubbles, micro-nano bubbles, nanobubbles, or the like may be added to this water, so that more functional water can be obtained. Such improvement of the function of liquid achieved by an electric field and fine bubbles is not limited to water, and can be achieved for a solution, emulsion, oil, and the like, for example.

When predetermined voltage is applied to the electrodes 13 and 14, hydration of water molecules in the moisture in the object is promoted. For example, deterioration of the object can be suppressed with proteins and the like included in the object hydrated to result in a state where the proteins and the like bond with the water molecules to be surrounded by the water molecules.

FIG. 3 is a photomicrograph of free water. FIG. 3A shows a state of the free water before application of an electric field. FIG. 3B shows a state of the free water after the application of the electric field. As illustrated in FIG. 3B, with free electrons after the application of the electric field, the pearl-chain structure of the water particles can be seen in portions marked with white underlines. On the other hand, as illustrated in FIG. 3A, in the free water before the application of the electric field, no pearl-chain structure of the water particles can be found. Thus, it is confirmed in FIG. 3 that the liquid control apparatus 1 according to the present embodiment can achieve the pearl-chain structure state of the free water. While FIG. 3 shows the pearl-chain structure of aqueous phase in oil phase, it is also possible to perform the similar control of liquid pearl-chain structure through control of the electric field to be applied, even between other types of liquids.

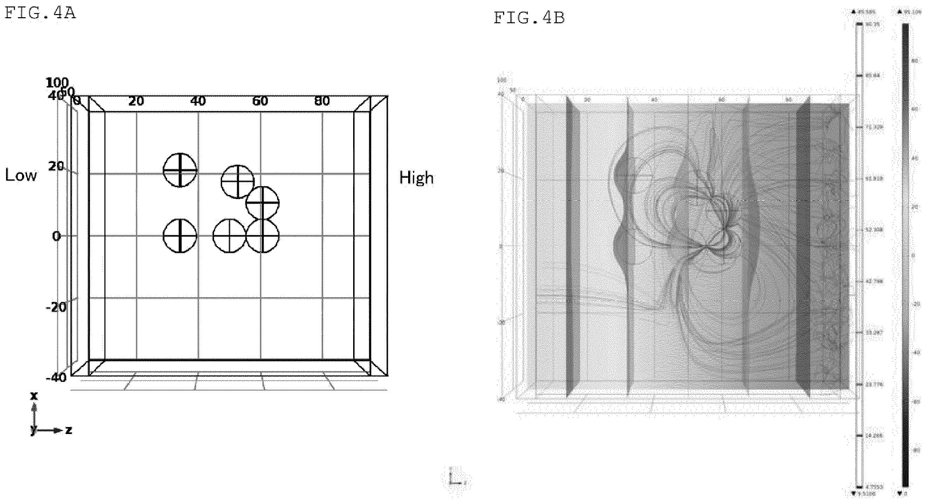

FIG. 4 illustrates potential simulation results of water particles. FIG. 4A is a diagram illustrating a simulation model. FIG. 4B illustrates a potential simulation result. As illustrated in FIG. 4A, free water in the simulation model includes four water particles in the pearl-chain structure in a center portion, and two independent water particles on the left side thereof.

FIG. 4B illustrates three portions of equipotential regions in a cross section in a vertical direction along a longitudinal direction of the water particles. In the rightmost cross section, it is indicated that in a portion where the water particles are in the pearl-chain structure, equipotential water particles are in the pearl-chain structure. The region with the four water particles in the pearl-chain structure, in the center portion in the figure, is substantially uniformly colored. Thus, it can be seen that the potential is substantially uniform in the region with the four water particles in the pearl-chain structure.

Electric field lines running among the four water particles in the pearl-chain structure indicate that these four water particles are attracted to each other. Electric field lines can also be found between the four water particles in the pearl-chain structure and the two independent water particles separated from and on the left side of the four water particles in the pearl-chain structure. Thus, it is expected that force in a direction to be attracted to the four water particles in the pearl-chain structure is also acting on the two independent water particles. Thus, the two independent water particles may join the pearl-chain structure of the four particles.

### [Reduction of interfacial tension]

In W/O emulsion (micro-water droplets in cooking oil, for example), interfacial tension can be reduced by applying the electromagnetic field with the liquid control apparatus 1 according to the present embodiment. In such a case, the interfacial tension can be reduced by 10% or more, or by 20% or more depending on the condition of the electromagnetic field. Furthermore, the interfacial tension can be reduced by 60% or more, by appropriately controlling the DC component voltage and the AC component voltage, for example. This effect is expected to be attributable to the increase in interfacial polarization as a result of the electromagnetic field application.

When food is cooked in cooking oil, for example, water droplets separated from the food to be in the cooking oil as a result of the moisture in the food turning into water vapor in the cooking oil are micro water droplets. If the interfacial polarization in the micro water droplets is sufficient for reducing the interfacial tension, the pearl-chain structure of the micro water droplets is achieved with attraction between dipoles. When the food is fried with cooking oil by using a fryer, the interfacial tension of the oil/water interface can be reduced with the pair of electrodes 13 and 14 of the liquid control apparatus 1 according to the present embodiment installed in the fryer.

Interfacial tension is not limited to that of the interface between water and oil. The interfacial tension of any interface between phases at least one of which is a liquid, such as the interface between two types of liquids, between a liquid and a gas, or between a liquid and a solid, can be controlled to be reduced by the liquid control apparatus according to the present embodiment.

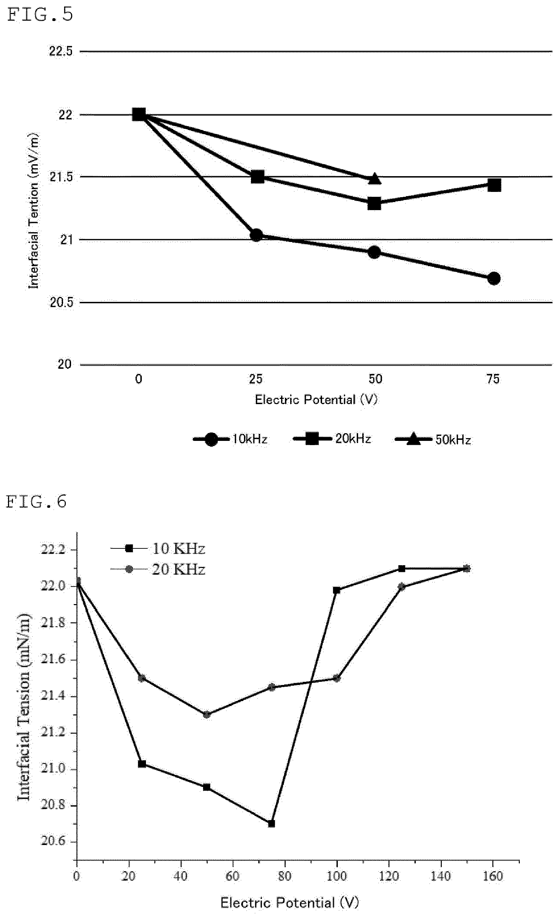

FIG. 5 and FIG. 6 are graphs showing reduction of interfacial tension between cooking oil and water achieved by the liquid control apparatus 1 according to the present embodiment. FIG. 5 is a graph of interfacial tension between cooking oil and water as a result of changing the frequency and the voltage value (0 to 75 V) of the applied voltage. FIG. 6 is a graph of interfacial tension between cooking oil and water as a result of changing the frequency and the voltage value (0 to 150 V) of the applied voltage. For FIG. 5 and FIG. 6, measurement different from that using the measurement apparatus described in the section [Reduction of interfacial tension] described above was conducted. Specifically, a pair of stainless electrodes were inserted into a container including water (lower layer) and cooking oil (upper layer) in a state of having their interfaces in contact with each other, and the interfacial tension between the cooking oil and the water was measured while applying AC voltage with various frequencies and voltage values. Face Automatic Surface Tensiometer (Kyowa Interface Science, Inc.) was used for measuring the interfacial tension. Here, a pair of flat plate electrodes were used as the electrode. However, this should not be construed in a limiting sense. For example, a curved electrode conforming to an inner wall of a cylindrical container may be used, or a flexible electrode such as stainless foil may be arranged along an inner surface of a container.

FIG. 5 is a graph showing the interfacial tension of the cooking oil and water, as a result of changing the frequency of the AC voltage applied to the electrode from 10 kHz to 50 kHz and changing the voltage from 0 V to 75 V. It can be seen in FIG. 5 that the interfacial tension between the cooking oil and water is associated with the frequency and voltage value of the AC voltage applied to the electrode. More specifically, the interfacial tension decreased with the frequency drop from 50 kHz to 20 kHz and then to 10 kHz, and increased with the increase in the voltage value from 0 V to 75 V. Thus, based on such association between the interfacial tension and the frequency and voltage value of the AC voltage applied to the electrode, the liquid control apparatus 1 can control the interfacial tension by adjusting the applied voltage. For example, when the liquid control apparatus 1 is applied to a fryer, reduction of the interfacial tension results in the moisture contained in food turning into dispersible micro water droplets with a small particle diameter in the cooking oil upon being separated from the food as described above. Thus, even when the water droplets evaporate in the heated cooking oil to be vaporized, resulting bumping is small. In such a situation, the magnitude of the bumping can be adjusted with the liquid control apparatus 1 controlling the interfacial tension. Thus, the voltage applied to the electrode can be set in accordance with various conditions of cooking using the fryer as well as various types, states, and amounts of food ingredients. Thus, even when the condition of the cooking using the fryer varies, the interfacial tension can be appropriately controlled by applying appropriate voltage to the electrode, whereby food cooked can have excellent mouthfeel and taste. This is also effective in a case where the feedback control is performed on the voltage applied to the electrode. The interfacial tension can be measured or estimated, and thus can be used as one of the control parameters.

FIG. 6 is a graph showing the interfacial tension of the cooking oil and water, as a result of changing the frequency of the AC voltage applied to the electrode from 10 kHz to 20 kHz and changing the voltage from 0 V to 160 V. FIG. 6 illustrates an example of measurement performed by a certain experiment apparatus. Of course, it is impossible to expand the measurement result to cover any kinds of measurement system. Still, the results at least indicate that the interfacial tension is associated with the frequency and the voltage value of the AC voltage applied to the electrode. Utilizing such association, the interfacial tension can be optimized by adjusting the frequency and the voltage value of the AC voltage applied to the electrode. As described above, the association between the effect of the liquid control apparatus 1 according to the present embodiment and the interfacial tension has been understood. Thus, the effect can be optimized based on the association with the interfacial tension, not only in the case of fryers, but also in cases where the present invention is applied to other targets, that is, used for cold storage, storage, and the like. The measurement of the interfacial tension described above is relatively easy. Thus, the voltage applied to the electrode can be more appropriately and more easily controlled with optimization of the liquid control apparatus 1 according to the present embodiment being analyzable based on the association with the interfacial tension.

While FIGS. 5 and 6 illustrate the reduction in interfacial tension between the aqueous phase and the oil phase, the interfacial tension between the liquid phase and phases other than the liquid phase can also be controlled by controlling the electromagnetic field to be applied in a similar manner. The control of the interfacial tension of the liquid can be applied not only to the interfacial tension between the liquid phase and another liquid phase, but also to the interfacial tension between the liquid phase and gas phase and to the interfacial tension between the liquid and solid phase. For example, the liquid control apparatus of the present embodiment can also control the interfacial tension, a contact angle, and the like by application of an electromagnetic field.

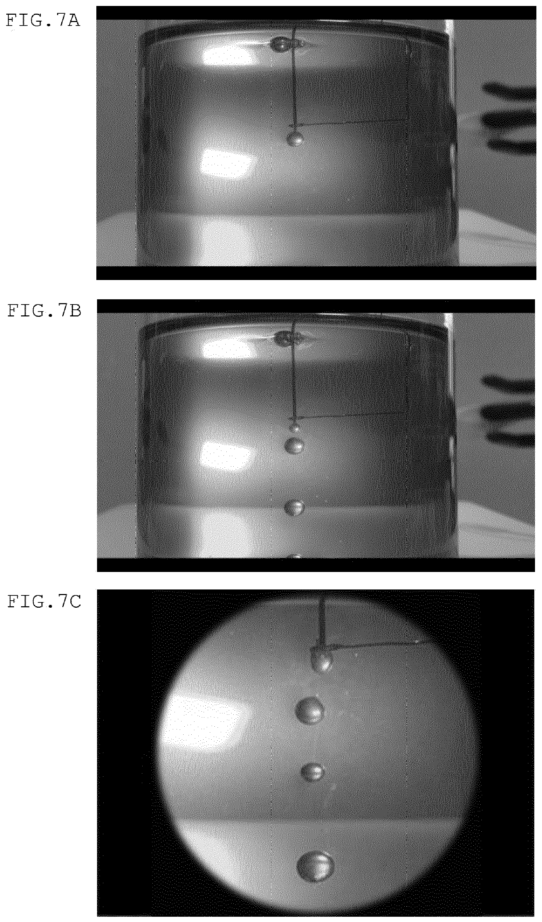

FIG. 7 includes photographs showing water droplets dropping into oil. The photograph shows a state where saline is dropped into cooking oil from a thin tube (a metal straw with a diameter of 1.0 mm) with an annular electrode provided to surround an area around a tip of the thin tube, and with voltage of 100 V applied between the thin tube and the annular electrode. Without the voltage application, no water droplet drops into the oil. The voltage application leads to reduction of interfacial tension between the cooking oil and saline, resulting in water droplets dropping into the oil. It can be seen in FIG. 7 that fine bubbles are dispersed around the dropping water droplets. The voltage application leads to the reduction of the interfacial tension, resulting in a smaller particle diameter of the water droplets as well as generation of fine bubbles when the water droplets drop.

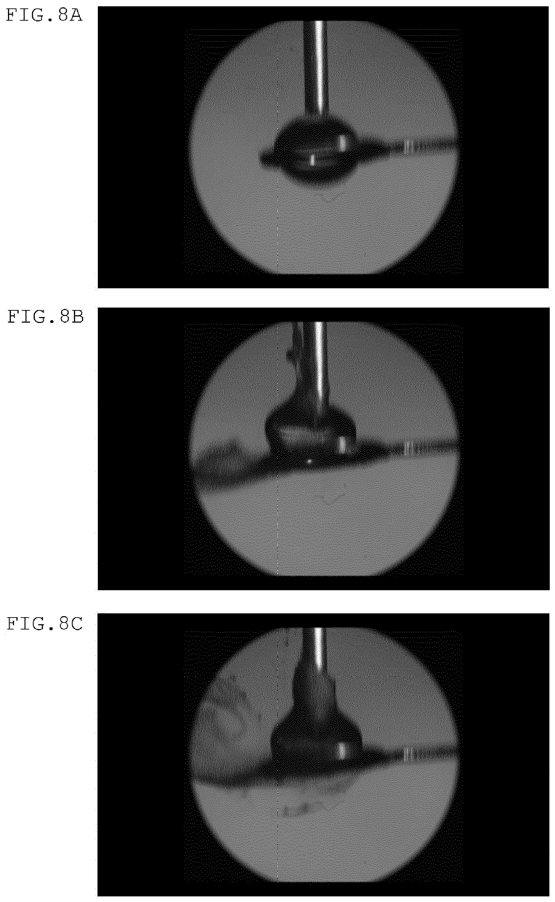

Water droplets of saline dropping into the cooking oil in response to the voltage application. The figure is a result of monitoring, with a high-speed camera, moment of the dropping of the water droplet. FIG. 8A illustrates a state before the voltage application, FIG. 8B illustrates a state where the voltage application has started, FIG. 8C illustrates a state after the voltage application. FIG. 8A, FIG. 8B, and FIG. 8C are in a chronological order. Fine bubbles can be found as illustrated in FIG. 8B and FIG. 8C as a result of voltage application. In some parts, they cannot be clearly distinguished from gas generated from the electrode due to electrolysis.

The function of the liquid control apparatus according to the present embodiment that controls, for example, the formation of fine bubbles of water droplets in oil by reducing the interfacial tension can be used to generate, for example, a water in oil (W/O) emulsion or an oil in water (O/W) emulsion and enhance the quality such as stability of emulsion. The emulsion is not limited to that of two phases. The emulsion of three or more phases can also be controlled. Here, the mixing of two phases, that is, the water phase and the oil phase, has been described as an emulsion by way of example. However, the liquid control apparatus according to the present embodiment can be used to control the mixing, stirring, or kneading of any two or more phases, for example, two liquids or a liquids and a solid, as long as one of the phases includes a liquid. Although the simple expression "control of emulsion" is used in the following, the control includes control of mixing of any two or more phases.

When the liquid control apparatus of the present embodiment controls the electromagnetic field applied to an object, the electromagnetic field acts on the moisture present inside or on the surface of the object, which makes it possible to prevent, suppress, or control moisture being rotten, moisture being turbid, color being turbid, algae growth, sliminess, rusting, molding, or the like.

### [First modification]

A liquid control apparatus according to a first modification will be described with reference to FIG. 9. FIG. 9 is a conceptual view of electrodes according to the first modification. For configurations that are the same as those in FIG. 1 to FIG. 8, the same reference numerals are used, and the descriptions thereof are omitted. The liquid control apparatus according to the first modification is different from the liquid control apparatus according to the first embodiment in that two pairs of electrodes are provided.

A liquid control apparatus 1A includes controllers 10A and 10B as well as first electrodes 13 and 14 and second electrodes 15 and 16 as two pairs of electrodes. The controllers 10A and 10B each include an AC component voltage generation unit and a DC component voltage generation unit. In the actual circuit configuration of the controller 10, the AC component voltage generation unit and the DC component voltage generation unit may not be separately provided, and thus the circuit configuration having the functions of both units may be employed. The two controllers 10A and 10B may be configured as a single controller. The single controller may apply voltage to both of the first electrodes 13 and 14 and the second electrodes 15 and 16, as long as the first electrodes 13 and 14 and the second electrodes 15 and 16 generate similar electromagnetic waves.

The liquid control apparatus 1A is driven by the controllers 10A and 10B and an electric field is generated between the pair of first electrodes 13 and 14 and between the pair of second electrodes 15 and 16. In this case, the electrodes 13 to 16 each function as an antenna, and an electromagnetic field is generated with electromagnetic waves radiated between the first electrodes 13 and 14 and between the second electrodes 15 and 16. Thus, at least one of an electric field, a magnetic field, an electromagnetic field, and electromagnetic waves is generated between the electrodes 13 and 14 and the electrodes 15 and 16. As in the first embodiment, the electrodes 13 and 14 may also be vibrated by an electric, magnetic, or mechanical unit, so that sound waves and/or ultrasonic waves can be generated between the electrodes. With the water molecules vibrated by predetermined sound waves and/or ultrasonic waves, the water molecules can be aligned without applying voltage between the electrodes.

A processing target object is disposed between the first electrodes 13 and 14 and between the second electrodes 15 and 16. The processing target object is not particularly limited as long as the object is at least one of solid, liquid, and gas, as in the first embodiment. When the liquid control apparatus 1A according to the present embodiment is provided to a refrigerator, for example, the first electrodes 13 and 14 may be provided on side surfaces in the refrigerator, and the second electrodes 15 and 16 may be provided on the ceiling surface, the bottom surface, or the tray. FIG. 9 illustrates an example where the first electrodes 13 and 14 and the second electrodes 15 and 16 are orthogonally arranged. However, the present invention is not limited to this, and the first electrodes 13 and 14 and the second electrodes 15 and 16 may be in any arrangement as long as at least part of the electromagnetic field generated by the first electrodes 13 and 14 and the electromagnetic field generated by the second electrodes 15 and 16 acts on the processing target object.

The controllers 10A and 10B perform feedback control on at least one of the value of the current and the voltage applied to the electrode, the frequency of the current and/or the voltage, and the phase of the current and/or the voltage, based on a detection signal from an unillustrated detection unit. The detection unit includes at least one of a voltage sensor configured to detect the voltage applied to the electrode, a current sensor configured to detect the current applied to the electrode, a frequency sensor configured to detect the frequency of the voltage and/or current applied to the electrode, a magnetic field sensor configured to detect a magnetic field between the electrodes 13 and 14 and between the electrodes 15 and 16, an electric field sensor configured to detect an electric field between the electrodes 13 and 14 and between the electrodes 15 and 16, a phase detection sensor for voltage, a phase detection sensor for current, and a phase detection sensor for voltage and current.

At least one of the control target values in the controllers 10A and 10B, which are the current value, the voltage value, their frequencies, and their phases, is set in accordance with the type and/or the state of the target object. For example, the current and/or voltage applied from the controller 10A to the first electrodes 13 and 14 as well as the frequency and the phase of the current and/or voltage may be respectively the same as, or different from, the current and/or voltage applied from the controller 10B to the second electrodes 15 and 16 as well as the frequency and the phase of the current and/or voltage. For example, various combinations may be employed including a combination with voltage and frequency being different therebetween, a combination with only the frequency being different, and a combination with frequency and the phase being different therebetween.

The control target value may be remotely set through an unillustrated communication device. The control parameters and/or the control amount of the controllers 10A and 10B can also be remotely controlled. Thus, the controllers 10A and 10B of a plurality of the liquid control apparatuses 1A can be collectively managed by the management server 40 at a remote location, whereby the controllers 10A and 10B can be appropriately controlled. However, the control mode for the controllers 10A and 10B is not limited to the remote control from the management server 40. The controllers 10A and 10B of each liquid control apparatus 1A can be individually controlled with the control target value and/or the control parameter directly set to each of the controllers 10A and 10B, for example.

The management server 40 can be a cloud server in the cloud. One or more liquid control apparatuses are connected to the cloud server via a network. The control information at each liquid control apparatus is managed by the cloud server, and optimal control parameters are set by information from the cloud server according to a target site and a state. Each piece of control result data is also collected by the cloud server. For example, the cloud server stores data on voltage, current, frequency, and interfacial tension for the target object, the target site, and the state of the target object, and the cloud server sets the control parameters of the controller of each liquid control apparatus according to these pieces of data. The cloud server may directly transmit, to the controller, the target values of voltage to be applied to the electrodes and its frequency as control parameters. Alternatively, the cloud server may transmit, to the controller, data necessary to calculate the voltage to be applied to the electrodes and its frequency, such as the interfacial tension according to the target object and its state, and the controller may calculate the target values. The former case can save the time and effort of setting by on-site operators because the target values can be set from the cloud server. In the liquid control apparatus according to the present embodiment, different types of controllers can be prepared according to the target object, the target site, the type and state of the target object or the target site, the control target, and the like. However, the same controller can be used to set various target values, which can be applied to the control of a variety of target objects. The optimal control parameters can be determined by machine learning as described later, using various history data collected in the cloud server. In setting the control parameters, a dummy measuring device for parameter measurement can be used, which can sense the electric field applied from the electrodes of the liquid control apparatus, as described later.

In FIG. 9, an electromagnetic field is applied from each of two orthogonal pairs of electrodes (X-direction electrodes 13 and 14 and Y-direction electrodes 15 and 16), so that controlling the current or voltage applied to each electrode can adjust the direction of the electromagnetic field generated between the electrodes as well as the strength of the electromagnetic field. This makes it possible to control the arrangement direction of water molecules or water particles. In the case where the controller 10A controls the current or voltage applied to the X-direction electrodes 13 and 14, and the controller 10B controls the current or voltage applied to the Y-direction electrodes 15 and 16, the strength and direction of the electromagnetic field generated by each electrode can be adjusted by adjusting, for example, a ratio between the values of the voltage applied to the X-direction electrodes 13 and 14 and the voltage applied to the Y-direction electrodes 15 and 16. This makes it possible to atomize the moisture inside or on the surface of an object, which is disposed in the electromagnetic field, into fine particles and to also control the arrangement state of the moisture, which is atomized into fine particles, to be in a desired direction. Since DC voltage as well as AC voltage can be applied to each electrode, adjusting the DC voltage component can control the arrangement of the moisture inside or on the surface of the object, which is disposed in the electromagnetic field generated by each electrode, in any direction with respect to two-dimensional X-Y coordinates. When an additional pair of Z-direction electrodes (not shown) and a controller 10C are added to the two pairs of electrodes, the X-direction electrodes 13 and 14, and the Y-direction electrodes 15 and 16 in FIG. 9, electromagnetic waves of a desired direction and strength can be generated in three-dimensional space. Hence, the arrangement of the moisture inside or on the surface of the object, which is disposed in the electromagnetic field generated by each electrode, can be controlled in any direction with respect to three-dimension. As described later, the liquid control apparatus of the present embodiment can improve properties of an object by atomizing water molecules inside or on the surface of the object into fine particles and by controlling the state of the pearl-chain structure in a specific direction and controlling the arrangement direction.

FIG. 10 is a conceptual view of different electrodes according to the first modification. FIG. 10A illustrates an example where a single electrode is used. FIG. 10B illustrates an example where a single electrode and two electrodes facing this electrode are used. In the example described in the first embodiment, a pair of electrodes are used. In the example described in the second embodiment, two pairs of electrodes are used. However, the present invention is not limited to this, and a single electrode may be used, and an odd number of electrodes such as three electrodes may be used. For example, as illustrated in FIG. 10A, the electromagnetic waves can be generated with a single electrode 17. For example, as illustrated in FIG. 10B, when three electrodes are used, two electrodes 19 and 20 may face a single electrode 18, or three electrodes may generate different types of electromagnetic waves. Thus, the number and arrangement of electrodes can be set as appropriate and are not limited.

### [Second modification]

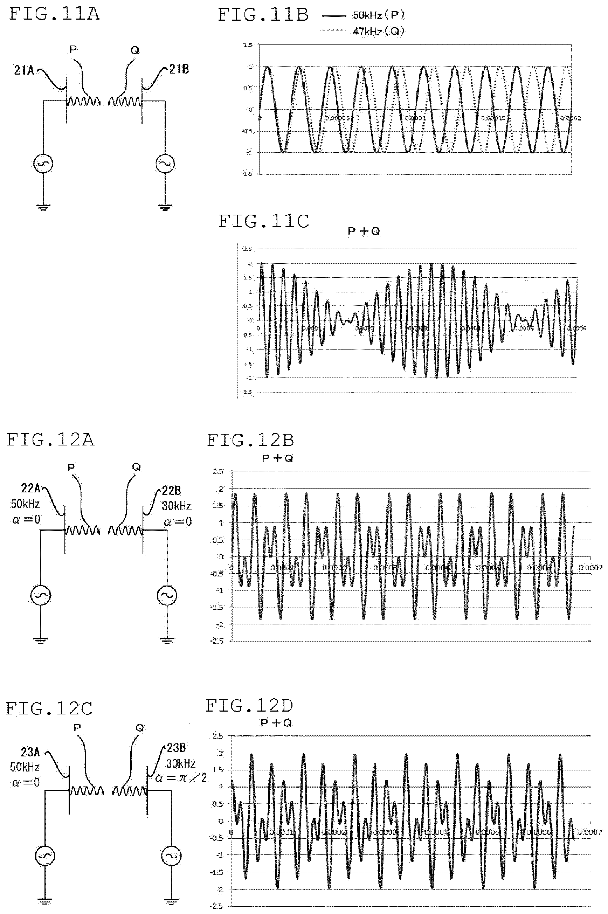

A liquid control apparatus according to a second modification of the present invention will be described with reference to FIG. 11 and FIG. 12. FIG. 11 is a diagram illustrating waveforms obtained by using voltage at different frequencies according to the second modification. FIG. 12 is a diagram illustrating waveforms obtained by using voltage in different phases according to the second modification. For configurations that are the same as those in FIG. 11 to FIG. 12, the same reference numerals are used and the descriptions thereof are omitted. A liquid control apparatus according to the second modification is different from that according to the first embodiment and the first modification in that a pair of electrodes are different from each other in the electromagnetic waves they generate.

In FIG. 11, an electrode 21A that is one of a pair of electrodes 21A and 21B generates electromagnetic waves (P wave) at a frequency of 50 kHz and the other electrode 21B generates electromagnetic waves (Q wave) at a frequency of 47 kHz. The P wave and the Q wave are represented by the following formulae corresponding to a position where both waves are V(t) = 0 at time t = 0 (the position just in the middle of the electrodes 21A and 21B, for example). In the formulae, A represents the amplitude of the electromagnetic waves.
P wave: V(t) = Asin(2πf1t), f1 = 50 kHz
Q wave: V(t) = Asin(2πf2t), f2 = 47 kHz
Thus, the electromagnetic wave that is the sum of the P and Q waves is applied between the pair of electrodes 21A and 21B as illustrated in FIG. 11C.

In FIG. 12, an electrode 22A that is one of a pair of electrodes 22A and 22B generates electromagnetic waves (P wave) at a frequency of 50 kHz and the other electrode 22B generates electromagnetic waves (Q wave) at a frequency of 30 kHz. Phases α of the waveforms match (α = 0). The P wave and the Q wave are represented by the following formulae corresponding to a position where both waves are V(t) = 0 at time t = 0 (the position just in the middle of the electrodes 21A and 21B, for example). In the formulae, A represents the amplitude of the electromagnetic waves.
P wave: V(t) = Asin(2πf1t), f1 = 50 kHz
Q wave: V(t) = Asin(2πf2t), f2 = 30 kHz
Thus, the electromagnetic wave that is the sum of the P and Q waves is applied between the pair of electrodes 22A and 22B as in FIG. 12B.

In FIG. 12C, an electrode 23A that is one of a pair of electrodes 23A and 23B generates electromagnetic waves (P wave) at a frequency of 50 kHz and with a phase α = 0 and the other electrode 23B generates electromagnetic waves (Q wave) at a frequency of 30 kHz and with a phase α = n/2. Thus, the phases of the waveforms are set to π/2. The P wave and the Q wave are represented by the following formulae corresponding to a position where P wave is V(t) = 0 and Q wave is V(T) = A at time t = 0 (the position just in the middle of the electrodes 21A and 21B, for example). In the formulae, A represents the amplitude of the electromagnetic waves.
P wave: V(t) = Asin(2πf1t), f1 = 50 kHz
Q wave: V(t) = Asin(2πf2t + n/2), f2 = 30 kHz
Thus, the electromagnetic wave that is the sum of the P and Q waves is applied between the pair of electrodes 23A and 23B as illustrated in FIG. 12D.

In FIGS. 11 to 12, the electrodes generate electromagnetic waves at different frequencies and/or with different phases. However, the present invention is not limited to these. For example, peak-to-peak voltage of the electromagnetic waves can be adjusted by adjusting the AC component voltage applied to the electrodes. The DC component voltage as offset voltage for the AC component voltage may be applied by adjusting the DC component voltage applied to the electrodes. The DC component voltage applied may be different between the electrodes. Furthermore, the AC component voltages applied to the electrodes may be different from each other in the peak-to-peak voltage value, frequency, and phase.

### [Third modification]

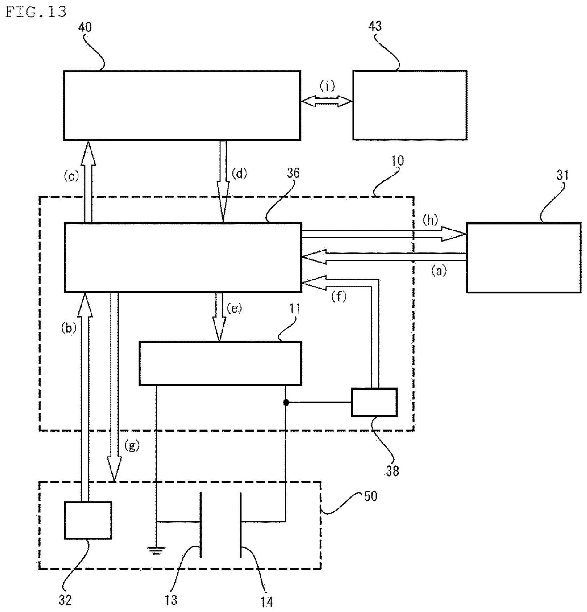

A liquid control apparatus according to a third modification of the first embodiment of the present invention will be described with reference to FIG. 13. FIG. 13 is a block diagram of a liquid control apparatus 1. For configurations that are the same as those in FIG. 1 to FIG. 12, the same reference numerals are used, and the descriptions thereof are omitted.

FIG. 13 is a block diagram corresponding to FIG. 1. Note that the communication unit 35, the storage unit 37, the external power supply 39, and the like are omitted. Specifically, although the control unit 36 actually communicates with the management server 40 and the like via the communication unit 35, inputs and outputs data to and from the storage unit 37, receives power from the external power supply 39, and controls the current voltage application unit 11 via the current voltage control unit 33, these operations are omitted in FIG. 13. In FIG. 13, the controller 10 is illustrated to be outside the housing 50 (for example, a refrigerator or the like). However, this should not be construed in a limiting sense, and the controller 10 may be provided inside the housing 50.

Flows (a) to (h) in FIG. 13 will be described in this order. In the flow (a), settings of the controller 10 are input through an input on the man-machine interface 31. The settings include a setting on ON/OFF and an operation mode of the controller 10, a type and/or a state of an object, output voltage and/or output current of the current voltage application unit 11, and the like. Examples of the operation mode include an automatic mode, an object input mode, a manual setting mode, and the like. For example, in the automatic mode, the controller 10 is automatically controlled so that an appropriate state of the object is achieved based on a detection signal from the object detection unit 32, a detection signal from the detection unit 38, and a control parameter and/or a control value from the management server 40, as described later. In the object input mode, for example, the controller 10 is appropriately controlled based on the object, with the type and/or the state of the object input through the man-machine interface 31. In the manual setting mode, for example, the output voltage and/or output current of the current voltage application unit 11 is manually set. The following description is given assuming that the automatic mode is set, for example, unless stated otherwise. In the flow (a), when the housing 50 further has an automatic adjustment function, a housing setting value for the housing 50 may be able to be input through the man-machine interface 31.

In the flow (b), information about an object is collected from the object detection unit 32 in response to an instruction from the control unit 36. In the example where the housing 50 is a refrigerator, the information about an object collected by the object detection unit 32 includes an image from a camera in the refrigerator, a detection signal related to moisture in a food product from a moisture amount sensor, a detection signal from a temperature sensor and/or a humidity sensor (including a detection signal from a sensor built in the refrigerator), and the like. In the example where the housing 50 is a container, the information about an object collected by the object detection unit 32 includes an image from a camera in the container, a detection signal from a temperature sensor and/or a humidity sensor in the container, a signal from a GPS provided to the container (the GPS may be provided to the controller 10), and the like. Although the example where the housing 50 is a refrigerator is described here, the present embodiment is not limited to this. Examples of the housing 50 include therapy table, treatment table, cot, electromagnetic therapy device, potential therapy device, low frequency therapy device, EMS, massage device, facial device, vibration device, cavitation device, micro-bubble device, micro-nano bubble device, nano bubble device, fine valve device, terahertz device, high frequency device, quantum therapy device, hair growth device, cellulite device, muscle relaxation device, ultrasonic therapy device, ozone generating device, hydrogen generating device, LED device, beauty device, slimming device, or apparatus, device, equipment, or facility with a storage container. The information about the object collected by the object detection unit 32 includes information from cameras and sensors for therapy or treatment, information collected or processed by the various devices listed above, and information from storage containers and storage devices.

In the flow (c), the information about the object collected by the object detection unit 32 in response to an instruction from the CPU 36 is transmitted to the management server 40 via the communication unit 35. When the setting input in the flow (a) is the object input mode, for example, information about the type and the state of the object input through the man-machine interface 31 is transmitted to the management server 40, for example.

When the setting input in the flow (a) is the manual setting mode, for example, the information about the output voltage and/or output current of the current voltage application unit 11 is transmitted to the management server 40. Then, after predetermined correction is performed in the management server 40, a predetermined control parameter and a control value may be transmitted from the management server 40 to the control unit 36. For example, the output voltage and/or output current of the current voltage application unit 11 manually set for the information collection in the management server 40 may be transmitted to the management server 40, and the control unit 36 may calculate the control value. For example, when the correction of the control value or the information collection are not required in the management server 40, the information about the output voltage and/or output current does not need to be transmitted to the management server 40 in the flow (c).

In the management server 40, a control parameter and/or a control value suitable for the type and the state of the object is calculated. When calculating the control parameter and/or the control value, the management server 40 may refer to information other than the type and the state of the object by communicating with a database 43 and the like. The other information includes season, weather, weather forecast, date and time, location, supply and demand forecast, warehousing and storage status of a refrigerator, a transport path of a container and traffic condition thereof, a status of a group of containers related to the container, inventory control information, supply and demand situation, economic indicators, information on the Web, and the like. In addition, the management server 40 collects therapy or treatment information for each liquid control apparatus, that is, information such as appointment information, therapy details, treatment details, item to be treated, item to be treated, target site, therapy purpose, and processing purpose.

Among information about the object collected by the object detection unit 32, the image from the camera enables the type and the state of the object to be determined by image recognition in the management server 40. For this image recognition, AI trained by, for example, deep learning can be used, for example, so that the type and the state of the object can be accurately recognized. Specifically, the type and the state of the object can be accurately recognized based on the image from the camera, by using a neural network trained by the image of a food product from the camera and data about the actual type and the state of the food product. The server can communicate with another controller 10 to accumulate a large amount of image recognition data, whereby the image recognition accuracy for various objects can be increased. When the controller 10 includes an AI program, the control unit 36 may perform the image recognition by using a learning model that has been trained by the management server 40 and transmit the result of the image recognition to the server 40 in the flow (c). When the image recognition is thus performed by the controller 10, the communication amount of data transmission in the flow (c) can be reduced.

In the flow (d), the control parameter and/or the control value calculated in the management server 40 is transmitted to the control unit 36 of the controller 10.

The management server can be a cloud server in the cloud. One or more liquid control apparatuses are connected to the cloud server via a network. The control information at each liquid control apparatus is managed by the cloud server, and appropriate control parameters are set by information from the cloud server according to a target site and a state. Each control result data is also collected by the cloud server. For example, the cloud server stores data on voltage, current, frequency, and interfacial tension for the target object, the target site, and the state of the target object, and the cloud server sets the control parameters of the controller of each liquid control apparatus according to these pieces of data. The cloud server may directly transmit, to the controller, the target values of voltage to be applied to the electrodes and its frequency as control parameters. Alternatively, the cloud server may transmit, to the controller, data necessary to calculate the voltage to be applied to the electrodes and its frequency, such as the interfacial tension according to the target object and its state, and the controller may calculate the target values. The former case can save the time and effort of setting by on-site operators because the target values can be set from the cloud server. In the liquid control apparatus according to the present embodiment, different types of controllers can be prepared according to the target object, the target site, the type and state of the target object or the target site, the control target, and the like. However, the same controller can be used to set various target values, which can be applied to the control of a variety of target objects.

In order to set various target values using the same controller, it is preferable to use AI for setting the control parameters. The optimal control parameters can be determined by machine learning using various history data collected in the cloud server. For example, deep learning can be used, although the type of machine learning is not particularly limited thereto. Each control parameter and the corresponding control result data can be used as training data. Since control result data of the other liquid control apparatuses connected to the network is also collected in the cloud server, in addition to each control parameter and the corresponding control result data, various big data such as environmental data, mechanism data, target site data, and situation data can be obtained. The big data can be used as training data to train the learning model by machine learning. The trained learning model can be used to set appropriate control parameters according to the target object, the target site, and the target situation. The data detected by the object detection unit 32 and the detection unit 38 in the third modification described later can also be collected by the cloud server, and these pieces of data can also be used as training data for machine learning. The machine learning of image recognition in the object detection unit 32 described later can be performed by the cloud server together with calculation of the control parameters.

As described above, settings of the controller 10 are input through an input on the man-machine interface 31 such as a PC, a tablet terminal, a smartphone, or a mobile phone. The settings include a setting on ON/OFF and an operation mode of the controller 10, a type and/or a state of an object, output voltage and/or output current of the current voltage application unit 11, and the like. Since the liquid control apparatus according to the present embodiment can control various targets, it is preferable that setting values can be input interactively from the man-machine interface 31 when inputting setting values of the controller 10. The liquid control apparatus of the present embodiment may be connected to a reservation system or a management system installed in a facility, a doctor's office, or a clinic where the liquid control apparatus is installed. A schedule of using the liquid control apparatus of the present embodiment, date and time, a target object, a target site, the state of a target object and a target site, a control target, and the like can be input to the liquid control apparatus, based on appointment information, schedule information, management information, and the like. Although not limited to particular item, as the information to be input, the state of a target object and a target site including information on physical condition and symptom including the subject's height, weight, temperature, blood pressure, blood glucose level, information on a medical questionnaire, medical record information, and history information is input and the control target including information on therapy goal, therapy policy, and therapy plan is input. In the case of inputting setting values related to therapy details, input methods that can be employed include checking and confirming the subject's input by a physician or a medical staff member, and inputting the results of a medical interview with the subject by a physician or a medical staff member. Since it is preferable to input therapeutic course or information, the input information includes medical records and therapy history. Machine learning for setting the above control parameters can utilize these various input information, information detected by the object detection unit 32 and the detection unit 38 of the third modification described later, information and image information from a therapy or procedure device used with the liquid control apparatus installed in a facility, a doctor's office, a clinic, or the like, control result data from the liquid control apparatus, medical information and follow-up information of the subject after procedure, medical checkup information of the subject and its history information, calibration information from a dummy measuring device described below, and the like. Machine learning for image recognition from image information and the like input from the object detection unit 32 may be performed independently only for image recognition, or may be performed collectively as machine learning for setting control parameters from image information in the cloud server.

In setting the control parameters, a dummy measuring device for parameter measurement can be used, which can sense the electric field applied from the electrode of the liquid control apparatus. The dummy measuring device includes an electric field measuring device that can measure the electric field applied from the electrode of the liquid control apparatus. The controller of the liquid control apparatus or the cloud server stores data such as the optimal electric field for the target object according to the characteristics of the target object, the target voltage and frequency for the electrode, and the relationship between the electric field and the interfacial tension of the liquid. Based on the stored data, the voltage value and the frequency of the voltage applied to the electrode of the liquid control apparatus can be calibrated using feedback data of the electric field detected by the dummy measuring device so that an appropriate electric field can be applied to the target object according to the type and the arrangement of electrodes of the liquid control apparatus at present. Such calibrated information of the appropriate electric field according to the type and the arrangement of electrodes can also be collected in the cloud server and used for machine learning of the optimal control parameters in the future. The control parameters at least include data such as voltage, current, and frequency applied to the electrodes, and electric field applied from the electrode to the target object. Instead of using a dummy measuring device, the electrode itself may have an electric field measuring function. For example, the value of the electric field applied from the electrode can be calculated based on inductance and capacitance of the electrode and current flowing through the electrode.

In setting the control parameters, various control targets and control goals can be set and include, for example, but is not particularly limited to, the following items: those related to infarction, necrosis, decubitus ulcer, burns, removal of active oxygen, removal of lactic acid, improvement of blood flow, improvement of lymph flow, cerebral infarction, myocardial infarction, thrombosis, embolism, arteriosclerosis, improvement or prevention of clogging of body fluid, drug solution, cosmetics, or liquid, improvement of fluidity of body fluid, drug solution, cosmetics, or liquid, improvement or prevention of induration or hardening, improvement or prevention of water release from cells or tissues, cellular edema, neuronal edema, hydropsy, pulmonary edema, joint edema, ascites edema, insulin secretion disorder, defecation disorder, defecation difficulty, constipation, urinary disorder, congestion, blisters, enhancement of drug delivery, reduction of viscosity of drug solution or body fluid, cell culture, regenerative medicine, reduction of culture time, enhancement of culture quality, or enhancement of culture efficiency in regenerative medicine, cell tissue regeneration, reduction of lactic acid, enlargement, swelling, edema, body fluid retention, dehydration of extracellular or intracellular fluid, skin diseases, pigmentation, chloasma, freckle, epidermal wrinkles, dermal wrinkles, expression wrinkles, xeroderma, improvement of physical condition, induration, muscular induration, myofascial release, myofascial potential improvement, nerve deformation disease, myofascial electromagnetic therapy, muscle imbalance, myofascial potential balance improvement, body balance improvement, myofascial balance improvement, occlusion improvement, osteopathic improvement, manual therapeutic improvement, improvement or prevention of bone fracture or joint disease, endocrine, lymphatic, or pathway balance improvement, bone fracture, joint disease, skin care, moisture retention, fertility improvement, infertility, improvement of sperm motility, sperm activity, egg activity, mitochondrial activity, autophagy activity, PMS, pain, itching, abnormal sensation, cold sensitivity, insensitivity, cramp, anti-aging, hair follicle care, improvement of menopausal disorder, enhancement of detergency, alopecia, AGA, ED, lipolysis promotion, improvement of contact lens fit, dry eye, visual acuity improvement, dynamic vision improvement, sleep disorder, sleep improvement, sleep apnea syndrome, preventive medicine improvement, nerve cell improvement, cellular edema improvement, control of viruses, bacteria, or molds, cancer, glaucoma, cataract, age-related macular degeneration, eye disease, hearing loss, hearing impairment, visual impairment, dementia, Alzheimer's disease, Parkinson's disease, sleep improvement, cosmetic improvement, health promotion, motor function improvement, water balance, gastrointestinal diseases, respiratory diseases, cardiovascular diseases, neurological diseases, hematologic diseases, kidney diseases, endocrinological diseases, internal medicine diseases, improvement or normalization of blood pressure, improvement or enhancement of pulse or heartbeat, improvement or enhancement of respiratory or pulmonary function, improvement or enhancement of dialysis function, osteopathic therapy, rigor mortis improvement, improvement of storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement of electrodes or containers for use in storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement or enhancement of efficacy or quality of medicines, cosmetics, or liquids, improvement or enhancement of extraction of body fluids, medicines, cosmetics, or liquids, improvement or enhancement of formation of teeth, bones, joints, blood vessels, lymph vessels, nerves, cells, skin, hair, or organs, improvement or enhancement of emulsion properties, improvement or enhancement of drug delivery properties, improvement or enhancement of efficacy or performance of medicines, prevention or control of growth of microorganisms, bacteria, fungi, or viruses, prevention of metastasis, reduction of viscosity of drug solution, or control of wave motion of liquids, control of ectoplasm, and the like.

In the flow (e), the control unit 36 uses the control parameter and/or the control value transmitted from the management server 40 to control the output voltage and/or output current of the current voltage application unit 11.

In the flow (f), the control unit 36 performs feedback control on at least one of the values of the current and voltage applied to the electrodes 13 and 14, their frequencies, and their phases, based on the detection signal detected by the detection unit 38. The detection signal detected by the detection unit 38 includes at least one of voltage applied to the electrode, the current applied to the electrode, the frequency and/or the phase of the voltage and/or current applied to the electrode, the magnetic field between the electrodes 13 and 14, the electric field between the electrodes 13 and 14, and the sound waves and/or the ultrasonic waves between the electrodes 13 and 14. The control value fed back in this case may be a control value calculated by the control unit 36 or may be a control value calculated by the management server 40.

When the control value fed back is the control value calculated by the control unit 36, the control target value is transmitted from the management server 40 to the control unit 36 in the flow (d). When the manual mode is set, the setting value as the control target value is input in the flow (a). The control target value may be set variably over time based on the information about the object collected by the object detection unit 32. When the control value fed back is a control value calculated by the management server 40, the detection signal as a result of the detection by the detection unit 38 is transmitted to the management server 40 in the flow (c) for calculating the control value fed back by the management server 40. Then, the management server 40 calculates the control value fed back, and the control value is transmitted from the management server 40 to the control unit 36 in the flow (d).

Although the example of using the detection unit 38 is described in the present embodiment, control not using the detection unit 38 may be employed. In such a case, the flow (f) is omitted, and the output voltage and/or output current of the current voltage application unit 11 is controlled in the flow (e). For the control in this case, various control such as sensor-less control and open loop control can be applied.

In the flow (g), the control unit 36 may transmit a control command to the housing 50 when the housing 50 has the automatic adjustment function. When the housing 50 is a refrigerator, the control command is a setting value of temperature and/or humidity in the refrigerator, for example. When the housing 50 is a container having a temperature/humidity adjustment function, the control command is a setting value of the temperature/humidity for the container, for example. When the housing 50 is a container and is stored in a warehouse with adjustable temperature/humidity, as described later, in the flow (i), information about the adjustment of the temperature/humidity of the container is transmitted to a management server of the warehouse that is the external server and the database 43, for appropriately adjusting the state of the temperature/humidity of all the containers also including other containers. When the housing 50 has no automatic adjustment function, the flow (g) is not a necessary element. In such a case, the information about the control command from the control unit 36 is displayed on the man-machine interface 31 in the flow (h).

In the flow (h), the man-machine interface 31 displays, for example, the control status of the output voltage and/or output current of the current voltage application unit 11 as an example of the control status in the control unit 36, information about the type and the state of the current target object, the status of the housing 50 (detection information from the object detection unit 32), and information about the control command from the CPU 36 to the housing 50 if the housing 50 has no automatic adjustment function. In addition to these pieces of information, the man-machine interface 31 can display information transmitted from the management server 40 in the flow (d) in addition to the control parameter and/or the control value, when required or in response to an operation on the man-machine interface 31. Examples of such information include season, weather, weather forecast, date and time, location, supply and demand forecast, warehousing and storage status of a refrigerator, a transport path of a container and traffic condition thereof, a status of a group of containers related to the container, inventory control information, supply and demand situation, economic indicators, information on the Web, and the like. An operator can appropriately manage the therapy or treatment by referring to such pieces of information.

The man-machine interface 31 may be integrated with the controller 10. The man-machine interface 31 and the controller 10 may be separately provided. The man-machine interface 31 as well as some of the functions of the controller 10 may be provided separately from the controller 10. In such a case, the man-machine interface 31 may be a mobile terminal having a communication function, examples of which include a smartphone, a mobile phone, a tablet terminal, and a PC. When the man-machine interface 31 as well as some of the functions of the controller 10 are provided separately from the controller 10, the man-machine interface 31 and at least one of the functions of the communication unit 35 and the storage unit 37, and the arithmetic function of the control unit 36 or some of such functions may be provided separately from the controller 10. Furthermore, the man-machine interface 31 as well as the functions of the object detection sensor 32 or the detection unit 38 or some of their functions can be integrated. For example, the camera function built in a smartphone, a mobile phone, a tablet terminal or a PC may be used as the object detection unit 32.

In the flow (i), the management server 40 communicates with the database 43 to exchange information required for object management or to collect data. The management server 40 can communicate with a required external server through the Internet. Thus, when the housing 50 is a container, for example, a management database or a management server for a warehouse managing the container can be accessed, for example.

While the electrode is disposed to face a target part, the controller adjusts at least one of a voltage value, a current value, a frequency, or a phase of voltage or current having a DC component and/or an AC component applied to the electrode, and controls at least one of an electric field, a magnetic field, an electromagnetic field, or electromagnetic waves generated from the electrode, and performs controls so that at least one of the frequency and the voltage applied to the electrode, and the emission angle of an electric field, a magnetic field, an electromagnetic field, or electromagnetic waves generated from the electrode changes over time, or control the duration of voltage applied to the electrode, according to the target part facing the electrode. When information about the target object is input from a detection signal from the object detection unit 32, a detection signal from the detection unit 38, or a setting signal from the man-machine interface, the controller controls least one of: changing the frequency and voltage applied to the electrode over time; changing the emission angle of the electric field, magnetic field, electromagnetic field, or electromagnetic waves generated from the electrode, that is, the angle of the electrode over time; or controlling the duration of voltage applied to the electrode, according to the type and state of the target object, based on predetermined settings by the control parameters and control values from the management server 40. The predetermined settings on the change in voltage and/or frequency over time are not limited to any particular manner, but can employ the sweeping characteristics as in a fourth modification described below.

### [Fourth modification]

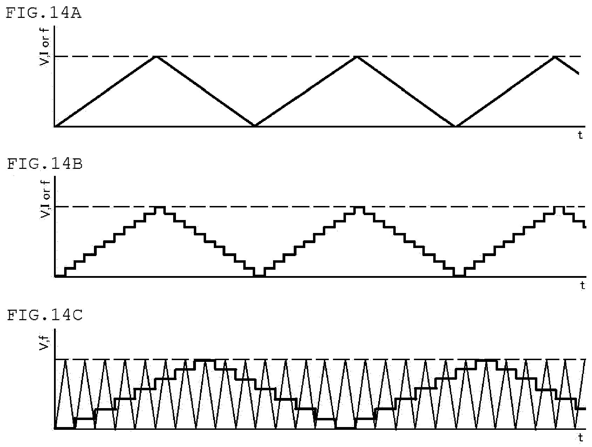

A liquid control apparatus, a quality control method, a program, and a storage medium according to a fourth modification of the present invention will be described with reference to FIG. 14. For configurations that are the same as those in FIG. 1 to FIG. 13, the same reference numerals are used, and the descriptions thereof are omitted. In the liquid control apparatuses 1 according to the first embodiment and the first to third modifications, the current value or voltage value and the frequency of the current or voltage are set to be predetermined values. In the fourth modification, the current value or voltage value and/or the frequency are changed within a predetermined range with a predetermined pattern, and thus are swept. FIG. 14A illustrates an example where the voltage value, current value, or frequency is linearly and continuously swept. FIG. 14B illustrates an example where the voltage value, current value, or frequency is changed linearly and stepwise. In FIG. 14C, for example, the voltage value is changed stepwise, and the frequency is swept linearly and continuously, or the frequency is changed stepwise and the voltage value is swept linearly and continuously, whereby electromagnetic waves with an appropriate current value or a voltage value and/or an appropriate frequency can be automatically generated for any target. Thus, an appropriate current value, voltage value, or frequency is generated at a predetermined timing within a sweeping range. Note that FIG. 14C is merely an example, and thus should not be construed in a limiting sense. In FIG. 14C, while one value is fixed, the other value changes from 0 to the peak and then from the peak to 0. However, this should not be construed in a limited sense. For example, a change may be repeated in which one value increases from 0 to the peak while the other value is fixed, and then decreases from the peak to 0 when the other value changes stepwise and is fixed at a certain value. In FIG. 14C, while one value changes stepwise, the other value changes from 0 to the peak continuously and frequently. However, this should not be construed in a limiting sense. For example, while one value changes gently and continuously, the other value may change from 0 to the peak continuously and frequently.

The sweeping pattern is not limited to those illustrated in FIG. 14. For example, in addition to the linear or stepwise change, a curved change, sinusoidal change, smooth analog change, discrete change, random change, and the like may be employed, for example. An AC voltage value, a DC voltage value, an AC current value, a DC current value, a frequency, and the like may be changed. In such a case, the values may be changed one by one, a plurality of the values may be changed in an interlocked manner (see, for example, the example of FIG. 14C), or a plurality of the values may be simultaneously changed. The sweep range may be within a range defined in the first to the third modifications, for example, or may be expanded to be even wider than such a range.

For any target, an appropriate current value, voltage value, or frequency is generated at a predetermined timing within a sweeping range. Furthermore, through feedback by the object detection sensor 32 and the like, the controller 10 may recognize the state of the target and analyze the state in association with the pattern of the sweep change or the analysis may be performed on the server side, so that an appropriate (or optimum) current value, voltage value, or frequency can be automatically detected. The detected appropriate value may be shared with another controller 10 through a server, in addition to being used by the controller 10 for the control thereafter.

The following describes the liquid control apparatus according to the first embodiment by taking improvement of blood flow as an example. In the liquid control apparatus of the present embodiment, for example, while a subject places both feet on a square plate electrode on the floor, electromagnetic waves are generated from the electrode to perform control to improve the subject's blood flow by adjusting the electromagnetic waves that act on the subject. The electrode may be monopolar or bipolar. In the case of a bipolar electrode, the electrode has a positive electrode and a negative electrode. The negative electrode can be at ground potential, although not particularly limited thereto. The positive electrode and the negative electrode may be arranged such that they are divided in the front-back direction or the width direction of the square electrode.

Although not limited to any particular arrangement, a preferred bipolar arrangement can be an electrode arrangement in which each electrode is comb-shaped and both electrodes face each other such that the comb teeth of both electrodes are arranged alternately. Although the expression "comb teeth" is used, the shape of the comb teeth is not limited to the shape having multiple rectangles and has any geometry. A variety of shapes such as appropriate combinations of protrusions and depressions or spaces can be employed. Each comb-shaped electrode has any size as long as the conductivity of the electrodes and the insulation between the electrodes can be ensured. For example, the clearance between the electrodes can be set to about 0.5 mm to 50 mm, and the width of the conductive part can be set to about 0.5 mm to 50 mm.

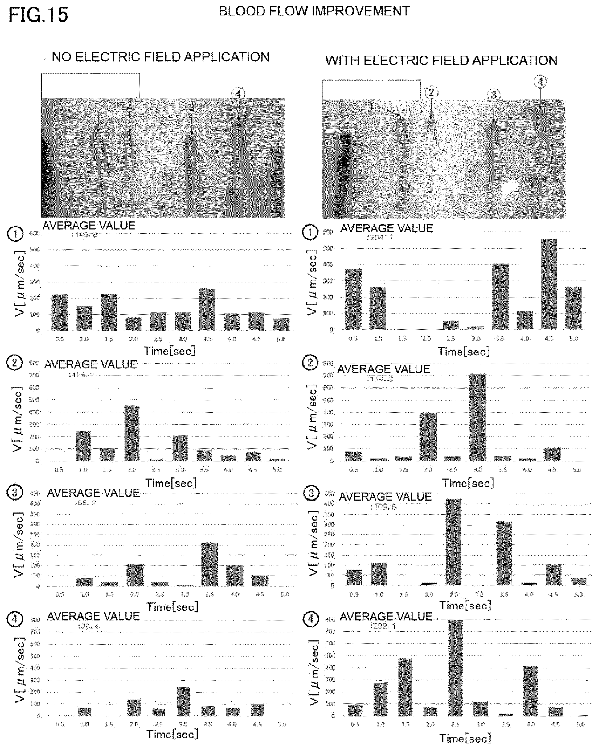

FIG. 15 illustrates the results of blood flow improvement after 30 minutes. FIG. 15 illustrates the results of blood flow improvement in the capillaries in a fingertip when electromagnetic waves act on a subject for 30 minutes with 50 kHz and 100 V applied from the controller to the electrode. The left side shows data without application of electromagnetic waves, and the right side shows data after application of electromagnetic waves for 30 minutes. The average blood flow velocity at each of locations (1) to (4) is improved after 30 minutes of electromagnetic wave application by the liquid control apparatus of the present embodiment.

Although not limited to any particular method, in the present embodiment, the blood flow velocity was measured by image analysis to capture the movement of blood, and the blood flow velocity was calculated.

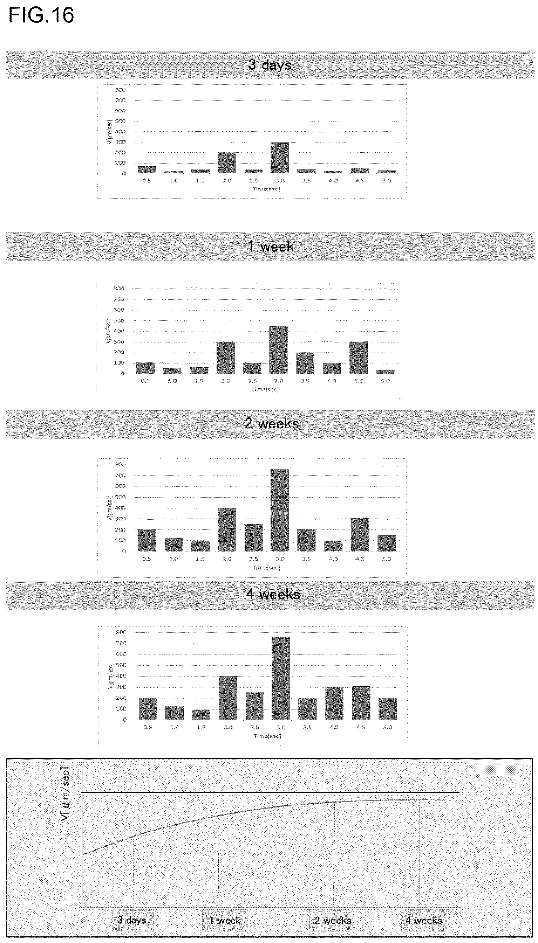

FIG. 16 illustrates the results of blood flow improvement over a four-week period. FIG. 16 indicates that blood flow is improved when the subject undergoes a treatment in which 50 kHz and 100 V is applied from the controller to the electrode to cause electromagnetic waves to act on the subject for 30 minutes every day. The four bar graphs from the top of FIG. 16 represent blood flow velocities over a five-second period and show data on the third day, the first week, the second week, and the fourth week from the top. The bottom graph shows the change in average blood flow velocity over a four-week period, and it has been confirmed that blood flow improves with the passage of days by keeping the treatment every day to cause electromagnetic waves to act on the subject for 30 minutes using the liquid control apparatus of the present embodiment.

The experimental results of blood flow improvement in FIGS. 15 and 16 have showed that the liquid control apparatus of the present embodiment can perform control of improving or enhancing the heart beat or pulse, perform control of improving or normalizing blood pressure, perform control of improving or enhancing dialysis function, perform control of improving a drug delivery system or the permeability of cosmetics. Furthermore, since the effect of blood flow improvement contributes to improvement of fluidity of not only blood but also plasma, lymph, and other body fluids, improvement of body fluid metabolism, and stabilization of water balance, it has been shown that the liquid control apparatus of the present embodiment can perform control of improving or enhancing visual acuity or dynamic vision, perform control of improving or preventing ophthalmologic diseases, perform control of improving or preventing internal medicine diseases, and perform control of improving or enhancing immune function.

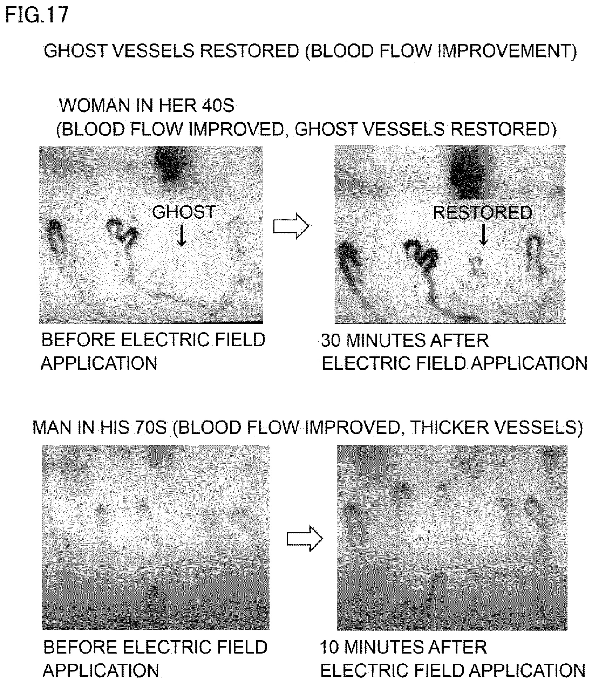

FIG. 17 illustrates the result of ghost vessels being restored. A subject (a woman in her 40s) underwent electromagnetic waves by the liquid control apparatus of the present embodiment for 30 minutes, and the ghost vessels in a fingertip of her hand were restored, as shown in the upper right photograph in FIG. 17. Another subject (a man in his 70s) underwent electromagnetic waves by the liquid control apparatus of the present embodiment for 10 minutes, and the blood flow in the capillaries in a fingertip of his hand improved, as shown in the lower right photograph.

In FIG. 15 to FIG. 17, while the subject placed both feet on a square plate electrode on the floor, electromagnetic waves were generated from the electrode. The electromagnetic waves acting on the subject were adjusted and controlled to improve the subject's blood flow. The voltage applied to the electrode was 50 kHz and 100 V. However, the conditions of voltage for generating electromagnetic waves acting on the subject are not limited to this. The frequency and the voltage value of the voltage applied to the electrode can be adjusted as appropriate for each subject or according to the subject's condition. It is also possible to perform control so that the frequency and the voltage value vary over time. The lower limit of frequency is 1 Hz, which is approximately equivalent to the heart rate, and the upper limit is not limited. For example, the setting condition can be up to 1 MHz, but not limited to this. The upper limit of frequency can be set in the terahertz wave region (0.1 THz to 100 THz). The range of voltage values is not limited, except for the restrictions of the power supply of the liquid control apparatus. For example, the lower limit can be set to about 5 V and the upper limit can be set to about 2000 V.

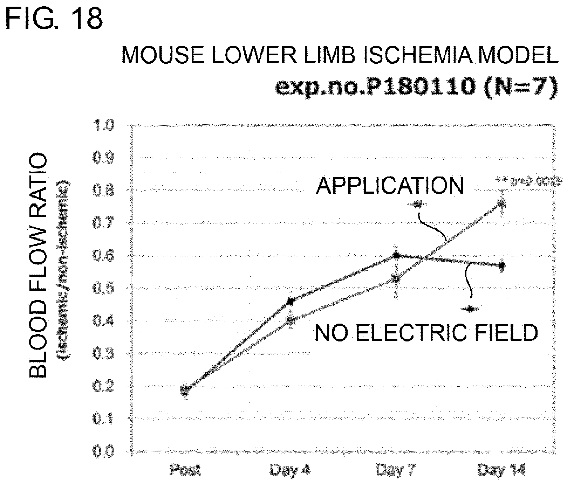

FIG. 18 illustrates the result of blood flow improvement in a mouse lower limb ischemia model. A mouse model of lower limb ischemia (N=7) underwent electromagnetic waves generated by applying a voltage of 50 KHz and 100 V to the electrode using the liquid control apparatus of the present embodiment, and the blood flow ratio was predominantly increased after 14 days.

The experiments in FIGS. 15 to 18 have showed that, by applying an electric field, the liquid control apparatus of the present embodiment can perform control of improving or preventing clogging of body fluid, drug solution, cosmetics, or liquid, perform control of improving or enhancing the fluidity of body fluid, drug solution, cosmetics, or liquid, perform control of improving or preventing induration or hardening, perform control of improving or preventing water release from cells or tissues, and remove active oxygen or lactic acid. Furthermore, it has been shown that, by applying an electric field, the liquid control apparatus of the present embodiment can perform control of improving or preventing dementia, Alzheimer's disease, or Parkinson's disease by enhancing body fluid metabolism in the brain, and perform control of preventing metastasis by enhancing immune function by enhancing body fluid metabolism.

### [Second embodiment]

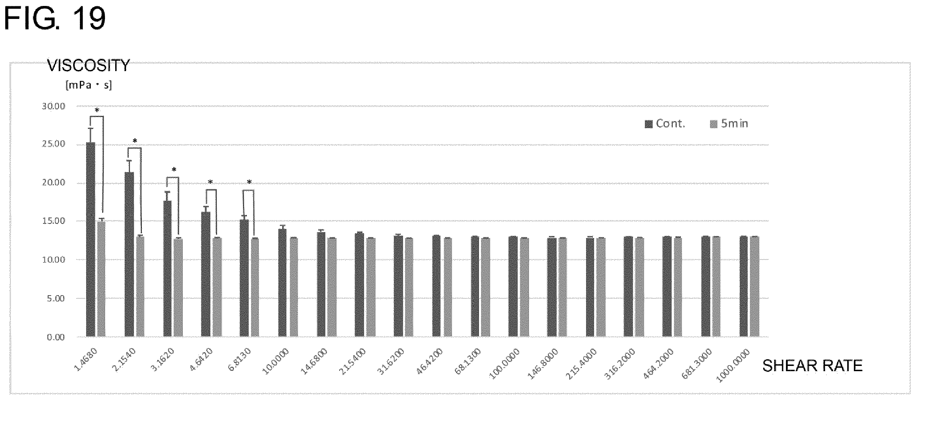

A liquid control apparatus according to a second embodiment will be described with reference to FIG. 19.

FIG. 19 illustrates the result of decrease in viscosity of a contrast agent. In FIG. 19, for a contrast agent to be administered by intravenous drip, a voltage of 50 kHz and 100 V was applied to the electrode by the liquid control apparatus of the present embodiment to cause electromagnetic waves to act on the contrast agent before the drip was administered. The electromagnetic waves were applied to the contrast agent for five minutes. As a result, as illustrated in FIG. 19, it was observed that the viscosity of the contrast agent was reduced. In FIG. 19, the horizontal axis is shear rate, the vertical axis is viscosity. For each shear rate, the bar on the right represents data after the contrast agent was subjected to electromagnetic waves for five minutes by the liquid control apparatus of the present embodiment, and the bar on the left represents control bresentdata. FIG. 19 indicates that the viscosity of the contrast agent is lower in the data obtained after the contrast agent was subjected to electromagnetic waves for five minutes by the liquid control apparatus of the present embodiment.

In FIG. 19, at the shear rates of 100 to 1000, the viscosity of the contrast agent is lower in the present embodiment than in the control. This is shown in Table 1. Table 1 shows the data in FIG. 19 in tabular form. It is understood that the viscosity of the contrast agent is lower in the present embodiment than in the control, at the shear rates in the range of 100 to 1000.

**[Table 1]**

| | | | | | | | | | | | | | | | | | | |
|---|---|---|---|---|---|---|---|---|---|---|---|---|---|---|---|---|---|---|
| Shear rate | | 1.5 | 2.2 | 3.2 | 4.6 | 6.8 | 10 | 14.7 | 21.5 | 46.4 | 68.1 | 100 | 146.8 | 215.4 | 316.2 | 464.2 | 681.3 | 1000 |
| Viscosity [mPa · s] | Present embodiment | 14.98 | 12.96 | 12.73 | 12.81 | 12.75 | 12.82 | 12.8 | 12.8 | 12.8 | 12.8 | 12.8 | 12.81 | 12.82 | 12.87 | 12.91 | 12.96 | 13.01 |
| | Control | 25.31 | 21.45 | 17.77 | 16.23 | 15.19 | 14.04 | 13.63 | 13.41 | 13.07 | 12.99 | 12.95 | 12.92 | 12.91 | 12.92 | 12.94 | 12.96 | 12.96 |

In the present embodiment, the voltage applied from the controller to the electrode is 50 kHz and 100 V. However, the present embodiment is not limited to this, and the frequency and the voltage value of the voltage applied from the controller to the electrode can be adjusted according to the type and the state of the contrast agent to control the electromagnetic waves that act on the contrast agent.

### [Third embodiment]

A liquid control apparatus according to a third embodiment will be described. In the third embodiment, improvement of cellular edema in neurons will be described. The liquid control apparatus of the present embodiment can micronize the moisture in cells by allowing electromagnetic waves to act on a target part. When the liquid control apparatus of the present embodiment applies a voltage of, for example, 50 kHz and 100 V from the controller to the electrode to cause electromagnetic waves to act on the target part to micronize the particles of moisture in the cells into 8 µm or smaller, the micronized moisture can easily pass through the cell membrane. Cellular edema in neurons is caused by water retention in the cell due to ionic imbalance across the cell membrane. The control apparatus of the present embodiment can improve cellular edema by micronizing water molecules in the cell to facilitate drainage of moisture from the cell through the cell membrane. Based on the above, it has been shown that, by performing the body fluid micronization control, the liquid control apparatus of the present embodiment can perform control of improving or preventing edema, cellular edema, or neuronal edema. Furthermore, it has been shown that, by performing the body fluid micronization control, the liquid control apparatus of the present embodiment can enhance body fluid metabolism, enhance body fluid fluidity, and stabilize water balance, in addition to the edema improving effect, and can control hormonal, endocrine, lymphatic, or pathway balance, control mitochondrial activity, autophagy activity, egg activity, or sperm activity, and perform control of improving or preventing burns, necrosis, or decubitus ulcer.

### [Fourth embodiment]

A liquid control apparatus according to a fourth embodiment will be described. In the fourth embodiment, suppression of growth of viruses, bacteria, molds, or cancer cells by the liquid control apparatus of the present embodiment will be described. When the body's immunity is high, the body's internal potential is stable and intercellular fluid bonding occurs, resulting in quasi-bonded liquid. In this state, therefore, viruses, bacteria, molds, or cancer cells are less likely to grow. In contrast, when the body's internal potential is disturbed and the body's immunity is lowered, the potential between intercellular fluids is disturbed, resulting in single nutrient-rich droplets (which may be referred to as "extracellular free fluid"). Viruses, cells, molds, or cancer cells bond with the extracellular free fluid and grow. The liquid control apparatus of the present embodiment can apply a voltage of, for example, 50 kHz and 100 V from the controller to the electrode to cause electromagnetic waves to act on the target part, whereby a pearl-chain structure of the intercellular fluid is achieved. Thus, the growth of viruses, bacteria, molds, or cancer cells can be suppressed.

### [Fifth embodiment]

A liquid control apparatus according to a fifth embodiment will be described. In the fifth embodiment, the antibacterial effect on bacteria or viruses and the infection-preventing activity of the liquid control apparatus of the present embodiment will be described.

First, the antibacterial activity on enveloped viruses, such as influenza virus (H1N1) and coronavirus, will be described. The liquid control apparatus of the present embodiment applies a voltage of, for example, 50 kHz and 100 V from the controller to the electrode to cause electromagnetic waves to act on the target part to decrease the interfacial tension of the moisture, whereby the envelope of the enveloped virus is destroyed. Thus, the liquid control apparatus of the present embodiment can achieve the antibacterial activity on the enveloped virus. As shown in Table **2,** the antibacterial activity of the liquid control apparatus of the present embodiment against the H1N1 virus was 1.12 (antibacterial activity rate 92.45%).

**[Table 2]**

| virus name | No. | Control | | Present embodiment |
|---|---|---|---|---|
| | | virus cultivation 0 hours after vaccination | Virus cultivation 2 hours after vaccination | Viruscultivation 2 hours after vaccination |
| | | (IgTCID50/ml) | (IgTCID50/ml) | (IgTCID50/ml) |
| H1N1 MDCK Cell | 1 | 6.52 | 6.38 | 5.1 |
| | 2 | 6.49 | 6.26 | 5.3 |
| | 3 | 6.56 | 6.34 | 5.2 |
| | Average value | 6.52 | 6.33 | 5.21 |

| | | |
|---|---|---|
| Antibacterial activity value | (lg) | 1.12 |
| Antibacterial activity rate | (%) | 92.45 |

When the liquid control apparatus of the present embodiment allows electromagnetic waves to act on a treatment part of the body, the blood flow improvement improves the transportability of immune cells as described above, thereby enhancing immune function and effectively preventing infection.

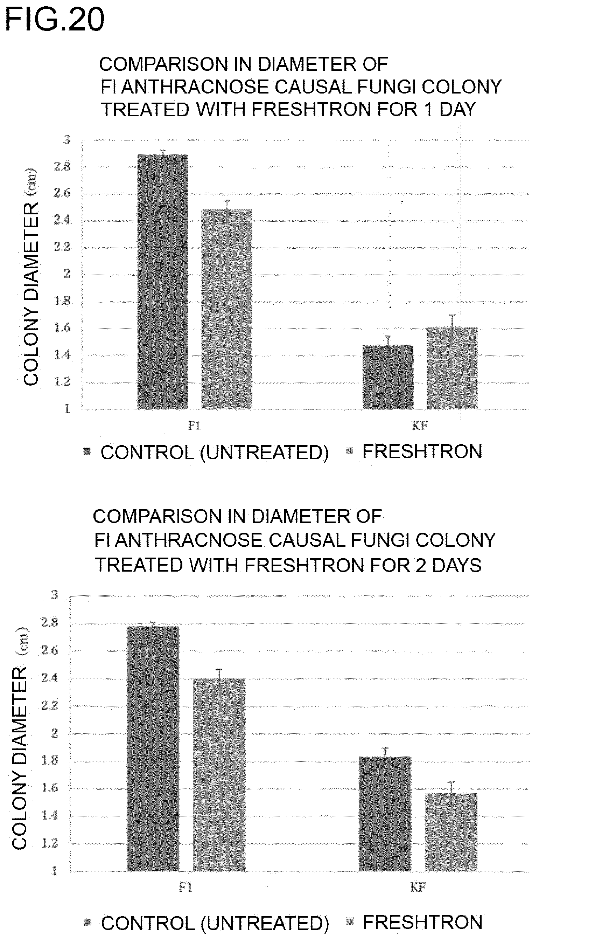

When the liquid control apparatus of the present embodiment allows electromagnetic waves to act on a treatment part, free water bonding in the cell occurs to form a pearl-chain structure as stable as bonded water. This state prevents supply of moisture to bacteria and viruses. FIG. 20 shows the antibacterial activity of the liquid control apparatus of the present embodiment on anthracnose causal fungi. As shown in the upper graph in FIG. 20, the treatment with the liquid control apparatus of the present embodiment for one day achieves a significant antibacterial effect on FI anthracnose causal fungi. As shown in the lower graph in FIG. 20, the treatment with the liquid control apparatus of the present embodiment for two days achieves a significant antibacterial effect on both FI anthracnose causal fungi and FK anthracnose causal fungi.

### [Sixth embodiment]

A liquid control apparatus according to a sixth embodiment will be described. In the sixth embodiment, the use of the liquid control apparatus of the present embodiment will be described. In the present embodiment, the voltage and the frequency applied to the electrode in each experimental example are 50 KHz and 100 V unless otherwise specified. However, the present embodiment is not limited to these values. The target values are set as appropriate by the cloud server and the controller, for example, from 0 to 1 MHz and 0 to 7000 V, according to the target object, the target site, the condition of the target site, and the therapy or treatment details.

The liquid control apparatus of the present embodiment applies an electric field from the electrode to the target site to increase the interfacial polarization of the liquid and decrease the interfacial tension, whereby the particles of the liquid are atomized into fine particles and the fine particles of the atomized liquid are controlled to be arranged in a pearl-chain structure. In addition, the emulsion state of the liquid atomized into fine particles is controlled to be improved. In other words, two liquids are atomized into fine particles and the degree of mixing of two liquids is enhanced, resulting in, for example, a state in which oil is well dispersed in water or water is well dispersed in oil. It is also possible to perform control such that three or more phases are mixed well. With the liquid control apparatus of the present embodiment, for example, the body fluid micronization control can enhance body fluid fluidity, enhance body fluid metabolism, and stabilize the moisture balance to achieve good physical condition. In addition, the arrangement of particles of liquid in a pearl-chain structure prevents the growth of microorganisms, bacteria, fungi, and viruses. In addition, by mixing well for enhancing emulsion quality, for example, it is possible to perform control of improving or enhancing the formation of teeth, bones, joints, blood vessels, lymph vessels, nerves, cells, skin, hair, or organs. In the following, a wide range of applications of the liquid control apparatus of the present embodiment and its functions, actions, and effects as well as various experimental examples will be described.

The liquid control apparatus of the present embodiment applies an electric field to decrease the interfacial tension of liquid and atomize the particles of liquid into fine particles, which enables the following control.
- Improvement of clogging, edema, congestion, swelling, puffiness, blockage, infarction
- Growth promotion, germination rate, water uptake enhancement
- Freshness keeping, taste and aroma enhancement
- Anti-oxidation, anti-aging
- Quality maintenance, deterioration prevention, strength enhancement, fluidity improvement, clogging prevention
- Decrease in oil penetration, decrease in vapor explosion
- Active oxygen removal, redox enhancement

The liquid control apparatus of the present embodiment applies an electric field, so that the liquid particles atomized into fine particles are arranged into a pearl-chain structure, which enables the following control.
- Dry skin, always moist skin
- Suppression of the growth of viruses and microorganisms, suppression of decomposition, suppression of necrosis, and bedsores
- Suppression of germs, molds, algae, and odor
- Decrease of water release, aroma, freshness keeping, quality, anti-oxidation, and extended shelf life
- Strength enhancement, quality maintenance, and prevention of deterioration
- Suppression of separation and water release

Furthermore, the application of an electric field by the liquid control apparatus of the present embodiment enhances the emulsion properties of the liquid particles atomized into fine particles, which enables the following control.
- Quality enhancement, performance enhancement, and effectiveness enhancement
- Quality maintenance, prevention of deterioration, and strength enhancement
- Suppression of separation and water release

The liquid control apparatus of the present embodiment performs body fluid micronization control using the technique that decreases the interfacial tension of the liquid and atomizes the particles of the liquid into fine particles by applying an electric field. The body fluid micronization control can provide control of improving the following symptoms.
- Enhance the metabolism of moisture in the body.
- Clogging of blood vessels, cellular edema being one of the causes
- Cerebral infarction, myocardial infarction, and clogging of blood vessels
- Fluid buildup in lungs or knees, ascites, and lactic acid buildup
- Insulin, urine, and stools are not produced.
- Cellular or neuronal edema, blister, congestion
- Swelling after sprains and surgeries
- Bedsores, decubitus ulcer, necrosis
- Itching, pain, insensitive, cold, stiffness
- Swollen face, wrinkles, dry skin
- Insomnia, apnea

The control to micronize body fluids by the liquid control apparatus of the present embodiment enables the control that improves symptoms as follows (called "body fluids micronization (BFM) therapy" because body fluids are micronized).
- Improve clogging of blood vessels and organs or cellular edema
- Improve circulatory disorder
- Suppress water convection
- Improve blood sugar level, urine, and stool
- Suppress diseases in the initial phase
- Suppress swelling in early stage
- Suppress bedsores, decubitus ulcer, and necrosis
- Suppress itching, pain, insensitivity, cold, and stiffness
- Suppress swelling, wrinkles, and dry skin
- Quality sleep and apnea suppression

In the control that micronizes the body fluids by the liquid control apparatus of the present embodiment, the following five approaches can be taken for symptom improvement control.
(**1**) Health (Health Tech) Health maintenance approach
   - Improve the clogging of blood vessels and organs by micronizing interstitial, lymphatic, and cellular fluids in the body without relying on scalpels, injections, or medications.
   - Improve circulatory system disorders
   - Health maintenance
   - Anti-aging, beautiful skin
(2) Sleep (Sleep Tech) Quality sleep approach
   - Improve apnea syndrome by relaxing throat muscles and improving blood flow, and provide quality sleep by micronizing moisture in the brain and improving blood flow.
   - Quality sleep
   - Apnea syndrome
   - Improvement of depression
(3) PMS, menstrual pain (Fem Tech) PMS, menstrual pain problem-solving approach
   - Improve menstrual pain
   - Premenstrual emotional stability
   - Shorter menstrual days
   - Reduction of pills and painkillers
(4) Anti-aging, rejuvenation, beauty (Beauty Tech) approach
   - For facial induration and myofascial release, improve wrinkles, produce fine texture skin, and improve pores by micronizing internal moisture with radio frequency vibration to change the cells into hydrated cells, and achieving muscle relaxation in a short time.
   - Small face
   - Beautiful, fair skin
   - Rejuvenation
   - Anti-aging
(5) Athlete (Sport Tech) Athletic performance enhancement, recovery from fatigue approach
   - Improve the clogging of blood vessels and organs by micronizing interstitial, lymphatic, and cellular fluids in the body. Prevent the accumulation of lactic acid and improve the problem of active oxygen in the body. Keep muscles in top condition.
   - Improvement of muscle fatigue
   - Best condition
   - Reduction of rehabilitation time

The human body is made up of 60 to 70% water. For example, 76% of muscle is made up of water. The liquid control apparatus of the present embodiment performs control to maintain health and improve physical condition by improving the circulation, metabolism, and balance of water. The body fluid micronization control allows for control to improve symptoms such as cellular and neuronal edema, congestion, clogging of blood vessels and organs, circulation, prevention of virus growth, and decreased viscosity. Conventional therapies aim to solve the following problems, all of which can be solved by the body fluid micronization control by the liquid control apparatus of the present embodiment.
- Solve the problem of bad bacteria.
- Solve the problem of the disability to deliver oxygen and nutrients to cells in every corner of the body.
- Solve the problem of the disability to remove carbon dioxide and waste from the body.
- Solve the problem of aging and oxidation.
- Solve the problem of clogging of organs and blood vessels.
- Solve the problem of loss, decay, and irreversibility.
- Solve the problem of body temperature and energy.
- Solve the problem of mind, feeling, stress, and hormone.

The body fluid micronization control by the liquid control apparatus of the present embodiment enables the following control.
- Prevent growth of coronavirus by achieving a pearl-chain structure of moisture.
- Eliminate clogging such as blood clots by micronizing blood without coagulation.
- Facilitate organ transplantation by maintaining cell freshness.
- Improve the flow of body fluid, interstitial fluid, and blood, and improve decubitus ulcer and necrosis.
- Maintain and promote sustained health by micronizing body fluids to facilitate circulation, improving body fluid metabolism, and maintaining moisture balance, thereby eliminating wastes, preventing the growth of bacteria and viruses, and eliminating clogging in organs and blood vessels.
- Applications include general surgery, general internal medicine, general dentistry, cosmetic and plastic surgery, general skin, gynecology, health, physical training, manual therapy, massage, beauty treatment, basic medicine, oriental medicine, and veterinary medicine.

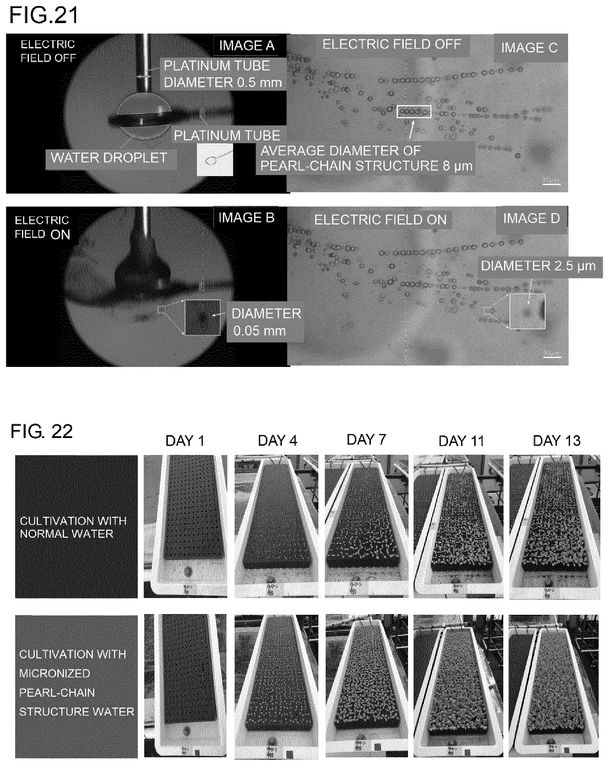

FIG. 21 illustrates the measurement result of particle diameters of water droplets. The liquid control apparatus of the present embodiment turns the state of electric field OFF in image A into the state of electric field ON in image B, whereby the moisture is split into small water droplet particles due to the application of an electric field. The particle diameter of one drop of water droplet particles held on a platinum electrode in image A is about 4.5 mm and its volume is about 0.05 cm³. In the state shown in image C, the average particle diameter of the split water droplet particles is 8 µm. In this case, one drop of water droplet particles held on the platinum electrode is split into about 180 million water droplet particles. Furthermore, in image D, the particle diameter of the water droplet particle group with the minimum particle diameter is 2.5 µm, even smaller than the average particle diameter in image C. In this case, one drop of the water droplet particles held on the platinum electrode is split into about 6.1 billion water droplet particles.

Conventional technologies for vibrating water droplets include known technologies using EMS, radio waves, ultrasonic waves, facial machines, Indiba, potential therapy devices, magnets, ores, terahertz waves, and the like. The particle diameter of water droplets vibrated by these technologies is 4500 µm. The particle diameter of raindrops is 1000 µm. The particle diameter of drizzle is 100 to 300 µm. The particle diameter of mist (haze) is 10 µm. The particle diameter of steam is 8 µm. In the present embodiment, the size of the water droplet particles obtained by the application of an electric field is 2.5 to 8 µm. Since the spacing between cells in a living body is 12 µm or less, the water droplets with a particle diameter of 12 µm or less obtained in the present embodiment have the property of easily penetrating between cells in a living body. In the present embodiment, the moisture particles atomized into fine particles by the application of an electric field penetrate between the cells of a living body such as animal and plant, resulting in a variety of efficacy.

FIG. 22 illustrates the improvement result in hydroponic cultivation of green leaf lettuce. The comparative photographs, especially on days 7, 11, and 13, show that the liquid control apparatus of the present embodiment contributes to the growth promotion of green leaf lettuce.

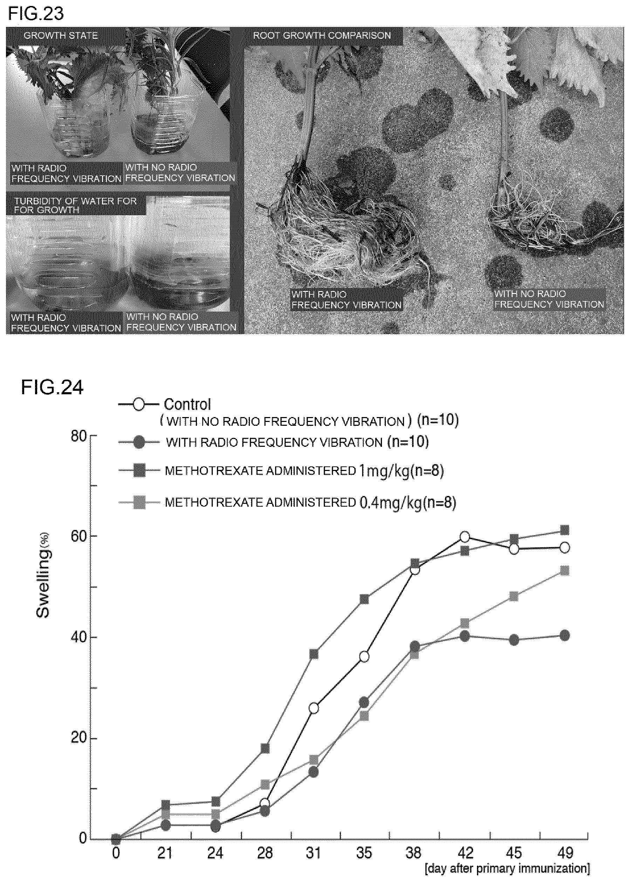

FIG. 23 illustrates the improvement result in perilla preservation. FIG. 23 shows comparison of water turbidity and the degree of growth of perilla, in a case where two groups of perilla of the same size and same quantity were kept at room temperature for two months with the equal amounts of water supply, one group with an electric field applied by the liquid control apparatus of the present embodiment and the other with no electric field applied. It is understood that the rooting of the perilla with an electric field applied is good. It is also understood that water is less turbid for the perilla with an electric field applied. FIG. 23 shows that the liquid control apparatus of the present embodiment contributes to the preservation of perilla and the improvement of water turbidity.

FIG. 22 and FIG. 23 indicate that the liquid control apparatus of the present embodiment can control the growth promotion of plants as well as animals by modifying and atomizing the moisture into fine particles. The liquid control apparatus of the present embodiment can be applied to control of enhancing any living bodies or cells.

FIG. 24 illustrates the improvement result of the edema rate of rheumatism. When radio frequency vibration was applied to a mouse model of chronic rheumatoid arthritis by the liquid control apparatus of the present embodiment, the edema rate was reduced by about 15 to 20% on days 38 to 49, indicating improvement in chronic rheumatoid arthritis. FIG. 24 shows that the edema improving effect greater than by administration of the anti-rheumatic drug methotrexate.

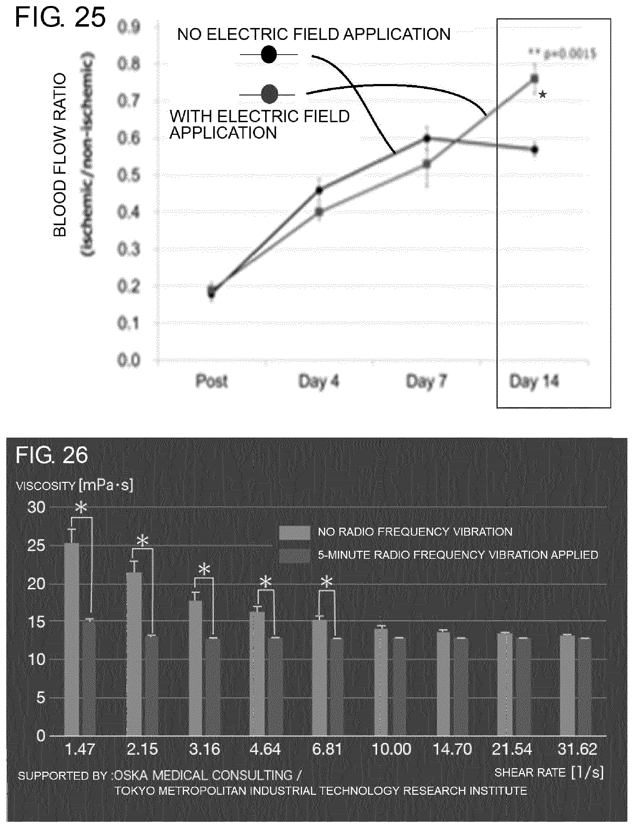

FIG. 25 illustrates the effect of improving blood flow in a mouse model of lower limb ischemia. When radio frequency vibration was applied to a mouse model of lower limb ischemia by the liquid control apparatus of the present embodiment, improvement in blood flow was observed on day 14. The mouse model of lower limb ischemia used in the experiment (exp. no. P180110, N=7) was generated from BALB/c male, 8-week-old mouse. Blood flow was measured in both limbs using a blood flow imaging device, moorFLPI (Moor Instruments Ltd.). The liquid control apparatus of the present embodiment makes the flow of intercellular fluid smooth, thereby promoting cell metabolism and keeping cell vitality. It took about two weeks to detect the blood flow improving effect in the form of data as a result of improved flow of intercellular fluid.

FIG. 26 illustrates the effect of reducing the viscosity of a highly viscous agent. MRI and radiographic contrast agents such as Iomeprol can be viscous and difficult to inject into patients, and their low fluidity can cause strong irritation in the patient's body. Increasing the fluidity of highly viscous medicines contributes to reduction of the burden on health care professionals and patients. The change in viscosity was observed with the application of an electric field by the liquid control apparatus of the present embodiment, as shown in FIG. 26. A decrease in viscosity was observed after 1 to 30 minutes of application. A significant decrease was observed with 5-minute application. The viscosity of Iomeprol was reduced to about 60%, that is, the viscosity was reduced by about 40% at maximum with the application of an electric field by the liquid control apparatus of the present embodiment. With the application of an electric field by the liquid control apparatus of the present embodiment, the fluid resistance was lowered and a change in high-speed shear state was observed. Since the electrode of the liquid control apparatus of the present embodiment can apply an electric field in a non-contact manner, there is no need for bringing a metal electrode into contact with a drug solution, which ensures sanitation.

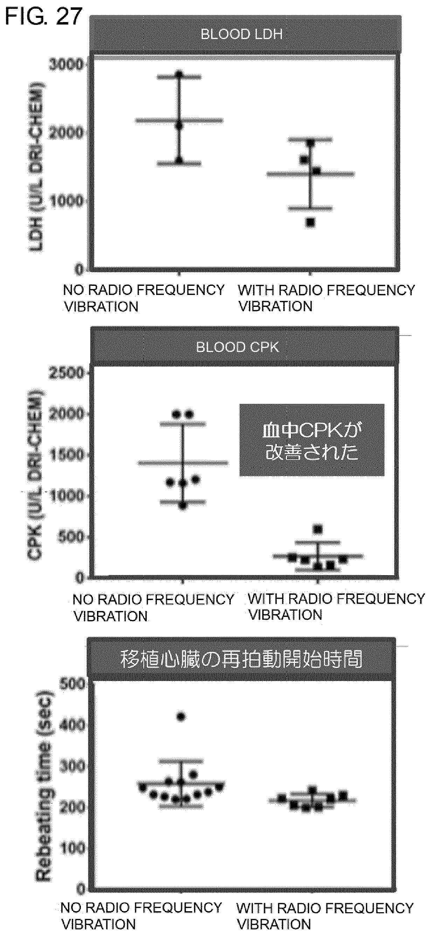

FIG. 27 illustrates the effect of improving blood CPK in a heart transplanted mouse. When radio frequency vibration was applied to a heart transplanted mouse by the liquid control apparatus of the present embodiment, improvement in blood CPK, which is an indicator of muscle and other tissue cell damage, was observed. In the middle graph in FIG. 27, the average value of blood CPK with no electric field applied is 1400, whereas the average value of blood CPK with an electric field applied is reduced to 200. CPK is an enzyme required for energy metabolism in muscle cells and is found in large amounts in skeletal, cardiac, and smooth muscle, as well as in the brain. Muscle damage increases blood CPK, which significantly increases, for example, in acute myocardial infarction and muscular dystrophy. In the upper graph in FIG. 27, which shows the measurement results of blood LDH, the average of LDH with no electric field applied is 2200, whereas the average of LDH with an electric field applied is about 1400, indicating that the application of an electric field by the liquid control apparatus of the present embodiment decreases blood LDH. The lower graph in FIG. 27 shows the measured start time of re-beating of the transplanted heart, which is unaffected by the liquid control apparatus of the present embodiment. The radio frequency vibration by the liquid control apparatus of the present embodiment is found to have a positive effect on muscle tissue damage repair by increasing energy metabolism in muscle cells, and can be applied to the treatment of myocardial infarction, angina pectoris.

The experimental results shown in FIG. 26 and FIG. 27 have indicated that the liquid control apparatus of the present embodiment can perform control of improving or enhancing the heart beat or pulse, and improve or prevent heart diseases.

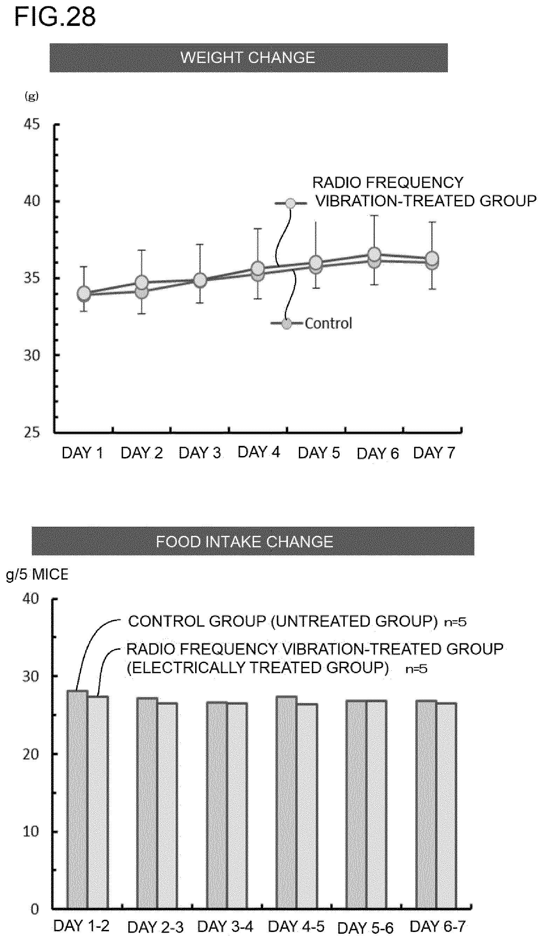

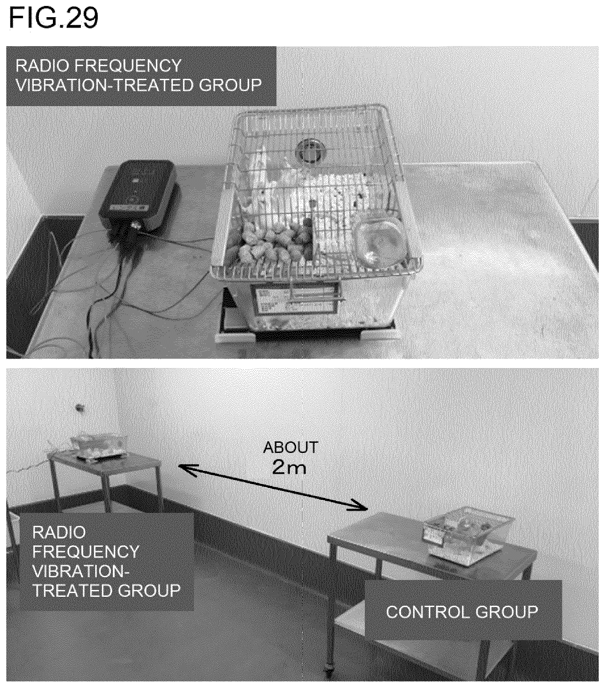

FIG. 28 illustrates the verification result of adverse reactions caused by electrical stimulation by radio frequency vibration. FIG. 29 illustrates experimental conditions in FIG. 28. The treatment group with electric field application was measured at a distance of about 2 m from the control group without electric field application and without the influence of electric field application. When normal mice were irradiated with radio frequency vibration by the liquid control apparatus of the present embodiment, there was no effect on their physical condition, and no change in body weight or food intake. Thus, safety was confirmed. Mice (Scl:ddY) used in the experiment were 9 weeks old (incoming 8 weeks old), weighing 32.66±1.09 g in the control group and 33.16±1.02 g in the stimulation group, male, and bred by Japan SLC, Inc. The stimulation time was 8:30 to 15:30, and the experiment was conducted for 7 consecutive days. As shown in FIG. 28, there was no change in body weight and food intake, indicating that the electrical stimulation by radio frequency vibration generated by the liquid control apparatus of the present embodiment does not affect the weight and food intake of the mice.

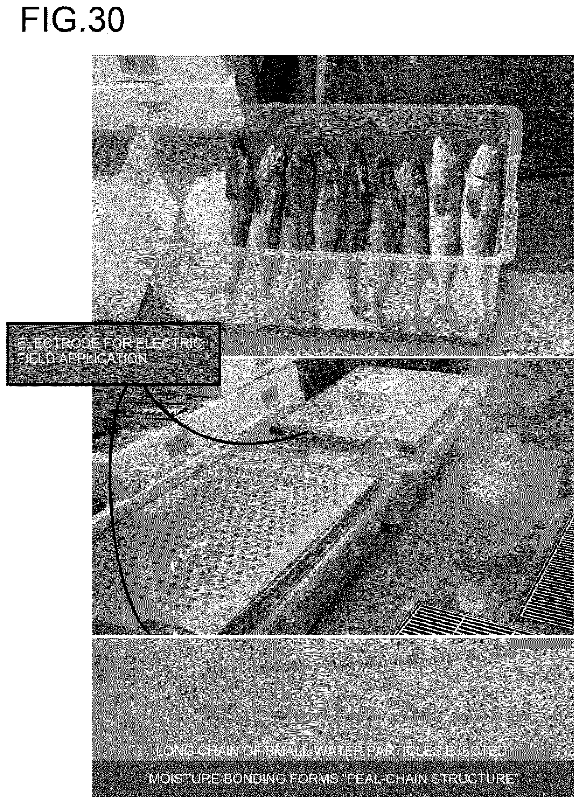

FIG. 30 illustrates the improvement result of rigor mortis and blood coagulation of fish. FIG. 31 illustrates a body decomposition preventing device. As shown in FIG. 30, a pair of flat plate electrodes was set horizontally on a case containing dead fish, and the liquid control apparatus of the present embodiment was used to apply radio frequency vibration to the fish immediately after death. Then, rigor mortis, blood coagulation, and dead smell were successfully prevented (verification location: Furubira Town, Hokkaido). Further, radio frequency vibration was applied for five minutes by the liquid control apparatus of the present embodiment to fish two days after purchase. Reduced fishy odor, improved resilient texture, and restored freshness were confirmed. Immediately after purchase, fish was incised only at the base of the tail, the artery was cut, and the fish was placed to stand on ice at an angle in a plastic case. Radio frequency vibration was applied by the liquid control apparatus of the present embodiment. Fish similarly placed as a comparative target had already blood coagulated and could not be bled out. For this reason, in the comparative target, it is necessary to gut the fish from the gills and drain the blood using water pressure. On the other hand, when radio frequency vibration was applied by the liquid control apparatus of the present embodiment, the viscosity of the fish blood decreased and the blood could be drained from the incision at the base of the tail.

The effect of preventing rigor mortis, blood coagulation, and dead smell in fish, the effect of restoring freshness, and the effect of reducing the viscosity of blood when draining blood, which were confirmed in the experiment in FIG. 30, can be applied to meats other than fish, as well as to storage of dead bodies. As shown in FIG. 31, the electrode of the liquid control apparatus of the invention of the present application is installed. The application of radio frequency vibration to a dead body has the effect of preventing decomposition and unusual odors, and can suppress the growth of bacteria and the progress of decomposition in the body bag. In addition, the storage period until cremation can be extended.

FIG. 32 illustrates a blood coagulation suppressing device. The effect of preventing rigor mortis, blood coagulation, and dead smell of fish, the effect of restoring freshness, and the effect of reducing the viscosity of blood when draining blood, which were confirmed in the experiment in FIG. 30, are applicable to meats other than fish, and also applicable to a blood coagulation preventing device. When an extracorporeal membrane oxygenation (ECMO) is used as shown in FIG. 32, the electrode of the liquid control apparatus of the invention of the present application is installed. The application of radio frequency vibration can suppress blood coagulation and reduce the risk of blood coagulation. As a result, it is possible to reduce the monitoring burden on health care professionals, such as periodic checks for blood coagulation during use of ECMO. Furthermore, in a dialysis machine, the electrode of the liquid control apparatus of the invention of the present application is installed and radio frequency vibration is applied, whereby blood coagulation in the dialysis machine is suppressed, and the risk of blood coagulation is reduced. In addition, in combination with the blood flow improving effect, contribution to shorter dialysis time and improved safety during dialysis is achieved.

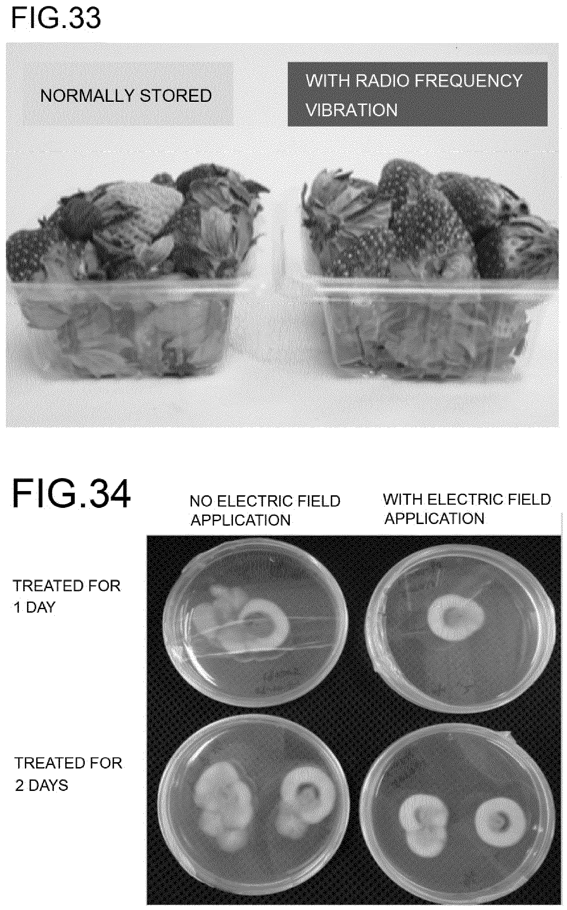

FIG. 33 illustrates the effect of suppressing mold of strawberries. The electrode of the liquid control apparatus of the invention of the present application was installed and radio frequency vibration was applied for one hour, followed by storage in a refrigerator for one month. A comparative target was stored in the refrigerator for one month without radio frequency vibration. As shown in FIG. 33, in the strawberries to which radio frequency vibration was applied by the liquid control apparatus of the invention of the present application (strawberries on the right side of FIG. 33), the free water in the strawberries form a pearl-chain structure by radio frequency vibration, and there is no free water to which molds, microorganisms, and germs bond. Therefore, even if molds, microorganisms, or germs exist on the surface of the strawberries, the molds, microorganisms, or germs do not grow, and the strawberries do not get moldy or rotten. On the other hand, in the strawberries in the comparative example (strawberries on the left side of FIG. 33), mold has grown and the strawberries get moldy.

FIG. 34 illustrates the effect of suppressing Bacillus anthracis growth. Bacillus anthracis was incubated for two days while the electrode of the liquid control apparatus of the invention of the present application was installed and radio frequency vibration was applied. As a comparative example, Bacillus anthracis was incubated for two days without radio frequency vibration. The radio frequency vibration applied by the liquid control apparatus of the invention of the present application inhibited the growth of Bacillus anthracis, compared with the comparative example.

The experimental results shown in FIG. 30 to FIG. 34 have indicated that, by applying an electric field from the electrode, the liquid control apparatus of the present embodiment performs control of improving the storage, freezing, thawing, culturing, or logistics of organs, body fluids, blood, cells, tissues, skin, hair, dead bodies, DNA, stem cells, sperms, eggs, medicines, cosmetics, or liquids, or improves an electrode or a container for use in the storage, freezing, thawing, culturing, or logistics of organs, body fluids, blood, cells, tissues, skin, hair, dead bodies, DNA, stem cells, sperms, eggs, medicines, cosmetics, or liquids.

Further, FIG. 33 and FIG. 34 have shown that the application of radio frequency vibration by the liquid control apparatus of the invention of the present application can suppress the growth of molds or Bacillus anthracis, and it was confirmed that even when molds, microorganisms, bacteria, germs, and viruses exist, the growth of the molds, microorganisms, bacteria, germs, and viruses was suppressed. When radio frequency vibration is applied to a human body by the liquid control apparatus of the invention of the present application, radio frequency vibration acts on free water in the body and cells to achieve a pearl-chain structure of free water. This is expected to suppress the growth of bacteria and viruses in the body. Thus, even in the presence of bacteria or viruses, their growth was suppressed, thereby achieving the infection preventing effect on bacteria and viruses.

Conventional measures to prevent infection of viruses aim to, for example, reduce the risk of infection by using existing coronavirus vaccine. However, if coronaviruses continue to mutate, it is necessary to develop effective vaccines against the mutant species each time. In contrast, the technique of suppressing the growth of molds and Bacillus anthracis by applying radio frequency vibration by the liquid control apparatus of the present embodiment shown in FIG. 33 and FIG. 34 is expected to contribute to suppression of initial symptoms caused by coronaviruses and prevention of severe symptoms if the coronaviruses do not grow in the lungs or other parts of the body infected with various mutant species of coronaviruses.

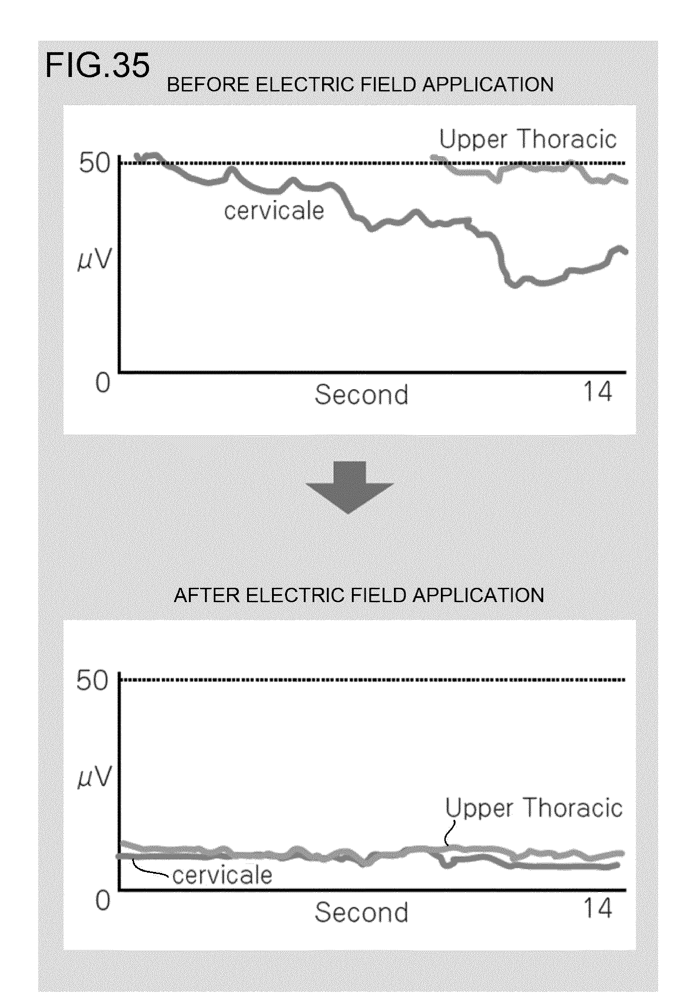

FIG. 35 illustrates the effect of improving masticatory balance by relaxing masseter tension. It has been confirmed that the application of an electric field to the masseter muscle (masticatory muscle) of the jaw for five minutes by the liquid control apparatus of the present embodiment decreased electromyograph (EMG) values and achieved muscle relaxation. In FIG. 35, the EMG values decrease in both the cervical spine (cervicale) and the upper thoracic spinal cord (upper thoracic).

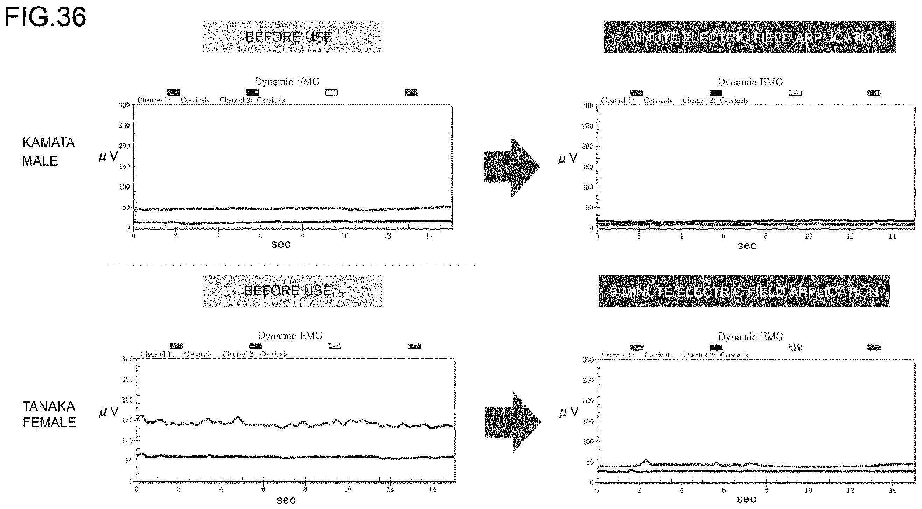

FIGS. 36 to 40 show the effect of improving the left-right balance of the body. Channel 1 of an EMG meter was attached to the left side of each body part and channel 2 to the right side to measure the left-right muscle balance. FIG. 36 shows the measurement result of sacrum 1. The acupuncture points at this location are related to bladder, ovary, or prostate. In the left graphs before use in FIG. 36, muscle strength varies between right and left. However, after an electric field was applied to the masseter muscle of the jaw for five minutes by the liquid control apparatus of the present embodiment, the difference between the left and right muscle strengths decreased in both Kamata's case and Tanaka's case, as shown in the right graphs, indicating that the left-right muscle balance was improved. In particular, in Tanaka's case, the values on the right graph are greatly reduced compared with those on the left graph, indicating that the left-right muscle balance was improved with muscle relaxation. In Kamata's case, muscle strength is balanced at the lower values after the application of an electric field.

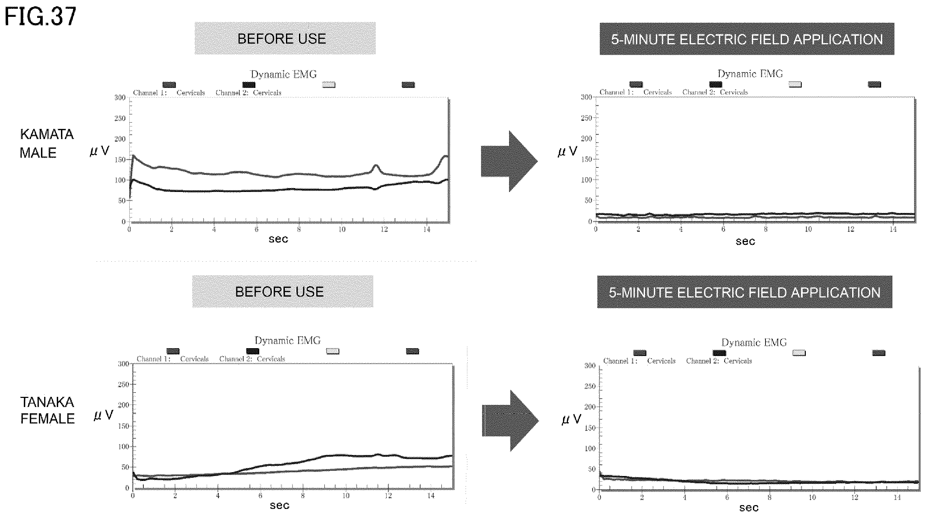

FIG. 37 shows the measurement result of thoracic vertebra 12. The acupuncture points at this location are related to kidney. In the left graphs before use in FIG. 37, muscle strength varies between right and left, and the EMG values are higher than those on the right graphs after the application of an electric field. After an electric field was applied to the masseter muscle of the jaw for five minutes by the liquid control apparatus of the present embodiment, the values decreased and the difference between the left and right muscle strengths decreased in both Kamata's case and Tanaka's case, as shown in the right graphs, indicating that the left-right muscle balance has improved with muscle relaxation.

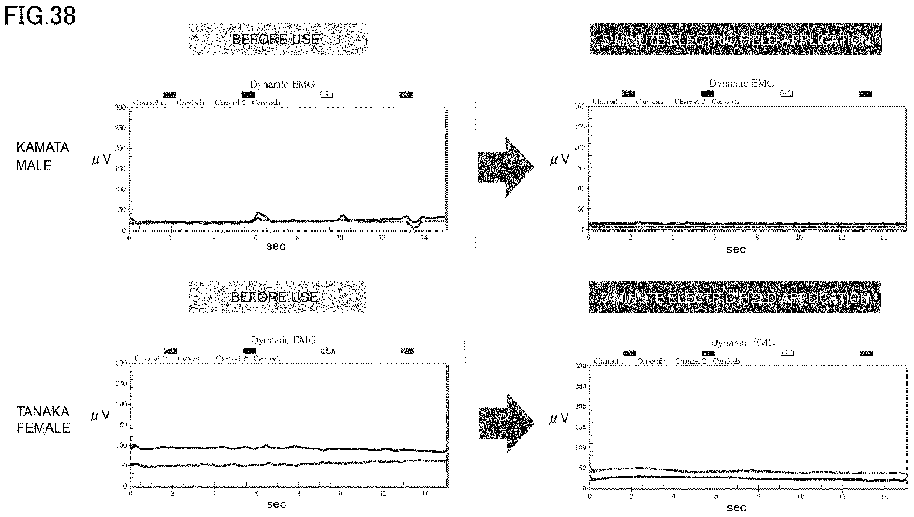

FIG. 38 shows the measurement result of thoracic vertebra 8. The acupuncture points at this location are related to liver. In the left graphs before use in FIG. 38, muscle strength varies between right and left particularly in Tanaka's case, and the EMG values are higher in both cases, compared with those on the right graphs after the application of an electric field. After an electric field was applied to the masseter muscle of the jaw for five minutes by the liquid control apparatus of the present embodiment, the values decreased in both Kamata's case and Tanaka's case, as shown in the right graphs, and the difference between the left and right muscle strengths decreased particularly in Tanaka's case, indicating that the left-right muscle balance has improved with muscle relaxation.

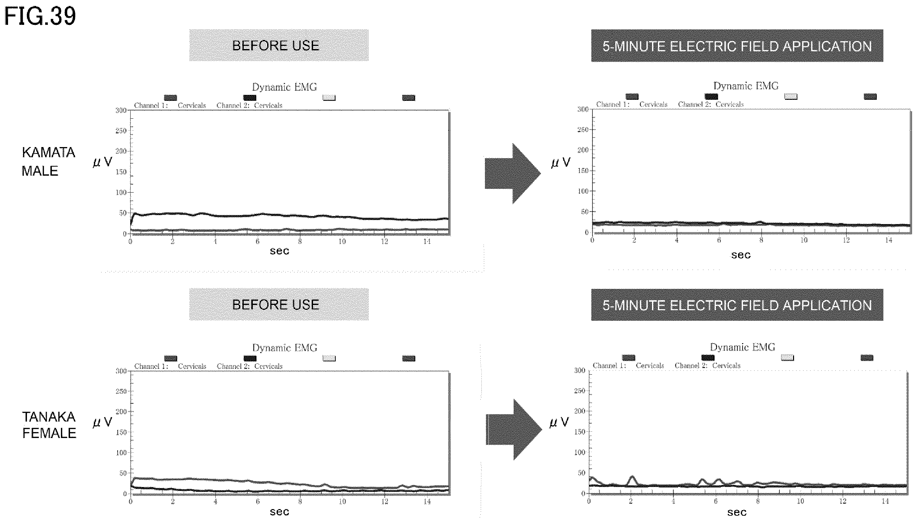

FIG. 39 shows the measurement result of thoracic vertebra 1. The acupuncture points at this location are related to heart and lungs. In the left graphs before use in FIG. 39, the EMG values vary greatly in both cases, compared with those on the right graphs after the application of an electric field. After an electric field is applied to the masseter muscle of the jaw for five minutes by the liquid control apparatus of the present embodiment, the difference between the left and right muscle strengths is reduced in both cases, and the values themselves are stably low.

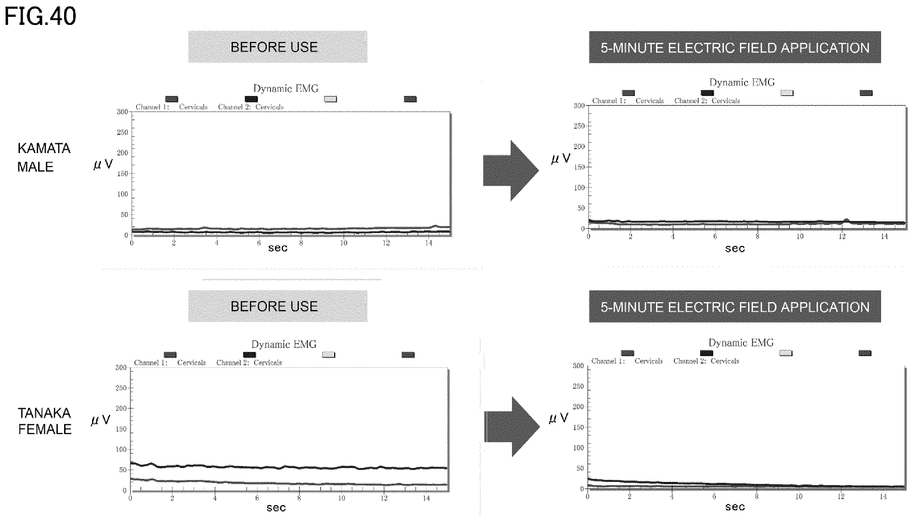

FIG. 40 shows the measurement result of cervical vertebra 3. The acupuncture points at this location are related to throat and paranasal sinuses. In the left graphs before use in FIG. 40, there is a great variation between the right and left EMG values, particularly in Tanaka's case, compared with the right graphs after the application of an electric field. After an electric field is applied to the masseter muscle of the jaw for five minutes by the liquid control apparatus of the present embodiment, the difference between the left and right muscle strengths is reduced in both cases, and the values themselves are stably low.

The measurement results from the EMG meter in FIG. 35 to FIG. 40 have indicated that the application of an electric field to the masseter muscle of the jaw for five minutes by the liquid control apparatus of the present embodiment relaxes the tension of the entire muscle through the masticatory muscle, thereby adjusting the left-right balance of the body as a whole. FIGS. 35 to 40 have shown that, by applying an electric field, the liquid control apparatus of the present embodiment adjusts mastication balance, muscle balance, and skeletal balance, and thereby performs control of improving or preventing muscle imbalance and performs control of improving or enhancing body balance, myofascial balance, occlusion, osteopathy, and manual therapy.

The following are evaluation comments by the subjects on the control by applying an electric field from the electrode by the liquid control apparatus of the present embodiment. In the evaluation comments, "Bodystation" refers to the liquid control apparatus of the present embodiment.
- The range of motion of the deep fascia was increased and muscle tension was reduced.
- The site of subcutaneous injection of insulin was indurated, but the skin in the area became soft.
- Having diabetes for 20 years, a normal value was exhibited for the pancreas after use three times.
- Having uterine cancer and severe heartburn due to cachexia, and extreme weight loss. Every attempt for improvement failed. On Bodystation for one hour just once, the heartburn was completely gone and I was able to enjoy my meals.
- The pain in the left ovary was gone away.
- I was supposed to take Mercazole for a year for Basedow's disease. Returned to the normal level on Bodystation for 1.5 hours just once. The doctor said it was hard to believe.
- I had involuntary movements (legs jumping up on their own) due to Parkinson's disease, but they disappeared after only one hour Bodystation just once.
- Bone pain was removed.
- Swelling of hands and feet can be removed.
- A person who experienced a stroke felt much lighter in head.
- Drainage of the semicircular canals has improved in a person with Meniere's disease.
- A person with rheumatism for many years becomes painless and grip by hand becomes easy.
- Bodystation for one hour only three times eliminated the need for an oxygen carrying machine.
- I sprained my right ankle badly and had bleeding internally in the size of a card. In spite of taping two hours later, I could not sleep that night due to extreme pain and was limping the next day, but two days later I could walk normally and go down stairs normally. There was no swelling at all.
- I had been suffering from diabetes for two years, but after just one hour on the BodyStation, my blood sugar level dropped significantly from 252 to 113 the next day, and 102 a month later.
- A person who was paralyzed on the left side of his body due to a stroke recovered to be able to travel alone six months later, simply by placing BodyStation under a pillow.
- I had rheumatism and could not bend my right middle finger, but after an hour on the BodyStation, I was able to bend it to a right angle.
- I had suffered from rheumatism for many years and could only descend stairs one at a time, but after one hour on BodyStation for the first time, I was impressed by being able to descend stairs continuously.
- I have had diabetes for 10 years and have had insulin injections and dialysis. Thanks to BodyStation, the blood sugar level dramatically decreased from 650 to 150 in half a year.
- I had juvenile Parkinson's disease and had to take medication, but after just one hour on BodyStation, the spasm stopped.
- Just an hour on BodyStation took away the pain in the lower left inner leg from shingles.
- Twelve years ago, I had an aftereffect of surgery for varicose veins in my right thigh, which caused pressure and pain from the inside to the outside of my leg and it looked like an elephant's foot. After just one hour on BodyStation, the swelling and pain went away and my legs felt lighter.
- Just one hour on BodyStation increased the range of motion of the frozen shoulder.
- A person with lumbar spinal stenosis with all five spinal canals narrowed was able to jog and play a round of golf after one hour of energized acupuncture insertion into the side of the spine, and as usual the next day.
- Left goiter, Basedow's disease were diagnosed by a blood test. BodyStation was applied to the left cervical armpit, thyroid gland, swelling site, and lymph nodes. The swelling became smaller, and two days later, during an examination at the prefectural hospital, the doctor in charge was surprised.
- The pain in the right arm from using a chainsaw for farmwork was relieved after one hour on BodyStation. Until then, for about half a month, I could not sleep because my elbow hurt at night.
- The entire right arm was sore from weeding, and I could not bend and stretch the elbow, but they became painless.
- The whole body becomes lighter.
- I had heavy, thick legs and I couldn't recognize my ankles, but they became thin and light.
- Sprained neck from stiff shoulders. Stiff shoulders, slight occipital headache.
- At 1:00 a.m., fell asleep with BodyStation on the shoulders and back. Wake up in the morning with no difficulty in breathing. It feels like a lot of air coming in. Neck and shoulders feel soft and fluffy. The pain goes away completely.
- When I started riding BodyStation, my personal trainer was surprised with myofascial release in my thighs.
- After just one hour on BodyStation, urination once every day became 3 times a day for a week. Swelling and lassitude in legs are gone and legs are light.
- I had a regular buildup of water in my knee and had it drained each time at the hospital. It built up again with pain, so I was on BodyStation until I went to the hospital, and the doctor told me that the water was gone.
- The gingiva was lowered with age, and the maxillary mucosa was surgically implanted.
   Implant surgery was scheduled, but after a month's ride, the gingiva returned to normal position. This is impossible with today's medicine.
- I was scheduled to have surgery, but my knee, which had been unbendable due to joint rheumatism, was bent 90 degrees.
- My serum lipids, called TG, have been 380 for many years (last November, the most recent data), but after 2 weeks of riding, they are now 130 on July 8. The doctor said that this was not normally possible.
- Contracted cellulitis, and the right leg became enlarged like an elephant's foot. The swelling then reduced within two weeks. It usually takes a month or more.

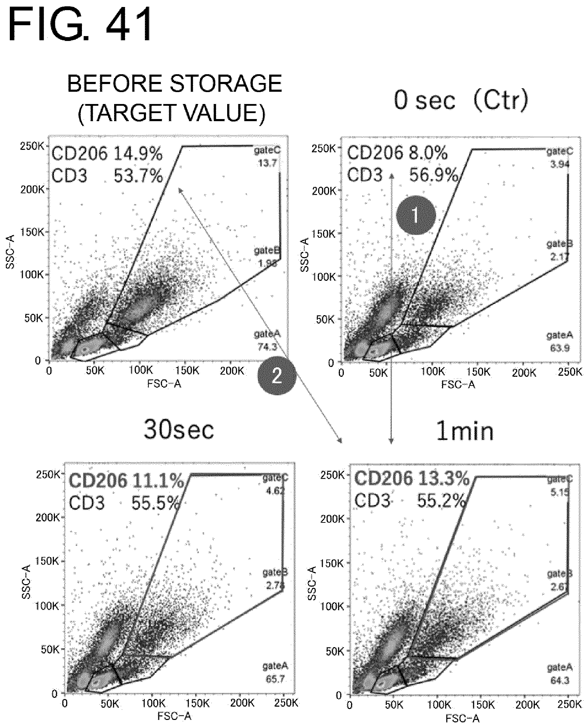

FIG. 41 illustrates the effect of enhancing the viability of human cultured cells. Peripheral blood mononuclear cells are expected to play an important role in regenerative medicine, but it is difficult to preserve them for more than 48 hours in an active state. They may be frozen and stored using a preservation solution such as DMSO, but washing is required at the time of injection. The application of an electric field can extend the storage period. In some cases, the application of radio frequency vibration improved the viability of CD206. The upper left graph shows the target value, and the viability of CD206 is targeted at 14.9%. In contrast, the viability of CD206 improved to 8.0% in the upper right graph with no electric field applied and to 11.1% in the lower left graph with an electric field applied for 30 seconds. In the lower right graph with an electric field applied for one minute, the viability of CD206 improved to 13.3%. CD206 is a type of macrophage that plays a prominent role in angiogenesis, which is important during tissue regeneration. For (1) in FIG. 41, the number of CD206 cells was about 1.7 times larger in the sample with radio frequency vibration applied for one minute, than in the sample with no vibration applied. For (2) in FIG. 41, when the sample before adjustment, that is, the fresh sample before storage is compared with the sample with radio frequency vibration applied for one minute, the number of cells decreased by only 1.6%. FIG. 41 has indicated that the application of an electric field by the liquid control apparatus of the present embodiment is effective in enhancing the viability of human cultured cells. Based on this, it has been shown that the liquid control apparatus of the present embodiment performs control of improving or enhancing the formation of teeth, bones, joints, blood vessels, lymph vessels, nerves, cells, skin, hair, or organs, performs control of improving the storage, freezing, thawing, culturing, or logistics of organs, body fluids, blood, cells, tissues, skin, hair, dead bodies, DNA, stem cells, sperms, eggs, medicines, cosmetics, or liquids, or improves an electrode or a container for use in the storage, freezing, thawing, culturing, or logistics of organs, body fluids, blood, cells, tissues, skin, hair, dead bodies, DNA, stem cells, sperms, eggs, medicines, cosmetics, or liquids.

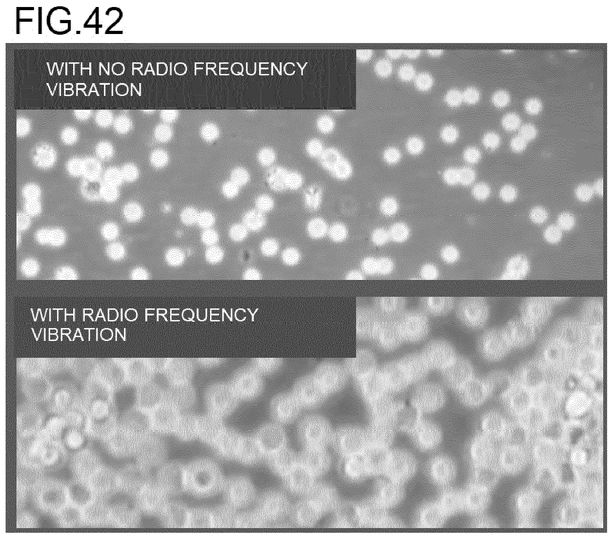

FIG. 42 illustrates the observation result of peripheral blood mononuclear cells. When radio frequency vibration is applied, cells aggregate on the culture dish. By applying an electric field, the liquid control apparatus of the present embodiment can perform control such that cells are arranged in a pearl-chain structure. As shown in FIG. 41, the viability of macrophage CD206, which is important for angiogenesis, may be higher. Although the effect varied from subject to subject, the predominant effect was confirmed. FIG. 41 and FIG. 42 have shown the function of forming organs and tissues by arranging cells by applying an electric field by the liquid control apparatus of the present embodiment to perform control such that the cells are arranged in a pearl-chain structure.

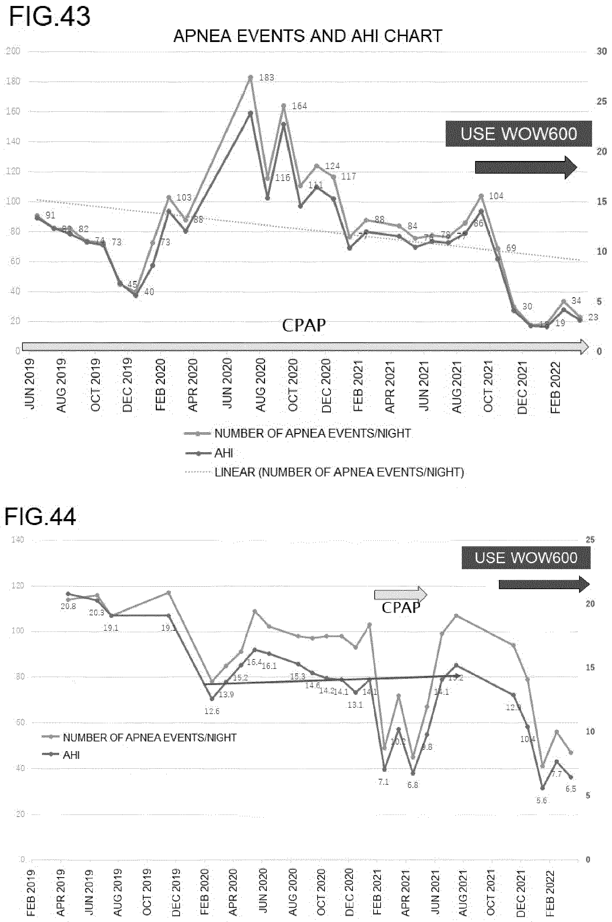

FIG. 43 illustrates a sleep improvement example 1, and FIG. 44 illustrates a sleep improvement example 2, both of which show the effect of improving an apnea-hypopnea index. With the use of continuous positive airway pressure (CPAP) during sleep, there was a decrease in the number of apnea events and in apnea-hypopnea index (AHI, the total number of apnea and hypopnea events per hour). However, the number of apnea events and AHI increased again after the end of use of CPAP. Instead of CPAP, an electric field was applied from the electrode by the liquid control apparatus of the present embodiment, and the number of apnea events and AHI were determined. Then, a decrease in the number of apnea events and in AHI was observed, which was similar to that observed when CPAP was used. The application of an electric field by the liquid control apparatus of the present embodiment was started after September 2021 in FIG. 43, and then the number of apnea events and AHI decreased significantly. Furthermore, it was observed that the use of CPAP in addition to the application of an electric field from the electrode by the liquid control apparatus of the present embodiment suppressed the number of apnea events and AHI more than when CPAP was used alone. FIG. 44 shows data for another subject in FIG. 43. The application of an electric field by the liquid control apparatus of the present embodiment was started after October, 2021, and then the number of apnea events and AHI decreased significantly.

In the present embodiment, in terms of promoting improvement of apnea syndrome, the mechanism of effect is that the application of an electric field from the electrode by the liquid control apparatus of the present embodiment micronizes the moisture in blood of the carotid arteries and veins and spinal arteries and veins, improves blood flow around the neck, removes muscle tension around the trachea, and returns the position of the cervical spine to normal. This prevents the lower jaw from rising too high, suppresses the sinking of the tongue, and relaxes tracheal contractions, resulting in smoother breathing. As a result, apnea symptoms are alleviated. Although not limited to any particular part, the electrode according to the present embodiment can be applied to, for example, 5 cm on both sides from the center of the larynx which is the most protruding portion.

The experimental results shown in FIG. 43 and FIG. 44 have indicated that, by applying an electric field from the electrode, the liquid control apparatus of the present embodiment can improve breathing, perform control of improving or enhancing respiratory function or lung function, and perform control of improving sleep.

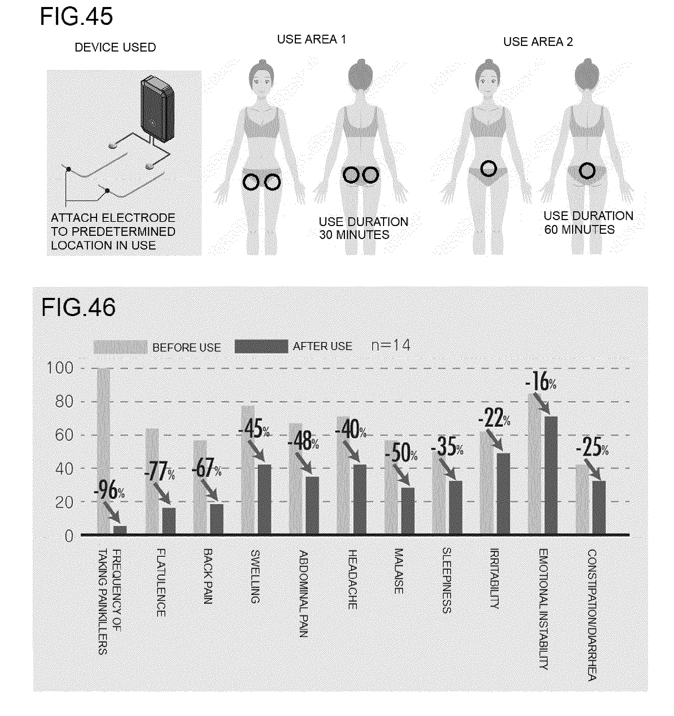

FIG. 45 illustrates an experiment on menstrual pain improvement. FIG. 45 shows the improvement in menstrual pain. Fourteen women (25 to 53 years old) who were taking painkillers for menstrual pain used the liquid control apparatus of the present embodiment as shown in FIG. 45 and answered a questionnaire for any changes in their menstruation before and after using the device. The results are shown in FIG. 46. As shown in FIG. 46, with application of an electric field from the electrode by the liquid control apparatus of the present embodiment, significant improvements were obtained in the following items: frequency of taking painkillers; flatulence; back pain; swelling; abdominal pain; headache; malaise; sleepiness; irritability; emotional instability; and constipation/diarrhea.

Conventional methods to relieve pain include warming the body, bathing, exercise, local cooling, massage, stretching, relaxation, counseling, pressing the acupuncture points, pelvic care, use of analgesics or anesthesia, and patience. With the liquid control apparatus of the present embodiment, the body fluid micronization control improves the fluidity of moisture and improves body fluid metabolism. Thus, it is possible to perform control of improving menstruation, PMS, infertility/fertility, change of life/menopause, pregnancy, postpartum care, general women's health, and symptoms such as stress and anxiety. The following evaluations were received from the subjects regarding the effect of improving the female physical condition by the body fluid micronization control by the liquid control apparatus of the present embodiment.
- My daughter's menstrual pain was gone.
- The pain in the left ovary has disappeared.
- Monthly ovulation pain has disappeared.
- Cold sensitivity may be alleviated.
- I was skeptical before trying it, but after applying it to my lower abdomen for an hour every day, the menstrual pain that had plagued me for over 20 years was completely gone. I was taking monthly painkillers, but no longer needed them, and my menstrual period, which used to last six to seven days, was reduced to four days.
- I tried it for three months, thinking the effect with only one time use was a fluke, but the effect lasted. The dull mood caused by menstrual pain has improved.
- Monthly menstrual period of 7 days ended in 4 days.
- The menstrual period went from 5 days to 3 days.
- With the use two days before the start of menstrual period, the painkillers I was taking every month was no longer necessary.
- In testing for only a few days, if the usual pain is 100%, the pain after using WOW200 was about 30%.
- Bleeding lingered even when my period was almost over, but now it is over in a snap.
- I think my make-up looks better.
- I think the swelling in my legs and face may have decreased.
- I was not able to do it every day, but I still felt enough change. In the first month, bleeding was over in 3 days and bleeding started again on the fifth day but it was over in 3 days. Total of 6 days. In the second month, the period came 20 days later. It was over just on the third day. Bleeding always lingered and I can do without pads for a week, but I was surprised at how bleeding ends. I had no menstrual pain (and usually very little), and menstrual blood on the first day was more than before and clean. I get constipated before my period, but in the second month I had my period with a good bowel movement, which I appreciated.

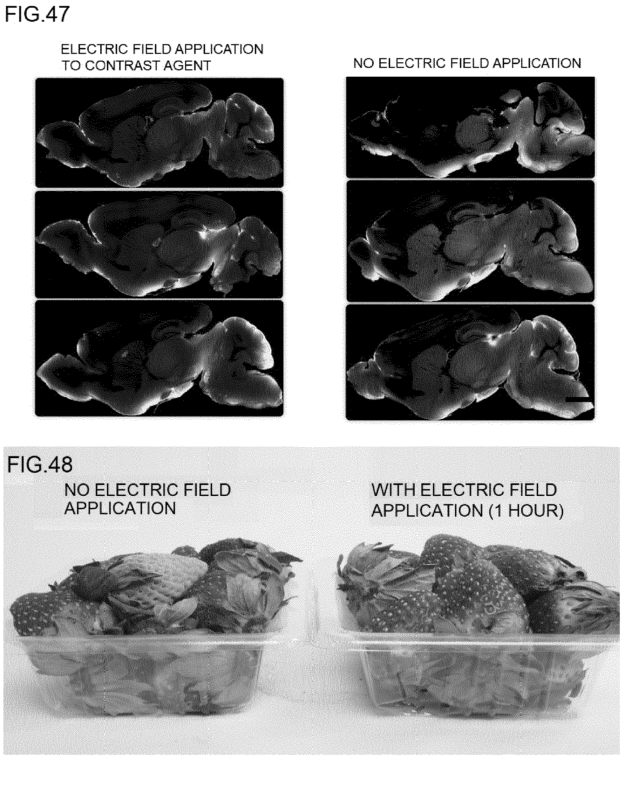

FIG. 47 illustrates the effect of improving a contrast agent. After an electric field was applied from the electrode to the contrast agent for 24 hours by the liquid control apparatus of the present embodiment, the contrast agent was injected from the mouse cisterna magna, and 30 minutes after the injection, the contrast agent seepage into the cerebral cortex was checked by imaging. As a comparative example, a contrast agent to which no electric field was applied was used. The average brightness for three mice was measured, and the average brightness of the mouse injected with the contrast agent to which an electric field was applied by the liquid control apparatus of the present embodiment was 19.7, while the average brightness of the mouse injected with the contrast agent to which no electric field was applied was 8.8. It was confirmed that the application of an electric field to the contrast agent by the liquid control apparatus of the present embodiment achieves the effect of facilitating the seepage of the contrast agent into the cerebral cortex of mice. The effect achieved by the present embodiment is not limited to the cerebral cortex of mice, and the effect of improving the penetration of contrast agents is also expected for other cells, tissues, and organs.

FIG. 48 illustrates the effect of preventing mold and rotting. The photographs are to compare strawberries stored in a refrigerator for 30 days after an electric field was applied from the electrode of the liquid control apparatus of the present embodiment for one hour. In the photograph on the right side, the strawberries, to which an electric field was applied, are fresh, not rotten, and free from molds. On the other hand, the comparative example in the photograph on the left side is strawberries stored in a refrigerator for 30 days without the application of an electric field. In the comparative example, the strawberries are rotten and mold growth is observed. This indicates that the application of an electric field by the liquid control apparatus of the present embodiment is effective in preventing rotting and molds.

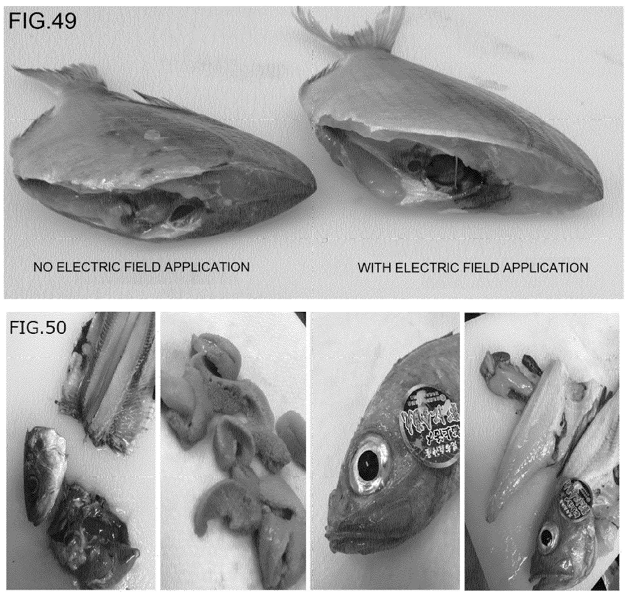

FIG. 49 illustrates the effect of preventing fish rotting. The photograph shows a comparison of sea bream stored in a refrigerator for five days after an electric field was applied from the electrode of the liquid control apparatus of the present embodiment for one hour. The sea bream on the right side in the photograph, to which an electric field was applied, is fresh and not rotten in the guts, and the freshness is kept. In contrast, the comparative example in the photograph on the left is sea bream stored in a refrigerator for five days with no electric field applied. In the comparative example, the meat of the fish is dried with rotten guts, and the freshness is lost. In the comparative example, the guts of the sea bream are rotten, because germs, viruses, or active oxygen bonds to free water (hydrogen bonding), causing the growth of germs or viruses and activation of active oxygen. In contrast, with application of an electric field by the liquid control apparatus of the present embodiment, free water inside the sea bream was controlled to form a pearl-chain structure, resulting in bonded water. Thus, the germs, viruses, and active oxygen was separated from the free water, whereby the growth of germs and viruses was prevented and the amount of active oxygen was reduced. Thus, it is understood that the application of an electric field by the liquid control apparatus of the present embodiment has the effect of suppressing the growth of microorganisms, bacteria, fungi, and viruses, the effect of preventing rotting, and the effect of reducing active oxygen.

FIG. 50 illustrates the effect of maintaining freshness. The leftmost photograph in FIG. 50 shows sardines stored in a refrigerator for three days after an electric field was applied for one hour by the liquid control apparatus of the present embodiment. The second photograph from the left in FIG. 50 shows sea urchin stored in a refrigerator for seven days after an electric field was applied for one hour by the liquid control apparatus of the present embodiment. The rightmost photograph and the second photograph from the right in FIG. 50 show broadbanded thornyhead stored in a refrigerator for seven days after an electric field was applied for one hour by the liquid control apparatus of the present embodiment. Compared with a comparison example, which is not shown, stored in a refrigerator for the same days with no electric field applied, when an electric field was applied by the liquid control apparatus of the present embodiment, the meat was fresh, the guts were not rotten, and the freshness was kept. With application of an electric field by the liquid control apparatus of the present embodiment, free water inside tissues and cells was controlled to form a pearl-chain structure, resulting in bonded water. Thus, the germs, viruses, and active oxygen were separated from the free water, whereby the growth of germs and viruses was prevented and the amount of active oxygen was reduced. Thus, it is understood that the application of an electric field by the liquid control apparatus of the present embodiment has the effect of suppressing the growth of microorganisms, bacteria, fungi, and viruses, the effect of preventing rotting, and the effect of reducing active oxygen.

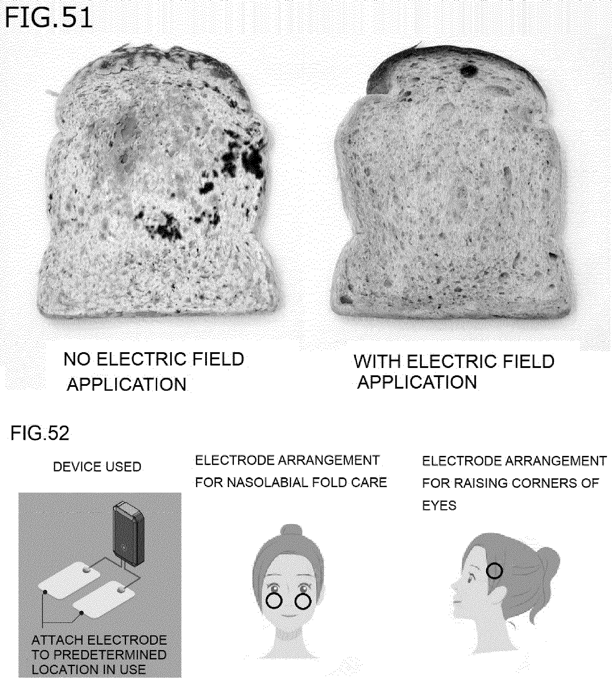

FIG. 51 illustrates the effect of preventing bread mold. The right side of FIG. 51 is a photograph of rye bread stored at room temperature for 13 days after an electric field was applied for one hour by the liquid control apparatus of the present embodiment. In the photograph on the right, there is no mold on the rye bread. In contrast, the comparative example in the photograph on the left shows rye bread stored at room temperature for 13 days without the application of an electric field. In the comparative example, mold has grown. In the comparative example, the mold has grown on the rye bread because the mold bonds to the free water (hydrogen bonding) in the rye bread and the mold grows. With application of an electric field by the liquid control apparatus of the present embodiment, the free water inside the rye bread was controlled to form a pearl-chain structure, resulting in bonded water. Thus, the mold was separated from the free water, whereby the growth of molds was prevented. Thus, it is understood that the application of an electric field by the liquid control apparatus of the present embodiment has the effect of suppressing the growth of microorganisms, bacteria, fungi, and viruses, the effect of preventing rotting, and the effect of reducing active oxygen.

The experiments shown in FIG. 48 to FIG. 51 have indicated that, by applying an electric field, the liquid control apparatus of the present embodiment performs control of preventing the growth of microorganisms, bacteria, fungi, or viruses, performs control of improving the storage, freezing, thawing, culturing, or logistics of organs, body fluids, blood, cells, tissues, skin, hair, dead bodies, DNA, stem cells, sperms, eggs, medicines, cosmetics, or liquids, improves an electrode or a container for use in the storage, freezing, thawing, culturing, or logistics of organs, body fluids, blood, cells, tissues, skin, hair, dead bodies, DNA, stem cells, sperms, eggs, medicines, cosmetics, or liquids, or performs control of removing active oxygen.

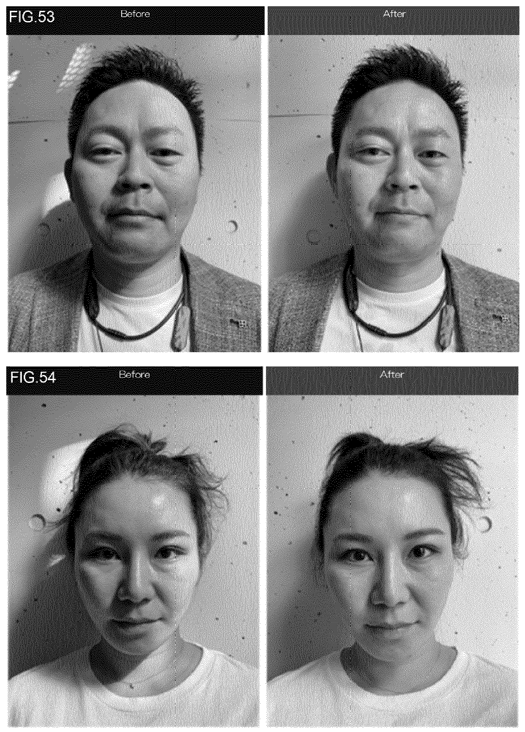

FIG. 52 illustrates a freckles/wrinkles improving device. FIGS. 53, 54, and 55 are comparative photographs before and after the application of an electric field by the electrode of the liquid control apparatus of the present embodiment for 20 minutes. The left diagram in FIG. 52 shows the device used. The middle diagram in FIG. 52 shows the electrode arrangement for nasolabial fold care. The right diagram in FIG. 52 shows the electrode arrangement for raising the corners of the eyes. The comparative photographs in FIGS. 53, 54, and 55 suggest that wrinkles are improved and the corners of the eyes are raised after an electric field is applied by the electrode of the liquid control apparatus of the present embodiment. It is also effective in removing freckles as well as improving wrinkles. At the same time, it has been shown that the liquid control apparatus of the present embodiment improves the body fluid metabolism and therefore is effective in reducing swelling and improving skin gloss due to improved blood flow, thereby improving cosmetic effects.

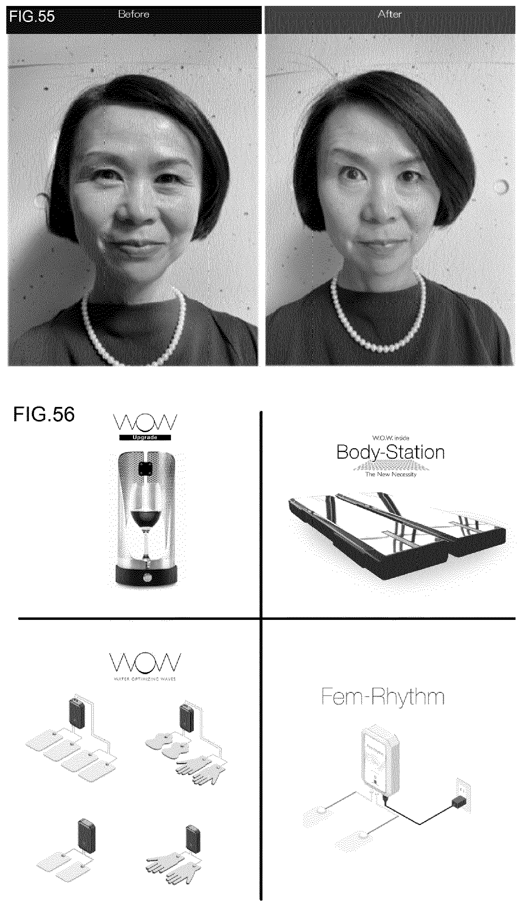

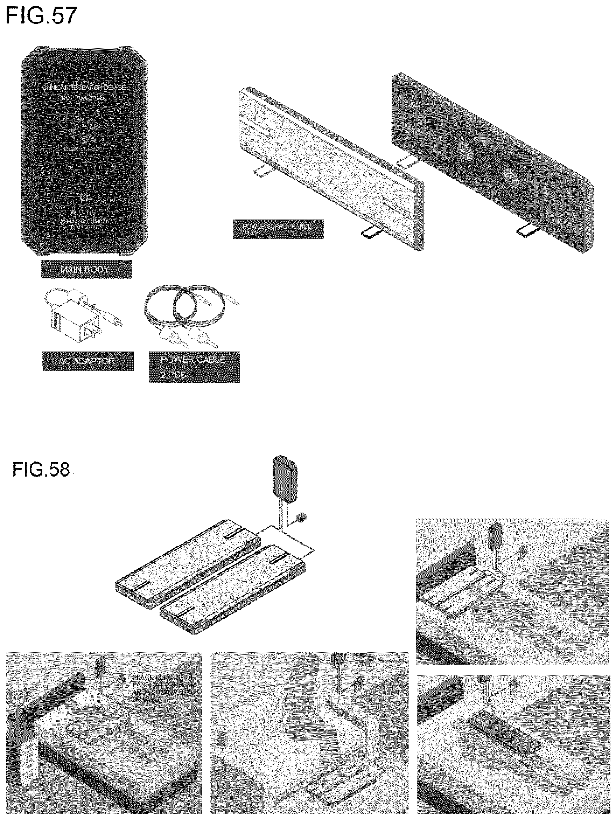

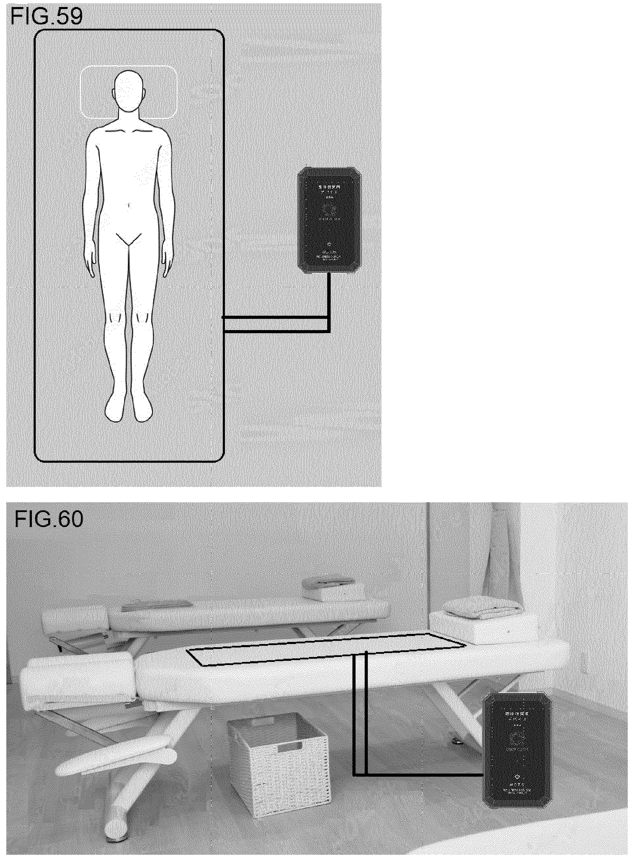

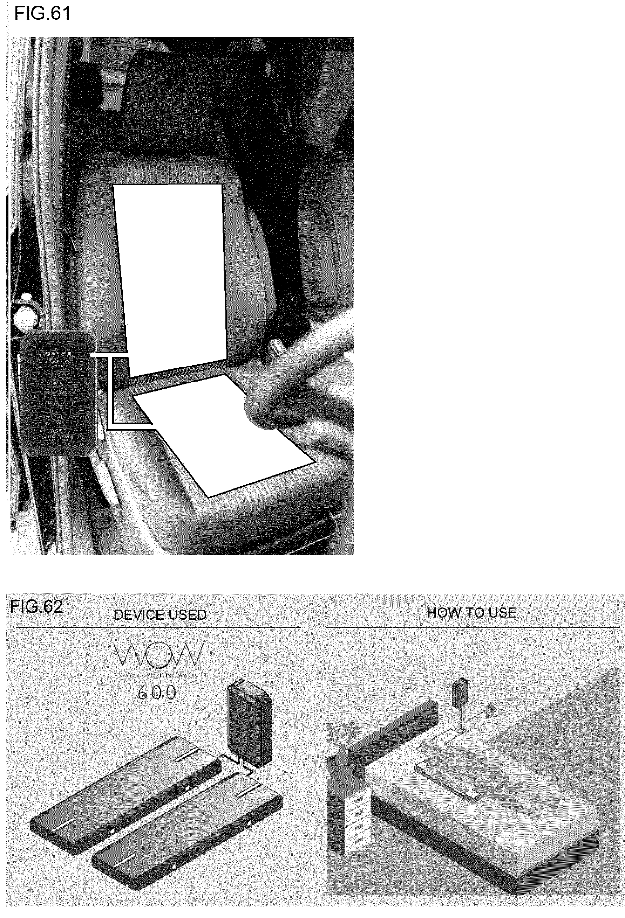

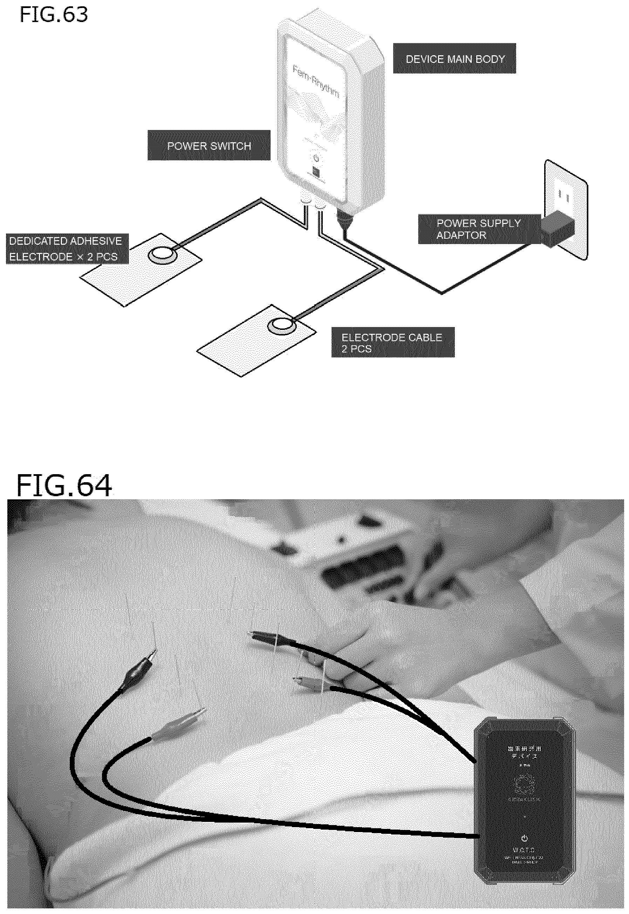

FIG. 56 illustrates an electrode structure example 1. FIG. 57 illustrates an electrode structure example 2. FIG. 58 illustrates an electrode structure example 3. FIG. 59 illustrates an electrode structure example 4. FIG. 60 illustrates an electrode structure example 5. FIG. 61 illustrates an electrode structure example 6. FIG. 62 illustrates an electrode structure example 7. FIG. 63 illustrates an electrode structure example 8. FIG. 64 illustrates an electrode structure example 9. In the electrode structure example 1 in FIG. 56, the upper left is a cylindrical electrode, which is suitable for applying an electric field to a cylindrical liquid container such as a bottle-shaped container. The upper right is a pair of plate electrodes. For example, a part of the body such as a foot can be placed on the electrodes, or the body can lie down on the electrodes. The lower left is two or four plate-shaped or flexible sheet-shaped electrodes. The plate-shaped electrode can be placed on or attached to a target site, or a body part can be placed on the plate-shaped electrode. The flexible sheet-shaped electrode is not limited to any particular shape, and can be shaped like a glove or a pad. The shape can be selected according to the target site. The lower right is an example of two plate-shaped electrodes. The plate-shaped plate can be placed on or attached to a target site. The method of fixing the electrode to the treatment site is not limited. For example, the electrode can be fixed with a band, dressing, net, supporter, or the like. The electrode structure example 2 in FIG. 57 has two electrode panels. The electrode panels are plate-shaped. The electrode panels can be installed in any orientation. For example, the electrode surface can be used in a vertical orientation, the electrode surface may be used horizontally, or the electrode surface can be inclined at any angle. The electrode panel can be placed on the target site, or the target site can be placed on the electrode. The electrode structure example 3 in FIG. 58 shows another use of the electrode panels in FIG. 57. In the upper right illustration, the electrode panel can be laid like a pillow on a part of the bed that touches the head such that the electrode surface is horizontal, and the head, which is the target site, can be placed on the electrode panel. In the lower left illustration, the electrode panel can be laid on a part of the bed that touches the hips, back, legs, and the like such that the electrode surface is horizontal, and the hips, back, legs, or the like, which is the target site, can be placed on the electrode panel. In the lower center illustration, the electrode panel can be laid on the floor such that the electrode surface is horizontal, and the feet, which are the target sites, can be placed on the electrode panel such that the soles of the feet touch the electrode surface. In the lower right illustration, one electrode panel is laid on the bed along the back such that the electrode surface is horizontal, and the back, which is the target site, is placed on the electrode panel. In addition, another electrode panel is used on the stomach side such that the electrode surface is horizontal. In this way, the installation position and orientation of the electrode panel and how it is applied to the target site are not limited as long as the electric field generated from the electrode panel can be applied. The number of electrode panels is not limited to two and may be one, three, four, or more. The electrode structure example 4 in FIG. 59 is an example in which a sheet-shaped electrode is arranged on a bed. Although not limited to any particular material, the sheet-shaped electrode is made of a flexible material and can be formed, for example, as an electrode in which a pair of comb-shaped electrodes are placed in close proximity to each other such that their comb teeth are alternately disposed. In this way, a plurality of electrodes can be integrated into a single sheet-shaped electrode. In the electrode structure example 5 in FIG. 60, a sheet-shaped or plate-shaped electrode is arranged on the top surface of a treatment bed, a therapy bed, a cot, or the like.
In the electrode structure example 6 in FIG. 61, a sheet-shaped or plate-shaped electrode is arranged on the driver's seat of a car. The application of an electric field from the electrode arranged on the driver's seat improves the fluidity of body fluids and body fluid metabolism, which is effective in preventing deep vein thrombosis. The electrode structure example 7 in FIG. 62 shows another use example of the electrode panel in FIG. 57, in which two electrodes are arranged in parallel along the back on the bed, and the back, which is the target site, is placed on the electrodes. In the electrode structure example 8 in FIG. 63, two adhesive terminals are provided. Since the adhesive terminals each have an adhesive electrode surface, the electrodes can be attached to the target site without the use of a separate band or the like. In the electrode structure example 9 in FIG. 64, needle-shaped electrodes are used. The electrodes are needle-shaped, and an electric field is applied from the needles to the target site by applying voltage to the needles punctured into the target site in the manner of needle therapy. In this way, for example, the electrode can have a shape of plate, rod, sheet, needle, or comb, or a shape combining two or more electrodes with each other.

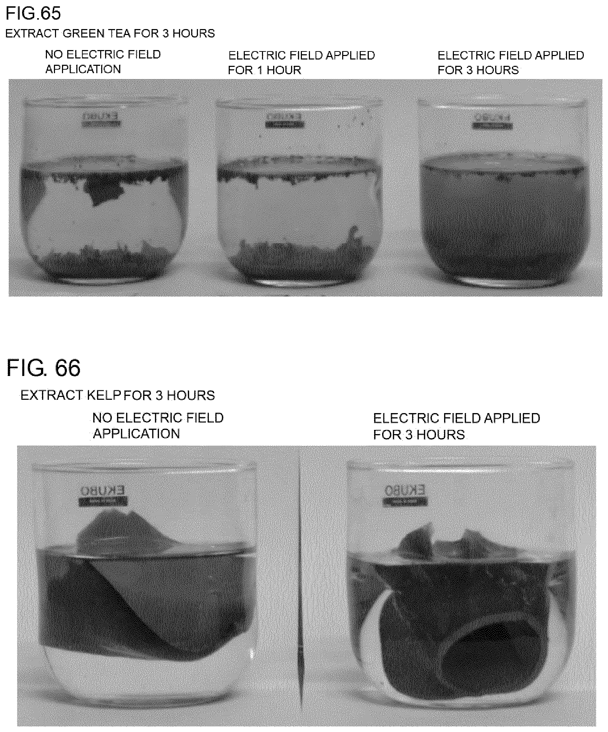

FIG. 65 is a photograph showing the extraction of green tea. Green tea was extracted for three hours while an electric field was applied using the electrode of the liquid control apparatus of the present embodiment. The left image shows a case with no electric field applied, the center image shows a case with an electric field applied for only one hour, and the right image shows a case with an electric field applied for three hours. As is clear from the photographs, the highest degree of extraction and highest concentration of green tea was obtained with the application of an electric field for three hours, the second highest degree of extraction was obtained with the application of an electric field for only one hour, and the lowest degree of extraction was obtained with no electric field applied. FIG. 65 has indicated that the application of an electric field by the liquid control apparatus of the present embodiment can increase the degree of extraction of green tea.

FIG. 66 is a photograph showing the extraction of kelp. Kelp was extracted for three hours while an electric field was applied using the electrode of the liquid control apparatus of the present embodiment. The left image shows a case with no electric field applied, and the right image shows a case with an electric field applied for three hours. As is clear from the photographs, with an electric field applied for three hours, the degree of extraction of kelp is higher, the kelp expansion is larger, and the amount of water absorption is larger. FIG. 66 has indicated that the application of an electric field by the liquid control apparatus of the present embodiment can increase the degree of extraction of kelp.

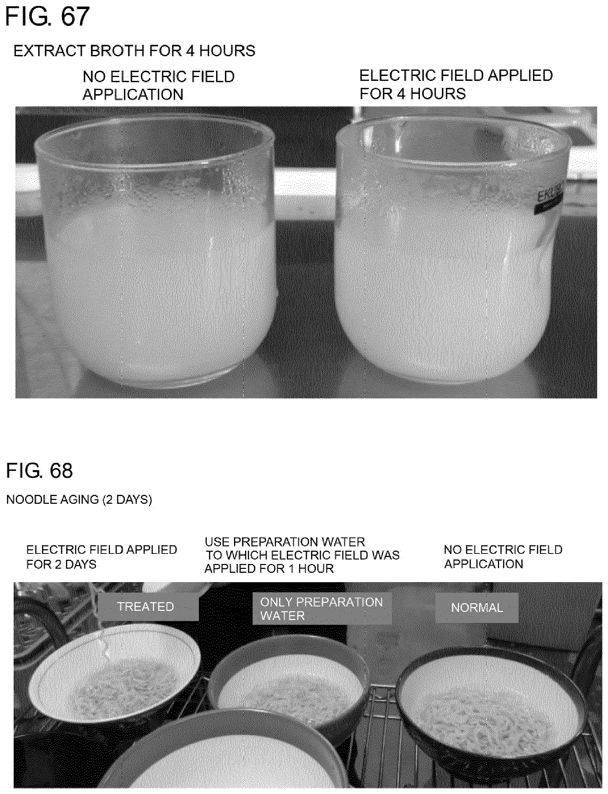

FIG. 67 is a photograph showing the extraction of broth. Ramen soup broth was extracted for four hours while an electric field was applied using the electrode of the liquid control apparatus of the present embodiment. The left image shows a case with no electric field applied, and the right image shows a case with an electric field applied for four hours. As is clear from the photographs, with the application of an electric field for four hours, the degree of extraction of the broth is higher and the concentration of the broth is thicker. When the concentration of the broth was measured, the broth to which an electric field was applied for four hours was thicker by 15%. In FIG. 67, the experiment was conducted using pork bone-based broth. However, the type of broth is not limited, and the degree of broth extraction can be increased by the liquid control apparatus of the present embodiment for fish-based, vegetable-based, or any type of broth. FIG. 67 has indicated that the application of an electric field by the liquid control apparatus of the present embodiment can increase the degree of broth extraction.

FIG. 68 is a photograph showing noodle aging. Noodles were aged for two days while an electric field was applied using the electrode of the liquid control apparatus of the present embodiment, and ramen was cooked. The water absorption, texture, and taste of the noodles were examined. The left image shows noodles aged for two days while an electric field was applied, the center image shows noodles cooked using preparation water to which an electric field was applied for one hour, and the right image shows noodles with no electric field applied. Amylase in flour is an enzyme that breaks down starch, and protease is an enzyme that breaks down protein. These enzymes are activated by moisture and moderate temperatures, and as a result, gluten formation is accelerated, resulting in elasticity and enhanced taste such as sweetness and umami. Ramen noodles are desired to absorb a moderate amount of water, and if the maturity is not enough, noodles absorb a large amount of water and get soggy quickly, resulting in a loss of texture and taste. The noodles aged for two days while applying an electric field in the left image had the most moderate degree of maturity and had the best texture and taste, because they were less soggy and absorbed a moderate amount of water. Although five to seven days of aging is usually required, an appropriate degree of maturity was achieved by applying an electric field by the liquid control apparatus of the present embodiment, even with the aging period of only two days. The noodles in the middle cooked with the preparation water to which an electric field was applied for one hour had the second most moderate degree of maturity and was less soggy. Although the degree of maturity is insufficient with the aging period of two days, compared with the needles without electric field application in the right image, the noodles get less soggy as can be understood from the degree of expansion of the noodles in the image. Based on the texture and the taste with the aging for two days, it is understood that the preparation water treated with an electric field by the liquid control apparatus of the present embodiment contributes to the quality enhancement of noodles. When ramen noodles are aged while an electric field is applied using the electrode of the liquid control apparatus of the present embodiment, the interfacial tension of the moisture in the ramen noodles decreases, and the moisture is atomized into fine particles. In addition, since the enzymes are activated, the aging such as gluten formation is promoted.

The experiments in FIGS. 65 to 68 show that the degrees of extraction and maturity can be increased by applying an electric field using the electrode of the liquid control apparatus of the present embodiment. This indicates that, by applying an electric field, the liquid control apparatus of the present embodiment performs control of improving or enhancing the extraction of body fluids, medicines, cosmetics, or liquids, and performs control of improving or enhancing the efficacy or quality of medicines, cosmetics, or liquids. In addition, it has been shown that, because of the enhancement of emulsion properties, the atomization of liquid into fine particles, and the arrangement in a pearl-chain structure, the liquid control apparatus of the present embodiment performs control of improving or enhancing the formation of teeth, bones, joints, blood vessels, lymph vessels, nerves, cells, skin, hair, or organs by applying an electric field using the electrode of the liquid control apparatus of the present embodiment.

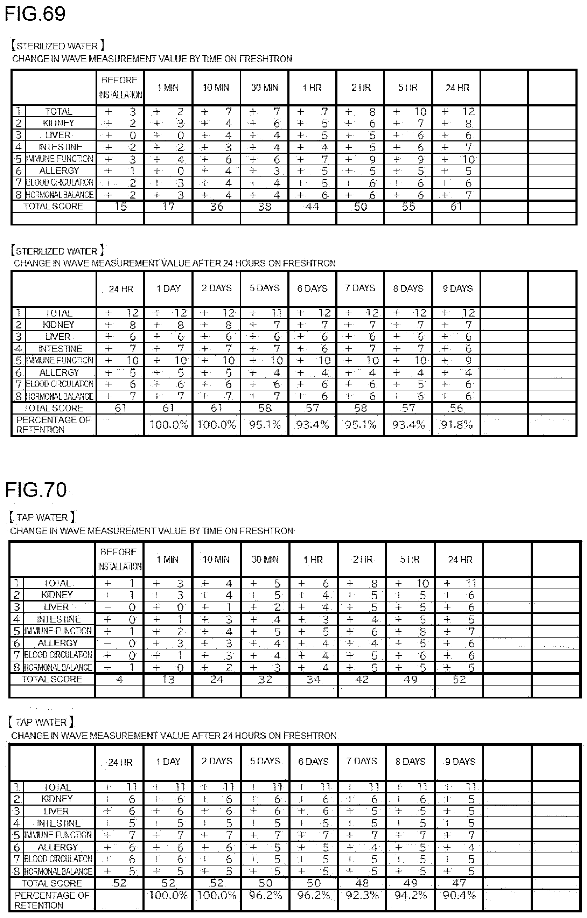

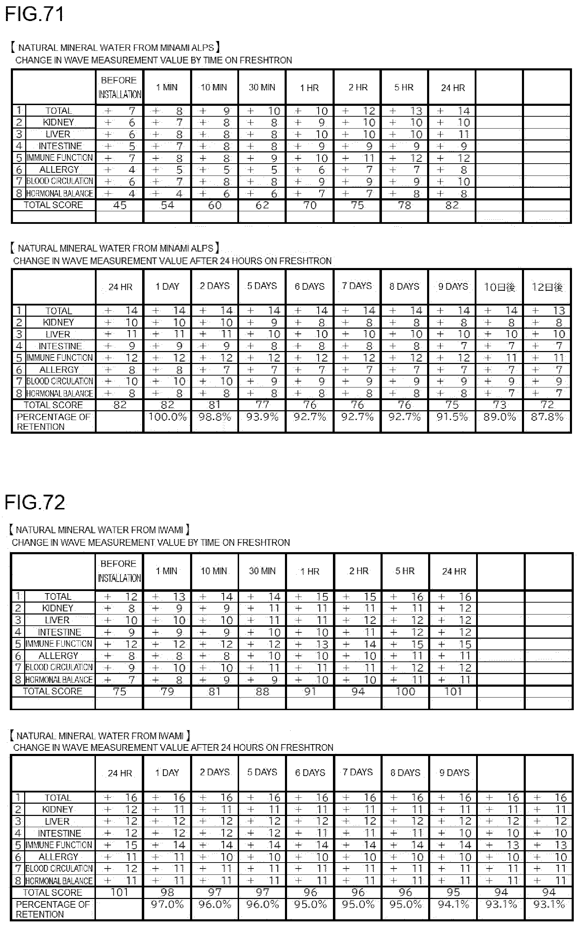

FIGS. 69 to 72 illustrate the measurement results of water wave motion. Not only living organisms but also all substances have weak energy that contributes to the health of living organisms. Water also has wave energy that contributes to the health of the human body. By measuring the wave energy of water as a relative value of its contribution to the human body (wave measurement value), the health status of the human body and the quality of water can be determined. The wave energy of water is quantified as a relative value of its contribution to human health according to the condition of water, the energy state of water, the quality of water, and the like. The more positive the value, the greater the wave energy, and the higher the total score, the higher the quality of water. Negative values are undesirable because human health may be disturbed. Each score quantifies the efficacy on the human body when the water is ingested. The following items: total; kidney; liver; intestine; immune function; allergy; blood circulation; and hormonal balance are quantified by relative positive and negative values. In each of FIGS. 69 to 72, the upper table shows the wave measurement values for each hour when an electric field was applied to the water by the electrode of the liquid control apparatus of the present invention, and the lower table shows the wave measurement values for each elapsed time after an electric field was applied to the water for 24 hours by the electrode of the liquid control apparatus of the present invention and then the electric field was turned off. The tables show the percentage of retention, where the total score at the time of the 24-hour application of an electric field is 100%. The measurement results in FIGS. 69 to 72 have indicated that, by applying an electric field from the electrode, the liquid control apparatus of the present embodiment can perform control to enhance the wave energy of liquid, that is, to increase the contribution of liquid to the health of the human body. By applying an electric field from the electrode, the liquid control apparatus of the present embodiment can enhance the quality of not only liquid for drinking but also body fluids present in the cells, organs, and tissues of the body, thereby contributing to enhancement of the fluidity of body fluids, enhancement of body fluid metabolism, stabilization of moisture balance, and, in addition, enhancement of body fluids themselves. In addition, it has been shown that since the liquid control apparatus of the present embodiment performs control to enhance the wave energy of water, it is also possible to control the energy that exists in nature, including water wave, for example, energy such as ectoplasm.

The respective embodiments described above are not intended to limit the present invention to these embodiments. The present invention is equally applicable to any other embodiments within the scope of the appended claims. The respective embodiments can be changed as appropriate and some of the respective embodiments can be used in combination as appropriate.

In the description of the principle of interfacial tension reduction control in the first embodiment, the interfacial tension between aqueous and oil phases has been described. However, the interfacial tension in the present embodiment is not limited to the interfacial tension between aqueous and oil phases and includes interfacial tension between any two phases including liquid, interfacial tension between two types of liquids, interfacial tension between liquid and solid, and interfacial tension between liquid and gas. The liquid control apparatus of the present embodiment can control any interfacial tension.

In FIG. 13, the configuration denoted as the housing 50 is not limited to the shape of a box and is meant to be any device. Therefore, the configuration includes not only refrigerator and storage container but also various devices and equipment using the electrode of the liquid control apparatus of the present embodiment, for example, therapy table, treatment table, cot, electromagnetic therapy device, potential therapy device, low frequency therapy device, EMS, massage device, facial device, vibration device, cavitation device, micro-bubble device, micro-nano bubble device, nano bubble device, fine valve device, terahertz device, high frequency device, quantum therapy device, hair growth device, cellulite device, muscle relaxation device, ultrasonic therapy device, ozone generating device, hydrogen generating device, LED device, beauty device, slimming device, or apparatus, device, equipment, or facility with a storage container.

In each experimental example and each measurement example in the foregoing embodiments, the voltage and the frequency applied to the electrode are set to 100 V and 50 KHz for ease of comparison and study. However, these values are only an example of the target values of the liquid control apparatus of the present embodiment and are not intended to limit the target values of the present embodiment. For example, the voltage value from 0 to 7000V and the frequency from 0 to 100 THz described in the present embodiment can be set as appropriate through a variety of computation such as machine learning, control parameters stored in advance, or control parameters input or set in advance by the cloud server (management server 40) and the controller 10, as described in the foregoing first embodiment. Among various control goals and control targets, the optimal control parameters are diverse. Appropriate control parameters are set according to a target object, a target site, the type and the state of a target object and a target site, a liquid control goal, and the form of the electrode, and the like, using the procedure described in the first embodiment. Although not limited to any particular frequency range, the frequency range of 1 Hz to 50 Hz, for example, may be used for the control of brain waves, nerves, and sleep. The frequencies in the terahertz range are used in terahertz devices. A voltage of 7000 V or lower has been described as a voltage range, for safety reasons, but if safety measures such as insulation can be employed, special high voltage exceeding 7000 V may be used as the target value. However, the liquid control apparatus of the present embodiment exhibits practically sufficient performance even in the voltage value and frequency range of about 50 KHz and 100 V, as indicated by the experimental examples. Therefore, the lower voltage range in power category, namely, AC 600 V or lower or DC 750 V or lower can be employed.

### Reference Signs List

- 1: liquid control apparatus

- 10: controller
- 11: current voltage application unit
- 13 to 29: electrode
- 30: active oxygen
- 31: man-machine interface (PC)
- 32: object detection sensor
- 33: current voltage control unit
- 35: communication unit
- 36: control unit
- 37: storage unit
- 38: detection unit
- 40: management server
- 41: connector
- 43: database
- 45: communication network
- 50: housing

## Claims

1. A liquid control apparatus comprising:
at least one electrode; and
a controller configured to control at least one of a voltage value, a current value, a frequency, or a phase of voltage or current applied to the electrode, wherein
while the electrode is disposed to face a target object,
the controller adjusts at least one of a voltage value, a current value, a frequency, or a phase of the voltage or the current having a DC component and/or an AC component applied to the electrode, controls at least one of an electric field, a magnetic field, an electromagnetic field, or electromagnetic waves generated from the electrode, and controls a state of liquid in the target object facing the electrode,
to perform control of improving or preventing edema, cellular edema, or neuronal edema,
to control hormonal, endocrine, lymphatic, or pathway balance, or
to control mitochondrial activity, autophagy activity, egg activity, or sperm activity.

2. A liquid control apparatus comprising:
at least one electrode; and
a controller configured to control at least one of a voltage value, a current value, a frequency, or a phase of voltage or current applied to the electrode, wherein
while the electrode is disposed to face a target object,
the controller adjusts at least one of a voltage value, a current value, a frequency, or a phase of voltage or current having a DC component and/or an AC component applied to the electrode, controls at least one of an electric field, a magnetic field, an electromagnetic field, or electromagnetic waves generated from the electrode, and controls a state of liquid in the target object facing the electrode,
to perform control of improving or preventing clogging of body fluid, drug solution, cosmetics, or liquid,
to perform control of improving or enhancing fluidity of body fluid, drug solution, cosmetics, or liquid,
to perform control of improving or preventing induration or hardening,
to perform control of improving or preventing water release from cells or tissues,
to perform control of removing active oxygen or lactic acid,
to perform control of improving or preventing muscle imbalance,
to perform control of improving or enhancing body balance, myofascial balance, occlusion, osteopathy, or manual therapy,
to perform control of improving or preventing bone fracture or joint disease,
to perform control of improving or enhancing heart beat or pulse,
to perform control of improving or normalizing blood pressure,
to perform control of improving or enhancing respiratory function or lung function,
to perform control of improving or enhancing dialysis function,
to perform control of improving or enhancing visual acuity or dynamic vision,
to perform control of improving or preventing ophthalmologic disease,
to perform control of improving or preventing internal medicine disease,
to perform control of improving or enhancing immune function,
to perform control of improving a drug delivery system or permeability of cosmetics,
to perform control of improving or enhancing efficacy or quality of medicine, cosmetics, or liquid,
to perform control of improving or enhancing extraction of body fluid, medicine, cosmetics, or liquid, or
to control wave motion of liquid or ectoplasm.

3. A liquid control apparatus comprising:
at least one electrode; and
a controller configured to control at least one of a voltage value, a current value, a frequency, or a phase of voltage or current applied to the electrode, wherein
while the electrode is disposed to face a target object,
the controller adjusts at least one of a voltage value, a current value, a frequency, or a phase of voltage or current having a DC component and/or an AC component applied to the electrode, controls at least one of an electric field, a magnetic field, an electromagnetic field, or electromagnetic waves generated from the electrode, and controls a state of liquid in the target object facing the electrode,
to perform control of preventing growth of microorganisms, bacteria, fungi, or viruses,
to perform control of improving or preventing burns, necrosis, or decubitus ulcer,
to perform control of preventing metastasis,
to perform control of improving or preventing dementia, Alzheimer's disease, or Parkinson's disease,
to perform control of sleep improvement, cosmetic improvement, PMS, menstrual pain, pain, itching, health promotion, motor function improvement, or anti-aging,
to perform control of improving or enhancing formation of teeth, bones, joints, blood vessels, lymph vessels, nerves, cells, skin, hair, or organs,
to perform control of improving storage, freezing, thawing, culturing, or logistics of organs, body fluids, blood, cells, tissues, skin, hair, dead bodies, DNA, stem cells, sperms, eggs, medicines, cosmetics, or liquids, or
to improve an electrode or a container for use in storage, freezing, thawing, culturing, or logistics of organs, body fluids, blood, cells, tissues, skin, hair, dead bodies, DNA, stem cells, sperms, eggs, medicines, cosmetics, or liquids.

4. The liquid control apparatus according to any one of claims 1 to 3, wherein the voltage value of voltage applied to the electrode is controlled in a range of 0 V to 7000 V, or the frequency of the AC component of the voltage is controlled in a range of 0 Hz to 1 MHz.

5. The liquid control apparatus according to any one of claims 1 to 3, wherein the voltage value and/or the frequency of the voltage varies over time smoothly or stepwise within a predetermined range.

6. The liquid control apparatus according to any one of claims 1 to 3, wherein the liquid control apparatus performs
controlling a pearl-chain structure, arrangement direction, moisture bonding, or moisture activity of moisture in the target object,
controlling interfacial polarization, interfacial tension, or emulsion state between an aqueous phase and another phase in the target object, or
controlling a state of active oxygen in the target object.

7. The liquid control apparatus according to any one of claims 1 to 3, wherein a control effect is sustained for a predetermined period of time even after an electric field, a magnetic field, an electromagnetic field, or electromagnetic waves are generated from the electrode for a predetermined period of time, and then the electric field, the magnetic field, the electromagnetic field, or the electromagnetic waves are removed.

8. The liquid control apparatus according to any one of claims 1 to 3, wherein the voltage applied to the electrode includes the AC component in addition to the DC component.

9. The liquid control apparatus according to any one of claims 1 to 3, wherein the electrode has a shape of plate, rod, sheet, needle, or comb, or a shape combining two or more electrodes with each other.

10. The liquid control apparatus according to any one of claims 1 to 3, wherein the controller is managed via a cloud, a server, or a network.

11. The liquid control apparatus according to any one of claims 1 to 3, wherein the controller sets a control parameter by machine learning.

12. The liquid control apparatus according to any one of claims 1 to 3, wherein control is performed to achieve efficacy for at least one of infarction, necrosis, decubitus ulcer, burns, removal of active oxygen, removal of lactic acid, improvement of blood flow, improvement of lymph flow, cerebral infarction, myocardial infarction, thrombosis, embolism, arteriosclerosis, improvement or prevention of clogging of body fluid, drug solution, cosmetics, or liquid, improvement of fluidity of body fluid, drug solution, cosmetics, or liquid, improvement or prevention of induration or hardening, improvement or prevention of water release from cells or tissues, cellular edema, neuronal edema, hydropsy, pulmonary edema, joint edema, ascites edema, insulin secretion disorder, defecation disorder, defecation difficulty, constipation, urinary disorder, congestion, blisters, enhancement of drug delivery, reduction of viscosity of drug solution or body fluid, cell culture, regenerative medicine, reduction of culture time, enhancement of culture quality, or enhancement of culture efficiency in regenerative medicine, cell tissue regeneration, reduction of lactic acid, enlargement, swelling, edema, body fluid retention, dehydration of extracellular or intracellular fluid, skin diseases, pigmentation, chloasma, freckle, epidermal wrinkles, dermal wrinkles, expression wrinkles, xeroderma, improvement of physical condition, induration, muscular induration, myofascial release, myofascial potential improvement, nerve deformation disease, myofascial electromagnetic therapy, muscle imbalance, myofascial potential balance improvement, body balance improvement, myofascial balance improvement, occlusion improvement, osteopathic improvement, manual therapeutic improvement, improvement or prevention of bone fracture or joint disease, endocrine, lymphatic, or pathway balance improvement, bone fracture, joint disease, skin care, moisture retention, fertility improvement, infertility, improvement of sperm motility, sperm activity, egg activity, mitochondrial activity, autophagy activity, PMS, pain, itching, abnormal sensation, cold sensitivity, insensitivity, cramp, anti-aging, hair follicle care, improvement of menopausal disorder, improvement of detergency, alopecia, AGA, ED, lipolysis promotion, improvement of contact lens fit, dry eye, visual acuity improvement, dynamic vision improvement, sleep disorder, sleep improvement, sleep apnea syndrome, preventive medicine improvement, nerve cell improvement, cell edema improvement, control of viruses, bacteria, or molds, cancer, glaucoma, cataract, age-related macular degeneration, eye disease, hearing loss, hearing impairment, visual impairment, dementia, Alzheimer's disease, Parkinson's disease, sleep improvement, cosmetic improvement, health promotion, motor function improvement, water balance, gastrointestinal diseases, respiratory diseases, cardiovascular diseases, neurological diseases, hematologic diseases, kidney diseases, endocrinological diseases, internal medicine diseases, improvement or normalization of blood pressure, improvement or enhancement of pulse or heartbeat, improvement or enhancement of respiratory function or pulmonary function, improvement or enhancement of dialysis function, osteopathic therapy, rigor mortis improvement, or therapy for rehabilitation medicine improvement.

13. The liquid control apparatus according to any one of claims 1 to 3, wherein control is performed to achieve efficacy for at least one of improvement of storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement of an electrode or a container for use in storage, freezing, thawing, culturing, or logistics of organs, body fluids, blood, cells, tissues, skin, teeth, bones, joints, hair, dead bodies, DNA, stem cells, sperms, eggs, medicines, cosmetics, or liquids, improvement or enhancement of efficacy or quality of medicines, cosmetics, or liquids, improvement or enhancement of extraction of body fluids, medicines, cosmetics, or liquids, improvement or enhancement of formation of teeth, bones, joints, blood vessels, lymph vessels, nerves, cells, skin, hair, or organs, improvement or enhancement of emulsion properties, improvement or enhancement of drug delivery properties, improvement or enhancement of efficacy or performance of medicines, prevention or control of growth of microorganisms, bacteria, fungi, or viruses, prevention of metastasis, reduction of viscosity of drug solution, or control of wave motion or ectoplasm.

14. The liquid control apparatus according to any one of claims 1 to 3, wherein control is performed to enhance performance of at least one of following devices: an electromagnetic therapy device; a potential therapy device; a low frequency therapy device; an EMS; a massage device; a facial device; a vibration device; a cavitation device; a micro-bubble device; a micro-nano bubble device; a nano bubble device; a fine bubble device; a terahertz device; a high frequency device; a quantum therapy device; a hair growth device; a cellulite device; a muscle relaxation device; an ultrasonic therapy device; an ozone generating device; a hydrogen generating device; an LED device; a beauty device, and a slimming device.

15. A liquid control apparatus comprising:
at least one electrode; and
a controller configured to control at least one of a voltage value, a current value, a frequency, or a phase of voltage or current applied to the electrode, wherein
while the electrode is disposed to face a target object,
the controller adjusts at least one of a voltage value, a current value, a frequency, or a phase of voltage or current having a DC component and/or an AC component applied to the electrode, and controls at least one of an electric field, a magnetic field, an electromagnetic field, or electromagnetic waves generated from the electrode, and
the controller performs control so that at least one of a frequency or a voltage applied to the electrode, or an emission direction of an electric field, a magnetic field, an electromagnetic field, or electromagnetic waves generated from the electrode changes over time, or controls a duration of voltage applied to the electrode, according to the target object facing the electrode.
